# EUROPEAN PATENT APPLICATION

(11) **EP 3 992 205 A1**
(43) Date of publication of application: **04.05.2022**
(21) Application number: 20205382.3
(22) Date of filing: 03.11.2020
(51) Int. Cl.: C07K 16/10, A61P 31/14, G01N 33/577

(54) **SARS CORONAVIRUS-2 SPIKE PROTEIN BINDING COMPOUNDS**

(71) Applicant: Rheinische Friedrich-Wilhelms-Universität Bonn, 53113 Bonn (DE)
(72) Inventor: KÖNIG, Paul-Albert, 53127 Bonn (DE); SCHMIDT, Florian, 53127 Bonn (DE); NORMANN, Sabine, 53127 Bonn (DE); TÖDTMANN, Jan Moritz-Paul, 53127 Bonn (DE); HÄLLLBERG, 17177 Stockholm (SE)
(74) Representative: Kuttenkeuler, David

(57) **Abstract**

The invention pertains to protein binding compounds, such as antibodies and antibody like compounds, specifically targeting epitopes of the Corona virus spike (S) protein which were found to stabilize a three-dimensional conformation of the protein which reduces or inhibits the viral mechanisms necessary for host cell entry. The invention provides the protein binding compounds alone and in combination and multispecific constructs, as well as their use in the prevention, treatment, patient stratification and diagnosis of viral infections caused by a corona virus such as severe acute respiratory syndrome corona virus 2 (SARS CoV2).

SEQUENCE LISTING

<110>  Rheinische Friedrich-Wilhelms-Universität Bonn

<120>  CORONA VIRUS SPIKE PROTEIN BINDING COMPOUNDS

<130>  U31021EP

<160>  95

<170>  PatentIn version 3.5

<210>  1
<211>  8
<212>  PRT
<213>  Homo sapiens

<400>  1

Gly Phe Thr Leu Asp Asp Tyr Ala
1               5

<210>  2
<211>  8
<212>  PRT
<213>  Homo sapiens

<400>  2

Ile Ser Ser Ser Asp Gly Ser Thr
1               5

<210>  3
<211>  19
<212>  PRT
<213>  Homo sapiens

<400>  3

Ala Ala Thr Tyr Trp Ser Gly Ser Val Trp Tyr Met Asp Ala Glu Glu
1               5                   10                  15

Tyr Asp Tyr

<210>  4
<211>  126
<212>  PRT
<213>  Homo sapiens

<400>  4

Gln Val Gln Leu Val Glu Thr Gly Gly Gly Leu Val Gln Ala Gly Gly
1               5                   10                  15

Ser Leu Arg Leu Ser Cys Ala Ala Ser Gly Phe Thr Leu Asp Asp Tyr
            20                  25                  30

Ala Ile Gly Trp Phe Arg Gln Ala Pro Gly Lys Glu Arg Glu Gly Val
        35                  40                  45

Ser Ser Ile Ser Ser Ser Asp Gly Ser Thr Tyr Tyr Ala Asp Ser Val
    50                  55                  60

Lys Ala Arg Phe Ala Ile Ser Ser Asp Asn Ala Lys Asn Val Val Tyr
65                  70                  75                  80

Leu Gln Met Asn Ser Leu Lys Pro Glu Asp Thr Ala Val Tyr Tyr Cys
                85                  90                  95

Ala Ala Thr Tyr Trp Ser Gly Ser Val Trp Tyr Met Asp Ala Glu Glu
            100                 105                 110

Tyr Asp Tyr Trp Gly Gln Gly Thr Gln Val Thr Val Ser Ala
        115                 120                 125

<210>  5
<211>  8
<212>  PRT
<213>  Homo sapiens

<400>  5

Gly Phe Gly Tyr Asp Asp His Val
1               5

<210>  6
<211>  8
<212>  PRT
<213>  Homo sapiens

<400>  6

Ile Ser Thr Asp Asp Gly Ser Thr
1               5

<210>  7
<211>  22
<212>  PRT
<213>  Homo sapiens

<400>  7

Ala Ala Thr Lys Gly Trp His Thr Glu Tyr Asp Leu Lys Arg Leu Arg
1               5                   10                  15

Asp Phe Asp Phe Glu Thr
            20

<210>  8

<211>  130
<212>  PRT
<213>  Homo sapiens

<400>  8

Gln Val Gln Leu Val Glu Thr Gly Gly Gly Leu Val Gln Pro Gly Gly
1               5                   10                  15

Ser Leu Ile Leu Ser Cys Gln Val Asp Gly Phe Gly Tyr Asp Asp His
            20                  25                  30

Val Ile Gly Trp Phe Arg Gln Ala Pro Gly Lys Glu Arg Glu Gly Val
        35                  40                  45

Ser Tyr Ile Ser Thr Asp Asp Asp Arg Ser Thr Trp Tyr Ala Glu Ser
    50                  55                  60

Val Lys Gly Arg Phe Ala Ile Ser Lys Asp Ser Ala Glu Asp Thr Thr
65                  70                  75                  80

Tyr Leu Gln Met Asn Asn Leu Lys Ala Glu Asp Thr Ala Val Tyr Tyr
                85                  90                  95

Cys Ala Ala Thr Lys Gly Trp His Thr Glu Tyr Asp Leu Lys Arg Leu
            100                 105                 110

Arg Asp Phe Asp Phe Glu Thr Trp Gly Gln Gly Thr Gln Val Thr Val
        115                 120                 125

Ser Ser
    130


<210>  9
<211>  8
<212>  PRT
<213>  Homo sapiens

<400>  9

Gly Phe Thr Leu Asp Tyr Tyr Ala
1               5


<210>  10
<211>  8
<212>  PRT
<213>  Homo sapiens

<400>  10

Val Ser Ser Ser Asp Gly Ser Thr
1               5

<210>  11
<211>  17
<212>  PRT
<213>  Homo sapiens

<400>  11

Ala His Lys Gln Arg Asp Ile Cys Ser Gly Tyr Glu Phe Leu Tyr Asp
1               5                   10                  15

Tyr

<210>  12
<211>  124
<212>  PRT
<213>  Homo sapiens

<400>  12

Gln Leu Gln Leu Val Glu Thr Gly Gly Gly Leu Val Gln Pro Gly Gly
1               5                   10                  15

Ser Leu Arg Leu Ser Cys Ala Ala Ser Gly Phe Thr Leu Asp Tyr Tyr
            20                  25                  30

Ala Ile Gly Trp Phe Arg Gln Ala Pro Gly Lys Glu Arg Glu Gly Val
        35                  40                  45

Ser Cys Val Ser Ser Ser Asp Gly Ser Thr Tyr Tyr Ala Asp Ser Val
    50                  55                  60

Lys Gly Arg Phe Thr Ile Ser Arg Asp Ser Ala Lys Asn Thr Val Tyr
65                  70                  75                  80

Leu Gln Met Asn Ser Leu Lys Pro Glu Asp Thr Ala Val Tyr Tyr Cys
                85                  90                  95

Ala His Lys Gln Arg Asp Ile Cys Ser Gly Tyr Glu Phe Leu Tyr Asp
            100                 105                 110

Tyr Trp Gly Gln Gly Thr Gln Val Thr Val Ser Ser
        115                 120

<210>  13
<211>  8
<212>  PRT
<213>  Homo sapiens

<400>  13

Gly Arg Thr Phe Gly Ser Tyr Ala

1               5


<210>  14
<211>  8
<212>  PRT
<213>  Homo sapiens

<400>  14

Met Thr Trp Ser Ser Met Ser Pro
1               5


<210>  15
<211>  19
<212>  PRT
<213>  Homo sapiens

<400>  15

Ala Ala Val Gly Leu Ser Val Pro Phe Ile Glu Thr His Glu His Glu
1               5                   10                  15


Tyr Asp Tyr



<210>  16
<211>  126
<212>  PRT
<213>  Homo sapiens

<400>  16

Gln Val Gln Leu Val Glu Thr Gly Gly Gly Arg Val Gln Ala Gly Asp
1               5                   10                  15


Ser Leu Arg Leu Ser Cys Val Ala Ser Gly Arg Thr Phe Gly Ser Tyr
            20                  25                  30


Ala Val Ala Trp Phe Arg Gln Ala Pro Gly Lys Glu Arg Glu Phe Val
        35                  40                  45


Ala Ala Met Thr Trp Ser Ser Met Ser Pro Asn Tyr Ala Asp Ala Val
    50                  55                  60


Lys Gly Arg Phe Thr Ile Ser Arg Asp Asn Ala Lys Asn Thr Ala Tyr
65                  70                  75                  80


Leu Gln Met Asn Arg Leu Ala Pro Glu Asp Thr Ala Val Tyr Tyr Cys
                85                  90                  95


Ala Ala Val Gly Leu Ser Val Pro Phe Ile Glu Thr His Glu His Glu
            100                 105                 110

Tyr Asp Tyr Trp Gly Gln Gly Thr Gln Val Thr Val Ser Ser
        115                 120                 125

<210>  17
<211>  8
<212>  PRT
<213>  Homo sapiens

<400>  17

Gly Val Thr Leu Asp Tyr Tyr Ala
1               5

<210>  18
<211>  8
<212>  PRT
<213>  Homo sapiens

<400>  18

Ile Gly Ser Ser Asp Gly Arg Thr
1               5

<210>  19
<211>  22
<212>  PRT
<213>  Homo sapiens

<400>  19

Ala Leu Thr Val Gly Thr Tyr Tyr Ser Gly Asn Tyr His Tyr Thr Cys
1               5                   10                  15

Ser Asp Asp Met Asp Tyr
            20

<210>  20
<211>  129
<212>  PRT
<213>  Homo sapiens

<400>  20

Gln Val Gln Leu Val Glu Thr Gly Gly Gly Phe Val Gln Pro Gly Gly
1               5                   10                  15

Ser Leu Arg Leu Ser Cys Ala Ala Ser Gly Val Thr Leu Asp Tyr Tyr
            20                  25                  30

Ala Ile Gly Trp Phe Arg Gln Ala Pro Gly Lys Glu Arg Glu Gly Val
        35                  40                  45

Ser Cys Ile Gly Ser Ser Asp Gly Arg Thr Tyr Tyr Ser Asp Ser Val
    50                  55                  60

Lys Gly Arg Phe Thr Ile Ser Arg Asp Asn Ala Lys Asn Thr Val Tyr
65                  70                  75                  80

Leu Gln Met Asn Ser Leu Lys Pro Glu Asp Thr Ala Val Tyr Tyr Cys
                85                  90                  95

Ala Leu Thr Val Gly Thr Tyr Tyr Ser Gly Asn Tyr His Tyr Thr Cys
            100                 105                 110

Ser Asp Asp Met Asp Tyr Trp Gly Lys Gly Thr Leu Val Thr Val Ser
        115                 120                 125

Ser

<210>  21
<211>  8
<212>  PRT
<213>  Homo sapiens

<400>  21

Gly Arg Thr Phe Ser Asp Tyr Ala
1               5

<210>  22
<211>  6
<212>  PRT
<213>  Homo sapiens

<400>  22

Ile Gly Gly Gly Ser Ile
1               5

<210>  23
<211>  23
<212>  PRT
<213>  Homo sapiens

<400>  23

Ala Val Ala Val Gly Ala Thr Thr Arg Pro Ser Trp Arg Ser Arg Val
1               5                   10                  15

Ala Glu Val Tyr Tyr Asp Tyr
            20

<210>  24
<211>  128
<212>  PRT
<213>  Homo sapiens

<400>  24

Gln Val Gln Leu Val Glu Thr Gly Gly Gly Leu Val Gln Ala Gly Asp
1               5                   10                  15

Ser Leu Arg Leu Ser Cys Thr Ala Ser Gly Arg Thr Phe Ser Asp Tyr
            20                  25                  30

Ala Leu Gly Trp Phe Arg Gln Ala Pro Gly Gln Glu Arg Glu Phe Val
        35                  40                  45

Ala Val Ile Gly Gly Gly Ser Ile Tyr Tyr Ala Glu Ser Val Glu Gly
    50                  55                  60

Arg Phe Ala Ile Ser Arg Asp Asn Ala Arg Asn Thr Ala Tyr Leu Gln
65                  70                  75                  80

Met Asn Ala Leu Lys Pro Glu Asp Thr Ala Asp Tyr Tyr Cys Ala Val
                85                  90                  95

Ala Val Gly Ala Thr Thr Arg Pro Ser Trp Arg Ser Arg Val Ala Glu
            100                 105                 110

Val Tyr Tyr Asp Tyr Trp Gly Gln Gly Thr Arg Val Thr Val Thr Thr
        115                 120                 125

<210>  25
<211>  5
<212>  PRT
<213>  Homo sapiens

<400>  25

Glu Asp Thr Asp Ala
1               5

<210>  26
<211>  8
<212>  PRT
<213>  Homo sapiens

<400>  26

Ile Thr Trp Asn Ala Asp Arg Thr
1               5

<210>  27
<211>  18
<212>  PRT
<213>  Homo sapiens

<400>  27

Ala Ala Ala Val Gly Gly Ser Tyr Tyr Pro Pro Val Pro Ser Glu Tyr
1               5                   10                  15

Asp Tyr

<210>  28
<211>  122
<212>  PRT
<213>  Homo sapiens

<400>  28

Gln Leu Gln Leu Val Glu Thr Gly Gly Gly Leu Val Gln Ala Gly Asp
1               5                   10                  15

Ser Leu Arg Leu Ser Cys Ala Ala Ser Glu Asp Thr Asp Ala Met Gly
            20                  25                  30

Trp Phe Arg Gln Ala Pro Gly Lys Glu Arg Glu Phe Val Ala Thr Ile
        35                  40                  45

Thr Trp Asn Ala Asp Arg Thr Tyr Tyr Ala Asp Ser Val Lys Gly Arg
    50                  55                  60

Phe Ala Ile Ser Arg Asp Asn Ala Lys Ser Thr Val Tyr Leu Gln Met
65                  70                  75                  80

Asn Ser Leu Lys Pro Glu Asp Thr Ala Val Tyr Tyr Cys Ala Ala Ala
                85                  90                  95

Val Gly Gly Ser Tyr Tyr Pro Pro Val Pro Ser Glu Tyr Asp Tyr Trp
            100                 105                 110

Gly Gln Gly Thr Gln Val Thr Val Ser Ser
        115                 120

<210>  29
<211>  8
<212>  PRT
<213>  Homo sapiens

<400>  29

Glu Asn Ile Val Asn Phe Gly Ser
1               5

<210>  30
<211>  7
<212>  PRT
<213>  Homo sapiens

<400>  30

Ile Thr Asp Gly Gly Ser Thr
1               5


<210>  31
<211>  16
<212>  PRT
<213>  Homo sapiens

<400>  31

Val Ala Ala Arg Ser Asn Leu Ala Gly Trp Gly Glu Asp Phe Asp Ser
1               5                   10                  15


<210>  32
<211>  122
<212>  PRT
<213>  Homo sapiens

<400>  32

Gln Val Gln Leu Val Glu Thr Gly Gly Gly Leu Val Gln Thr Gly Asp
1               5                   10                  15

Ser Leu Arg Leu Ser Cys Thr Ala Ser Glu Asn Ile Val Asn Phe Gly
            20                  25                  30

Ser Met Gly Trp Tyr Arg Gln Ala Pro Gly Lys Gln Arg Glu Leu Val
        35                  40                  45

Ala Ala Ile Thr Asp Gly Gly Ser Thr Ile Tyr Thr Asp Ser Val Lys
    50                  55                  60

Gly Arg Phe Ile Ile Ser Arg Asp Asn Ala Lys Asn Thr Val Phe Met
65                  70                  75                  80

Gln Met Asn Ser Leu Arg Pro Ala Asp Ser Ala Thr Tyr Tyr Cys Val
                85                  90                  95

Ala Ala Arg Ser Asn Leu Ala Gly Trp Gly Glu Asp Phe Asp Ser Trp
            100                 105                 110

Gly Gln Gly Thr Gln Val Thr Val Ser Ser
        115                 120


<210>  33
<211>  8
<212>  PRT
<213>  Homo sapiens

<400>  33

Gly Ser Ile Phe Ser Ile Gln Ala
1               5

<210>  34
<211>  7
<212>  PRT
<213>  Homo sapiens

<400>  34

Ile Gly Ala Ser Gly Asn Thr
1               5

<210>  35
<211>  14
<212>  PRT
<213>  Homo sapiens

<400>  35

Tyr Ala Leu Thr Arg Leu Pro Leu Pro Asp Gly Met Asp Tyr
1               5                   10

<210>  36
<211>  120
<212>  PRT
<213>  Homo sapiens

<400>  36

Gln Leu Gln Leu Val Glu Thr Gly Gly Gly Leu Val Gln Ala Gly Asp
1               5                   10                  15

Ser Leu Arg Leu Ser Cys Val Ala Ser Gly Ser Ile Phe Ser Ile Gln
            20                  25                  30

Ala Met Gly Trp Tyr Arg Gln Ala Pro Gly Asn Glu Arg Glu Leu Val
        35                  40                  45

Ala Gly Ile Gly Ala Ser Gly Asn Thr Lys Tyr Gly Asp Ser Val Arg
    50                  55                  60

Gly Arg Phe Thr Ile Ser Arg Asp Asp Glu Glu Asn Lys Val Tyr Val
65                  70                  75                  80

Gln Met Asn Asp Leu Lys Thr Glu Asp Thr Ala Val Tyr Arg Cys Tyr
                85                  90                  95

Ala Leu Thr Arg Leu Pro Leu Pro Asp Gly Met Asp Tyr Trp Gly Glu
            100                 105                 110

Gly Thr Pro Val Thr Val Ser Ser

115                 120

<210>  37
<211>  5
<212>  PRT
<213>  Homo sapiens

<400>  37

Gly Arg Ala Tyr Ala
1               5

<210>  38
<211>  7
<212>  PRT
<213>  Homo sapiens

<400>  38

Ile Leu Trp Ser Gly Ala Ser
1               5

<210>  39
<211>  18
<212>  PRT
<213>  Homo sapiens

<400>  39

Ala Ala Asp Leu Gln Phe Pro Leu Thr Thr Pro Gln Phe His Glu Tyr
1               5                   10                  15

Asp Val

<210>  40
<211>  120
<212>  PRT
<213>  Homo sapiens

<400>  40

Gln Leu Gln Leu Val Glu Thr Gly Gly Gly Arg Val Gln Ala Gly Asp
1               5                   10                  15

Ser Leu Arg Leu Ser Cys Thr Ala Ser Gly Arg Ala Tyr Ala Met Gly
            20                  25                  30

Trp Phe Arg Gln Ala Pro Gly Lys Glu Arg Glu Phe Val Ala Ala Ile
        35                  40                  45

Leu Trp Ser Gly Ala Ser Tyr Ala Asp Ser Val Lys Asp Arg Phe Thr
    50                  55                  60

Ile Ser Arg Asp Asn Ala Lys Asn Thr Val Tyr Leu Gln Met Asn Ser
65                  70                  75                  80

Leu Lys Pro Glu Asp Thr Ala Val Tyr Tyr Cys Ala Ala Asp Leu Gln
                85                  90                  95

Phe Pro Leu Thr Thr Pro Gln Phe His Glu Tyr Asp Val Trp Gly Gln
            100                 105                 110

Gly Thr Gln Val Thr Val Ser Ser
        115                 120

<210>  41
<211>  8
<212>  PRT
<213>  Homo sapiens

<400>  41

Gly Arg Ala Phe Asn Asn Asn Gly
1               5

<210>  42
<211>  8
<212>  PRT
<213>  Homo sapiens

<400>  42

Ile Asn Trp Ser Gly Tyr Ser Thr
1               5

<210>  43
<211>  18
<212>  PRT
<213>  Homo sapiens

<400>  43

Ala Ala Ser Leu Gly Ser Ser Trp Tyr Ser His Asp Ala Asn Asp Tyr
1               5                   10                  15

Pro Tyr

<210>  44
<211>  125
<212>  PRT
<213>  Homo sapiens

<400>  44

Gln Leu Gln Leu Val Glu Thr Gly Gly Gly Leu Val Gln Ala Gly Asp
1               5                   10                  15

Ser Leu Arg Leu Ser Cys Ala Ala Ser Gly Arg Ala Phe Asn Asn Asn
            20                  25                  30

Gly Ile Ala Trp Phe Arg Gln Ala Pro Gly Gln Glu Arg Glu Phe Val
        35                  40                  45

Ala Val Ile Asn Trp Ser Gly Tyr Ser Thr Asp Tyr Ala Asp Ser Val
    50                  55                  60

Lys Gly Arg Phe Thr Ile Ser Arg Asp Asn Val Lys Ser Val Val Tyr
65                  70                  75                  80

Leu Gln Met Asn Ser Leu Glu Ser Glu Asp Thr Ala Val Tyr Tyr Cys
                85                  90                  95

Ala Ala Ser Leu Gly Ser Ser Trp Tyr Ser His Asp Ala Asn Asp Tyr
            100                 105                 110

Pro Tyr Trp Gly Gln Gly Thr Gln Val Thr Val Ser Ser
        115                 120                 125

<210>  45
<211>  8
<212>  PRT
<213>  Homo sapiens

<400>  45

Ala Phe Thr Phe Gly Tyr Tyr Ala
1               5

<210>  46
<211>  8
<212>  PRT
<213>  Homo sapiens

<400>  46

Ile Asn Tyr Asn Gly Arg Asp Thr
1               5

<210>  47
<211>  17
<212>  PRT
<213>  Homo sapiens

<400>  47

Ala Ala Glu Arg Ala Ser Ile Cys Thr Leu Gln Ala Asp Pro Tyr Glu
1               5                   10                  15

Tyr

<210>  48
<211>  124
<212>  PRT
<213>  Homo sapiens

<400>  48

Gln Leu Gln Leu Val Glu Thr Gly Gly Gly Leu Val Gln Pro Gly Gly
1               5                   10                  15

Ser Leu Arg Leu Ser Cys Ala Ser Ser Ala Phe Thr Phe Gly Tyr Tyr
            20                  25                  30

Ala Ile Gly Trp Phe Arg Gln Ala Pro Gly Lys Glu Arg Glu Gly Ile
        35                  40                  45

Ala Cys Ile Asn Tyr Asn Gly Arg Asp Thr Asn Phe Ala Asp Ser Val
    50                  55                  60

Lys Gly Arg Phe Thr Ile Ser Arg Asp Asp Ala Lys Lys Thr Val Thr
65                  70                  75                  80

Leu Gln Met Asn Ser Leu Lys Pro Glu Asp Thr Ala Asn Tyr Tyr Cys
                85                  90                  95

Ala Ala Glu Arg Ala Ser Ile Cys Thr Leu Gln Ala Asp Pro Tyr Glu
            100                 105                 110

Tyr Trp Gly Gln Gly Thr His Val Thr Val Ser Ser
        115                 120


<210>  49
<211>  8
<212>  PRT
<213>  Homo sapiens

<400>  49

Gly Ile Leu Phe Asp Tyr Phe Ala
1               5


<210>  50
<211>  8
<212>  PRT
<213>  Homo sapiens

<400>  50

Ile Asn Tyr Asn Gly Arg Asp Thr
1               5

<210>  51
<211>  17
<212>  PRT
<213>  Homo sapiens

<400>  51

Ala Ala Glu Arg Ala Ser Ile Cys Thr Leu Gln Glu Asp Pro Tyr Glu
1               5                   10                  15

Tyr

<210>  52
<211>  124
<212>  PRT
<213>  Homo sapiens

<400>  52

Gln Leu Gln Leu Val Glu Thr Gly Gly Gly Leu Val Gln Pro Gly Gly
1               5                   10                  15

Ser Leu Thr Ile Ser Cys Ala Ser Ser Gly Ile Leu Phe Asp Tyr Phe
            20                  25                  30

Ala Ile Gly Trp Phe Arg Gln Ala Pro Gly Lys Glu Arg Glu Gly Ile
        35                  40                  45

Ser Cys Ile Asn Tyr Asn Gly Arg Asp Thr Asn Phe Ala Asp Ser Val
    50                  55                  60

Glu Gly Arg Phe Thr Ile Ser Arg Asp Asp Ala Lys Lys Thr Val His
65                  70                  75                  80

Leu Gln Met Lys Asp Leu Lys Pro Glu Asp Thr Gly Thr Tyr Phe Cys
                85                  90                  95

Ala Ala Glu Arg Ala Ser Ile Cys Thr Leu Gln Glu Asp Pro Tyr Glu
            100                 105                 110

Tyr Trp Gly Gln Gly Thr Gln Val Thr Val Ser Ser
        115                 120

<210>  53
<211>  8
<212>  PRT
<213>  Homo sapiens

<400>  53

Gly Asp Thr Leu Asp Tyr Tyr Ser

1               5


<210>  54
<211>  7
<212>  PRT
<213>  Homo sapiens

<400>  54

Met Thr Arg Asn Gly Arg Ile
1               5


<210>  55
<211>  19
<212>  PRT
<213>  Homo sapiens

<400>  55

Ala Ala Pro Glu Arg Phe Arg Leu Cys Thr Leu Arg Ser Leu Thr Asp
1               5                   10                  15


Phe Gly Ala


<210>  56
<211>  125
<212>  PRT
<213>  Homo sapiens

<400>  56

Gln Leu Gln Leu Val Glu Thr Gly Gly Gly Leu Val Gln Pro Gly Gly
1               5                   10                  15


Ser Leu Arg Leu Thr Cys Thr Ala Ser Gly Asp Thr Leu Asp Tyr Tyr
            20                  25                  30


Ser Ile Val Trp Phe Arg Arg Ser Pro Gly Lys Glu Arg Glu Gly Val
        35                  40                  45


Ser Cys Met Thr Arg Asn Gly Arg Ile Glu Tyr Gly Asp Ser Val Lys
    50                  55                  60


Gly Arg Phe Thr Ile Ser Arg Asp Asn Ala Lys Arg Thr Val Tyr Leu
65                  70                  75                  80


Gln Met Ser Asn Leu Lys Pro Glu Asp Thr Ala Val Tyr Tyr Cys Ala
                85                  90                  95


Ala Pro Glu Arg Phe Arg Leu Cys Thr Leu Arg Ser Leu Thr Asp Phe
            100                 105                 110

Gly Ala Trp Gly Gln Gly Thr Gln Val Thr Val Ser Ser
        115                 120                 125

<210>  57
<211>  8
<212>  PRT
<213>  Homo sapiens

<400>  57

Gly Phe Thr Leu Asp Tyr Tyr Ala
1               5

<210>  58
<211>  8
<212>  PRT
<213>  Homo sapiens

<400>  58

Ile Ser Ser Ser Gly Gly Ser Thr
1               5

<210>  59
<211>  19
<212>  PRT
<213>  Homo sapiens

<400>  59

Ala Ala Asp Arg Gly Ile His Ser Glu Cys Ser Ser Trp Ser Ala Asp
1               5                   10                  15

Phe Ala Ser

<210>  60
<211>  126
<212>  PRT
<213>  Homo sapiens

<400>  60

Gln Leu Gln Leu Val Glu Ser Gly Gly Gly Leu Val Gln Pro Gly Gly
1               5                   10                  15

Ser Leu Arg Leu Ser Cys Ala Ala Ser Gly Phe Thr Leu Asp Tyr Tyr
            20                  25                  30

Ala Ile Gly Trp Phe Arg Gln Ala Pro Gly Lys Glu Arg Glu Gly Val
        35                  40                  45

Ser Cys Ile Ser Ser Ser Gly Gly Ser Thr Asn Tyr Ala Asp Ser Val
    50                  55                  60

Glu Gly Arg Phe Thr Ile Ser Arg Asp Asn Ala Lys Asn Thr Val Tyr
65                  70                  75                  80

Leu Gln Ile Arg Ser Leu Lys Pro Glu Asp Thr Ala Val Tyr Tyr Cys
                85                  90                  95

Ala Ala Asp Arg Gly Ile His Ser Glu Cys Ser Ser Trp Ser Ala Asp
            100                 105                 110

Phe Ala Ser Trp Gly Gln Gly Thr Gln Val Thr Val Ser Ser
        115                 120                 125

<210>  61
<211>  8
<212>  PRT
<213>  Homo sapiens

<400>  61

Gly Phe Ile Leu Asp Tyr Tyr Asp
1               5

<210>  62
<211>  8
<212>  PRT
<213>  Homo sapiens

<400>  62

Ile Ser Ser Ser Gly Ser Thr Thr
1               5

<210>  63
<211>  19
<212>  PRT
<213>  Homo sapiens

<400>  63

Val Leu Asp Pro Pro Cys His Gln Ser Glu Ser Ala Leu His Tyr Glu
1               5                   10                  15

Arg Ala Phe

<210>  64
<211>  126
<212>  PRT
<213>  Homo sapiens

<400>  64

Gln Val Gln Leu Val Glu Ser Gly Gly Gly Leu Val Gln Pro Gly Gly

1               5                   10                  15

Ser Leu Arg Leu Ser Cys Val Leu Ser Gly Phe Ile Leu Asp Tyr Tyr
            20                  25                  30

Asp Ile Gly Trp Phe Arg Gln Ala Pro Gly Lys Glu Arg Glu Gly Val
        35                  40                  45

Ser Cys Ile Ser Ser Ser Gly Ser Thr Thr Tyr Tyr Gly Gly Ser Val
    50                  55                  60

Lys Gly Arg Phe Thr Ile Ser Arg Asp Asn Ala Lys Asn Thr Val Tyr
65                  70                  75                  80

Leu Glu Met Asn Ser Leu Lys Ala Glu Asp Thr Gly Arg Tyr Tyr Cys
                85                  90                  95

Val Leu Asp Pro Pro Cys His Gln Ser Glu Ser Ala Leu His Tyr Glu
            100                 105                 110

Arg Ala Phe Trp Gly Gln Gly Thr Gln Val Thr Val Ser Ser
        115                 120                 125

<210>  65
<211>  8
<212>  PRT
<213>  Homo sapiens

<400>  65

Gly Arg Thr Thr Arg Asn Asp Ala
1               5

<210>  66
<211>  8
<212>  PRT
<213>  Homo sapiens

<400>  66

Ile Ser Trp Asn Gly Arg Asn Thr
1               5

<210>  67
<211>  18
<212>  PRT
<213>  Homo sapiens

<400>  67

Ala Ile Gly Ile Asn Asp Tyr Gly Val Pro Ser Arg Pro Asn Met Tyr
1               5                   10                  15

Asp Tyr

<210> 68
<211> 125
<212> PRT
<213> Homo sapiens

<400> 68

Gln Leu Gln Leu Val Glu Ser Gly Gly Gly Leu Val Gln Ala Gly Gly
1               5                   10                  15

Ser Leu Lys Leu Ser Cys Ala Ala Ser Gly Arg Thr Thr Arg Asn Asp
            20                  25                  30

Ala Met Gly Trp Phe Arg Gln Ala Pro Gly Lys Glu Arg Glu Phe Val
        35                  40                  45

Ala Ala Ile Ser Trp Asn Gly Arg Asn Thr Ala Tyr Ala Asp Ser Ile
    50                  55                  60

Lys Gly Arg Phe Thr Ile Ser Arg Asp Asn Ala Lys Asn Thr Gly Tyr
65                  70                  75                  80

Leu Gln Met Asn Arg Leu Lys Pro Asp Asp Thr Ala Ala Tyr Tyr Cys
                85                  90                  95

Ala Ile Gly Ile Asn Asp Tyr Gly Val Pro Ser Arg Pro Asn Met Tyr
            100                 105                 110

Asp Tyr Trp Gly Gln Gly Thr Gln Val Thr Val Ser Ser
        115                 120                 125

<210> 69
<211> 8
<212> PRT
<213> Homo sapiens

<400> 69

Gly Phe Thr Leu Asp Tyr Tyr Ala
1               5

<210> 70
<211> 8
<212> PRT
<213> Homo sapiens

<400> 70

Ile Ser Ser Gly Gly Gly Tyr Thr

1               5


<210>  71
<211>  25
<212>  PRT
<213>  Homo sapiens

<400>  71

Ala Ala Asp Asp Pro His Pro Gln Gly Pro Asp Val Cys Ser Asp Ser
1               5                   10                  15


Trp Leu Glu Tyr Arg Tyr Arg Glu Leu
            20                  25


<210>  72
<211>  132
<212>  PRT
<213>  Homo sapiens

<400>  72

Gln Val Gln Leu Val Glu Thr Gly Gly Gly Leu Val Gln Pro Gly Gly
1               5                   10                  15


Ser Leu Arg Leu Ser Cys Ala Ala Pro Gly Phe Thr Leu Asp Tyr Tyr
            20                  25                  30


Ala Ile Gly Trp Phe Arg Gln Ala Pro Gly Ala Glu Arg Glu Gly Val
        35                  40                  45


Ser Cys Ile Ser Ser Gly Gly Gly Tyr Thr Lys Tyr Ala Asp Ser Val
    50                  55                  60


Lys Gly Arg Phe Thr Ile Ser Arg Asp Ser Ala Lys Asn Thr Val Tyr
65                  70                  75                  80


Leu Gln Met Asn Gly Leu Lys Pro Glu Asp Thr Ala Val Tyr Tyr Cys
                85                  90                  95


Ala Ala Asp Asp Pro His Pro Gln Gly Pro Asp Val Cys Ser Asp Ser
            100                 105                 110


Trp Leu Glu Tyr Arg Tyr Arg Glu Leu Trp Gly Gln Gly Thr Leu Val
        115                 120                 125


Thr Val Ser Ser
    130


<210>  73
<211>  8

<212>  PRT
<213>  Homo sapiens

<400>  73

Glu Ser Ile Leu Glu Asn Asn Asn
1               5


<210>  74
<211>  7
<212>  PRT
<213>  Homo sapiens

<400>  74

Ile Ser Asn Ser Gly Gly Ala
1               5


<210>  75
<211>  15
<212>  PRT
<213>  Homo sapiens

<400>  75

Leu Arg Ala His Ile Thr Pro Ser Arg Cys Leu Asp Phe Gly Ala
1               5                   10                  15


<210>  76
<211>  121
<212>  PRT
<213>  Homo sapiens

<400>  76

Gln Val Gln Leu Val Glu Thr Gly Gly Gly Leu Val Gln Pro Gly Gly
1               5                   10                  15


Ser Leu Thr Leu Ser Cys Val Ala Ser Glu Ser Ile Leu Glu Asn Asn
            20                  25                  30


Asn Val Gly Trp Phe Arg Gln Gly Pro Gly Glu Glu His Glu Ala Val
        35                  40                  45


Ser Cys Ile Ser Asn Ser Gly Gly Ala Phe Tyr Tyr Gly Ser Gly Glu
    50                  55                  60


Gly Arg Phe Thr Ile Ser Arg Asp Thr Ala Lys Asn Met Val Tyr Leu
65                  70                  75                  80


His Met Arg Asp Leu Lys His Glu Asp Ser Gly Ile Tyr His Cys Leu
                85                  90                  95


Arg Ala His Ile Thr Pro Ser Arg Cys Leu Asp Phe Gly Ala Val Gly

            100                 105                 110

Gln Gly Thr Gln Val Thr Val Ser Ser
        115                 120

<210> 77
<211> 8
<212> PRT
<213> Homo sapiens

<400> 77

Gly Phe Ser Leu Asp Tyr Tyr Ala
1               5

<210> 78
<211> 8
<212> PRT
<213> Homo sapiens

<400> 78

Ile Ser Ser Ser Gly Gly Ser Thr
1               5

<210> 79
<211> 21
<212> PRT
<213> Homo sapiens

<400> 79

Ala Ala Ala Pro Ser Thr Tyr Tyr Gly Gly Ser Tyr Tyr His Gln Cys
1               5                   10                  15

Leu Glu Tyr Asp Tyr
            20

<210> 80
<211> 128
<212> PRT
<213> Homo sapiens

<400> 80

Gln Val Gln Leu Val Glu Ser Gly Gly Gly Leu Val Gln Pro Gly Gly
1               5                   10                  15

Ser Leu Arg Leu Ser Cys Ala Ala Ser Gly Phe Ser Leu Asp Tyr Tyr
            20                  25                  30

Ala Ile Gly Trp Phe Arg Gln Ala Pro Gly Lys Glu Arg Glu Gly Val
        35                  40                  45

Ser Cys Ile Ser Ser Ser Gly Gly Ser Thr Asn Tyr Ala Asp Ser Val
    50                  55                  60

Lys Gly Arg Phe Thr Ile Ser Arg Asp Asn Ala Lys Asn Thr Val Tyr
65                  70                  75                  80

Leu Gln Met Asn Ser Leu Lys Pro Glu Asp Thr Ala Val Tyr Tyr Cys
                85                  90                  95

Ala Ala Ala Pro Ser Thr Tyr Tyr Gly Gly Ser Tyr Tyr His Gln Cys
            100                 105                 110

Leu Glu Tyr Asp Tyr Trp Gly Gln Gly Thr Gln Val Thr Val Ser Ser
        115                 120                 125

<210>  81
<211>  8
<212>  PRT
<213>  Homo sapiens

<400>  81

Gly Phe Thr Leu Asp Tyr Tyr Ala
1               5

<210>  82
<211>  8
<212>  PRT
<213>  Homo sapiens

<400>  82

Ile Ser Ser Ser Gly Gly Ser Thr
1               5

<210>  83
<211>  19
<212>  PRT
<213>  Homo sapiens

<400>  83

Ala Ala Gln Ser Gly Ser Tyr Tyr Trp Cys Gly Ser Asp Trp His Glu
1               5                   10                  15

Tyr Glu Tyr

<210>  84
<211>  126
<212>  PRT
<213>  Homo sapiens

<400>  84

Gln Leu Gln Leu Val Glu Ser Gly Gly Gly Leu Val Gln Pro Gly Gly
1               5                   10                  15

Ser Leu Arg Leu Ser Cys Ala Ala Ser Gly Phe Thr Leu Asp Tyr Tyr
            20                  25                  30

Ala Ile Gly Trp Phe Arg Gln Ala Pro Gly Lys Glu Arg Glu Gly Val
        35                  40                  45

Ser Cys Ile Ser Ser Ser Gly Gly Ser Thr His Phe Ala Asp Ser Val
    50                  55                  60

Lys Gly Arg Phe Thr Ile Ser Arg Asp Asn Ala Lys Asn Thr Val Tyr
65                  70                  75                  80

Leu Gln Met Asn Ser Leu Ile Pro Glu Asp Thr Ala Val Tyr Tyr Cys
                85                  90                  95

Ala Ala Gln Ser Gly Ser Tyr Tyr Trp Cys Gly Ser Asp Trp His Glu
            100                 105                 110

Tyr Glu Tyr Trp Gly Gln Gly Thr Gln Val Thr Val Ser Ser
        115                 120                 125

<210>  85
<211>  8
<212>  PRT
<213>  Homo sapiens

<400>  85

Gly Phe Thr Phe Ser Ser Tyr Ala
1               5

<210>  86
<211>  7
<212>  PRT
<213>  Homo sapiens

<400>  86

Ile Tyr Ser Asp Gly Ser Thr
1               5

<210>  87
<211>  15
<212>  PRT
<213>  Homo sapiens

<400>  87

Ala Thr Glu Gly Ser Leu Gly Gly Trp Gly Arg Asp Phe Gly Ser

1               5                   10                  15


<210>  88
<211>  121
<212>  PRT
<213>  Homo sapiens

<400>  88

Gln Val Gln Leu Val Glu Thr Gly Gly Gly Leu Val Gln Pro Gly Gly
1               5                   10                  15


Ser Leu Arg Leu Ser Cys Ala Ala Ser Gly Phe Thr Phe Ser Ser Tyr
            20                  25                  30


Ala Met Gly Trp Ala Arg Gln Val Pro Gly Lys Gly Leu Glu Trp Val
        35                  40                  45


Ser Tyr Ile Tyr Ser Asp Gly Ser Thr Glu Tyr Gln Asp Ser Val Lys
    50                  55                  60


Gly Arg Phe Thr Ile Ser Arg Asp Asn Ala Lys Ser Thr Val Tyr Leu
65                  70                  75                  80


Gln Met Asn Ser Leu Lys Pro Glu Asp Thr Ala Val Tyr Tyr Cys Ala
                85                  90                  95


Thr Glu Gly Ser Leu Gly Gly Trp Gly Arg Asp Phe Gly Ser Trp Gly
            100                 105                 110


Gln Gly Thr Gln Val Thr Val Ser Ser
        115                 120


<210>  89
<211>  8
<212>  PRT
<213>  Homo sapiens

<400>  89

Gly Phe Thr Leu Asp Tyr Tyr Ala
1               5


<210>  90
<211>  8
<212>  PRT
<213>  Homo sapiens

<400>  90

Ile Ser Ser Ser Gly Asp Ser Thr
1               5

<210>  91
<211>  19
<212>  PRT
<213>  Homo sapiens

<400>  91

Ala Ala Gln Ser Gly Ser Tyr Tyr Trp Cys Gly Ser Asp Trp His Glu
1               5                   10                  15

Tyr Asp Tyr

<210>  92
<211>  126
<212>  PRT
<213>  Homo sapiens

<400>  92

Gln Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Leu Asp Tyr Tyr
            20                  25                  30

Ala Ile Gly Trp Phe Arg Gln Ala Pro Gly Lys Glu Arg Glu Gly Val
        35                  40                  45

Ser Cys Ile Ser Ser Ser Gly Asp Ser Thr His Tyr Val Asp Ser Val
    50                  55                  60

Lys Gly Arg Phe Thr Ile Ser Arg Asp Asn Ala Lys Asn Thr Val Tyr
65                  70                  75                  80

Leu Gln Met Asn Ser Leu Lys Pro Glu Asp Thr Ala Val Tyr Tyr Cys
                85                  90                  95

Ala Ala Gln Ser Gly Ser Tyr Tyr Trp Cys Gly Ser Asp Trp His Glu
            100                 105                 110

Tyr Asp Tyr Arg Gly Gln Gly Thr Gln Val Thr Val Ser Ser
        115                 120                 125

<210>  93
<211>  1273
<212>  PRT
<213>  Homo sapiens

<400>  93

<210> 94
<211> 223
<212> PRT
<213> Homo sapiens

<400> 94

Arg Val Gln Pro Thr Glu Ser Ile Val Arg Phe Pro Asn Ile Thr Asn
1               5                   10                  15

Leu Cys Pro Phe Gly Glu Val Phe Asn Ala Thr Arg Phe Ala Ser Val
            20                  25                  30

Tyr Ala Trp Asn Arg Lys Arg Ile Ser Asn Cys Val Ala Asp Tyr Ser
        35                  40                  45

Val Leu Tyr Asn Ser Ala Ser Phe Ser Thr Phe Lys Cys Tyr Gly Val
    50                  55                  60

Ser Pro Thr Lys Leu Asn Asp Leu Cys Phe Thr Asn Val Tyr Ala Asp
65                  70                  75                  80

Ser Phe Val Ile Arg Gly Asp Glu Val Arg Gln Ile Ala Pro Gly Gln
                85                  90                  95

Thr Gly Lys Ile Ala Asp Tyr Asn Tyr Lys Leu Pro Asp Asp Phe Thr
            100                 105                 110

Gly Cys Val Ile Ala Trp Asn Ser Asn Asn Leu Asp Ser Lys Val Gly
        115                 120                 125

Gly Asn Tyr Asn Tyr Leu Tyr Arg Leu Phe Arg Lys Ser Asn Leu Lys
    130                 135                 140

Pro Phe Glu Arg Asp Ile Ser Thr Glu Ile Tyr Gln Ala Gly Ser Thr
145                 150                 155                 160

Pro Cys Asn Gly Val Glu Gly Phe Asn Cys Tyr Phe Pro Leu Gln Ser
                165                 170                 175

Tyr Gly Phe Gln Pro Thr Asn Gly Val Gly Tyr Gln Pro Tyr Arg Val
            180                 185                 190

Val Val Leu Ser Phe Glu Leu Leu His Ala Pro Ala Thr Val Cys Gly
        195                 200                 205

Pro Lys Lys Ser Thr Asn Leu Val Lys Asn Lys Cys Val Asn Phe
    210                 215                 220

<210>  95
<211>  72
<212>  PRT
<213>  Homo sapiens

<400>  95

Asn Ser Asn Asn Leu Asp Ser Lys Val Gly Gly Asn Tyr Asn Tyr Leu
1               5                   10                  15

Tyr Arg Leu Phe Arg Lys Ser Asn Leu Lys Pro Phe Glu Arg Asp Ile
            20                  25                  30

Ser Thr Glu Ile Tyr Gln Ala Gly Ser Thr Pro Cys Asn Gly Val Glu
        35                  40                  45

Gly Phe Asn Cys Tyr Phe Pro Leu Gln Ser Tyr Gly Phe Gln Pro Thr
    50                  55                  60

Asn Gly Val Gly Tyr Gln Pro Tyr
65                  70

## Description

### FIELD OF THE INVENTION

The invention pertains to protein binding compounds, such as antibodies and antibody like compounds, specifically targeting epitopes of the Corona virus spike (S) protein which were found to stabilize a three-dimensional conformation of the protein which reduces or inhibits the viral mechanisms necessary for host cell entry. The invention provides the protein binding compounds alone and in combination and multispecific constructs, as well as their use in the prevention, treatment, patient stratification and diagnosis of viral infections caused by a corona virus such as severe acute respiratory syndrome corona virus 2 (SARS CoV2).

### DESCRIPTION

The current pandemic of severe acute respiratory-syndrome coronavirus 2 (SARS-CoV-2) poses severe challenges to patients, health care systems, economy and public life. While efforts to develop vaccines are making rapid advances, safe and effective vaccines will not be available to the broader public in the near term. Furthermore, vaccines will likely not be suitable for immunocompromised patients, and cannot provide prophylaxis shortly before or after exposure. Neutralizing antibodies or related molecules offer great potential as immediate and direct-acting antiviral agents (1). Yet, they cannot be easily and economically produced in sufficient amounts for mass application, and cannot be readily modified to include multiple specificities without major costs in yield and quality. Conventional antibodies will also have to be assessed for antibody-dependent enhancement of infection (2). Variable domains of camelid heavy chain-only antibodies (VHHs) offer a unique opportunity to rapidly produce antiviral agents for passive immunization. Production in prokaryotic expression systems is cheap, easily upscaled, and importantly allows straight-forward protein engineering, including multivalent VHHs with enhanced functionalities (3). They have favourable biochemical properties, including high thermostability and deep tissue penetration. VHHs have been successfully developed to treat respiratory diseases by inhalator-based delivery (4).

The SARS-CoV-2 spike protein binds the cellular receptor ACE2 and catalyzes membrane fusion (5). The spike protein is the major target of neutralizing antibodies, where binding of antibodies to the receptor binding domain (RBD) typically competes with ACE2 binding and thereby hinders infection (6). Importantly, conformational flexibility of the spike protein allows each RBD to exist in two conformations: A down conformation that is thought to be less sensitive to antibody binding, and an up conformation that binds ACE2 (7, 8).

The inventors identified four distinct VHHs from immunized camelids that neutralize SARS-CoV-2, characterized their binding site to the SARS-CoV-2 RBD by x-ray crystallography, defined the effects of binding to trimeric spike by cryo-electron microscopy (EM), and used the findings to design biparatopic and other multivalent VHHs with substantial neutralizing potency and protection against viral escape mutants. Surprisingly, neutralization was achieved by at least two distinct mechanisms: Competition with ACE2 binding and irreversible, pre-mature activation of the fusion machinery. The inventors show that neutralizing VHHs can protect from infection in a mouse model of COVID-19, highlighting the clinical potential of the described set of VHHs.

Thus, it is an objection of the invention to provide new therapeutic strategies to tackle diseases caused by viral proteins which use a spike protein mediated entry mechanism into host cells during infection. In particular the objection of the invention is to provide novel compounds and compositions, as well as their use, for the treatment of diseases caused by an infection with SARS-CoV2.

### BRIEF DESCRIPTION OF THE INVENTION

The invention is grounded by the surprising finding that certain protein binding compounds such as antibodies which bind to specific epitopes on the Corona virus 2 spike protein stabilize a three-dimensional conformation of the spike protein that reduces or inhibits entry of a virus into a host cell. The new compounds of the invention are used alone or in combination for a treatment or prevention of an infection with a Corona virus and in particular SARS-CoV2, which is the causing virus of the Corona virus disease and pandemic of 2019 (COVID19).

Generally, and by way of brief description, the main aspects of the present invention can be described as follows:
In **a first aspect,** the invention pertains to a compound or composition, optionally for use in the treatment of disease caused by an infection with a corona virus, wherein the compound or composition comprises at least a first antigen binding domain binding to a first epitope of a spike protein of the corona virus, characterized in that a binding of the at least first antigen binding domain to the first epitope of the spike protein stabilizes and/or induces an up-conformation of a receptor binding domain (RBD) of the spike protein.

In **a second aspect,** the invention pertains to a protein binding compound (PBC), comprising a first antigen binding domain specifically binding to an epitope of a corona virus spike (S) protein, wherein the PBC when bound to the epitope of the spike protein an up-conformation of the spike protein is induced and/or stabilized

In **a third aspect,** the invention pertains to a protein binding compound (PBC), which is an isolated PBC comprising an amino acid sequence at least 80% identical to a sequence according to any one of SEQ ID NO: 1 to 92.

In **a fourth aspect,** the invention pertains to an isolated PBC, which competes with a PBC as recited in the second or third aspect for binding to the epitope of the spike protein.

In **a fifth aspect,** the invention pertains to a nucleic acid encoding for a PBC which is a protein or polypeptide according to any one of the preceding aspects.

In **a sixth aspect,** the invention pertains to a recombinant host cell, comprising a PBC or an isolated nucleic acid according to any one of the preceding aspects.

In **a seventh aspect,** the invention pertains to a pharmaceutical composition comprising a PBC recited in any of the second, third or fourth aspects, an isolated nucleic acid recited in the fifth aspect, a recombinant host cell recited in the sixth aspect, together with a pharmaceutically acceptable carrier and/or excipient.

In **an eighth aspect,** the invention pertains to a compound or composition of the preceding aspects for use in, or pertains to, a method of prevention or treatment of a disease.

In **a ninth aspect,** the invention pertains to a compound or composition of the preceding aspects for use in, or pertaining to, a method of diagnosing of a disease.

In **a tenths aspect,** the invention pertains to a method for identifying and/or characterising a compound suitable for the treatment of a disease, disorder or condition that is associated with, or caused by, a virus expressing a viral spike (S) protein, the method comprising the steps of:
- bringing into contact a candidate compound and a spike protein (or a variant or fragment thereof); and
- detecting and/or quantifying an induction and/or stabilization of an up-conformation of the spike protein (or the variant or fragment thereof) upon binding of the candidate compound to the spike protein (or the variant or fragment thereof),
wherein the induction and/or stabilization of an up-conformation of the spike protein (or the variant or fragment thereof) upon binding of the candidate compound to the spike protein (or the variant or fragment thereof)) compared to a control indicates that the compound is suitable for the treatment of a disease, disorder or condition that is associated with, or caused by, a virus expressing the spike protein.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **a first aspect,** the invention pertains to a compound or composition, optionally for use in the treatment of disease caused by an infection with a corona virus, wherein the compound or composition comprises at least a first antigen binding domain specifically binding to a first epitope of a spike protein of the corona virus, characterized in that a binding of the at least first antigen binding domain to the first epitope of the spike protein stabilizes and/or induces an up-conformation of a receptor binding domain (RBD) of the spike protein.

In the present disclosure all herein disclosed compounds and compositions in the individual sections, for examples relating to antibodies and antibody-like proteins, nucleic acids, host cells, pharmaceutical compositions, and any of the herein disclosed specific or general protein binding compounds are preferred entities as compounds and compositions of both the first aspect and the second aspects below. Further, such compounds and compositions are in particular useful in medicine, which is in detail disclosed herein below under the section "Treatment and Prevention of Diseases" herein elsewhere.

In **a second aspect,** the invention pertains to a protein binding compound (PBC), comprising a first antigen binding domain specifically binding to an epitope of a corona virus spike (S) protein, and, optionally, wherein the PBC is able to when bound to the epitope of the spike protein an up-conformation of the spike protein is induced and/or stabilized

As such, a PBC of the invention may be a compound of the first aspect or comprised in a composition of the first aspect.

The present invention revolves around compounds and compositions and their uses to tackle infections with a corona virus, as well as any adverse consequences of such an infection. A "corona virus" is a virus of the group of RNA viruses that cause mainly respiratory diseases in mammals and birds. Respiratory tract infections of corona viruses can range from mild to lethal, the latter causing severe acute respiratory syndrome (SARS), middle east respiratory syndrome (MERS), and COVID19. Corona viruses are enveloped viruses having an RNA genome. The envelope is covered with a so called "spike (S) protein", which is a component known to be essential for host cell entry of the corona virus. The present invention in preferred embodiments pertains to SARS-CoV2, the causing virus of the 2019 pandemic (see Hu, B., Guo, H., Zhou, P. et al. Nat Rev Microbiol (2020). https://doi.org/10.1038/s41579-020-00459-7).

A corona virus "spike protein" or "S protein" in context of the present invention shall refer to a corona virus S protein, preferably exclusively a SARS CoV2 S protein; optionally, for the context of the present invention in preferred embodiments, the S protein shall not include SARS CoV1 S protein. The spike protein exists as a homotrimer; each protomer consisting of a Si and a S2 subunit, where each resulting peplomers protrudes from the virus surface as spikes. SARS CoV2 S protein has preferably an amino acid shown in SEQ ID NO: 93. Since the SARS CoV2 might in the future mutate and produce additional S protein variants, such future variants shall be included by the term insofar binding epitopes of the S protein to which the compounds and compositions of the present inventions bind according to the present disclosure remain identical or at least similar to an extend that allows for a specific binding of such inventive compounds and compositions to the S protein. The amino acid sequence of the SARS CoV2 S protein may also be derived from the UniProt database in the version of October 25 2020, under the accession number: PoDTC2 (https://www.uniprot.org/uniprot/PoDTC2). Further referenced in context of the present invention is a receptor-binding domain (RBD) of the S protein, which in the case of SARS-CoV2 S protein is located between amino acids 319-541 of SARS-CoV2 S protein. Such RBD further comprises a receptor-binding motif, located between amino acids 437-508 of SARS-CoV2 S protein.

For the purposes of the invention, the term "three-dimensional conformation" or "three-dimensional structure", "conformation" of a protein, or protein multimer, preferably a spike protein, or of a receptor binding domain (RBD) of a spike protein, as described herein, means the spatial arrangement of the amino acid sequences of this protein, or protein multimer, preferably a spike protein, or of a receptor binding domain (RBD) of a spike protein, as described herein, is composed, taken together, when they interact with one another. A spike protein conformation may be preferably in the so called RBD "up" or "down" conformation, which translates in context to the spike trimer to a "one up", "two up" or "three up" conformation of the RBD. Such conformational states of the spike protein are described in references (7, 8). Hence, in preferred embodiments of the invention the compound or composition comprising such antigen binding domain of the invention is characterized in that a binding of the at least first antigen binding domain (e.g. of the PBC) to a trimeric spike protein stabilizes and/or induces a post fusion confirmation of the trimeric spike protein as a 2-up, more preferably a 3-up, conformation of the trimeric spike protein. Without being bound to theory, stabilizing (favouring or inducing) such post-fusion conformation of the spike protein using the compounds and compositions of the invention is assumed to block/inhibit the viral entry mechanism and thereby responsible for the herein disclosed therapeutic effect of the invention.

In context of the present invention an RBD comprises preferably an amino acid sequence that is at least 80%, 85%, 90%, 95%, 96%, 97%, 98%, 99%, or most preferably 100% identical to the amino acid sequence shown in SEQ ID NO: 94 (SARS-CoV2 S protein RBD amino acid sequence).

The term "antigen-binding domain" shall refer to any entity of a compound or composition, such as a PBC, that has capability to direct a specific binding of the compound or composition to a target epitope. Preferably such antigen binding domains in context of the present invention are proteinaceous and provided in context of a protein, such as an PBC, for example such as an antibody (or "antigen-binding fragment" thereof). Such antigen binding domains refers therefore specifically to one or more fragments of an antibody or antibody-like molecules, that retain the ability to specifically bind to an antigen (e.g., epitope of a spike protein). It has been shown that the antigen-binding function of an antibody can be performed by fragments of a full-length antibody. Examples of binding fragments encompassed within the term "antigen-binding domain" of an antibody include (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region; (iii) an Fd fragment consisting of the VH and CH1 domains; (iv) a Fv fragment consisting of the VL_ and VH domains of a single arm of an antibody, (v) a single domain or dAb fragment (Ward et al., (1989) Nature 341 :544-546), which consists of a VH domain; and (vi) an isolated complementarity determining region (CDR) or (vii) a combination of two or more isolated CDRs which may optionally be joined by a synthetic linker. Furthermore, although the two domains of the Fv fragment, Vi_ and VH, are coded for by separate genes, they can be joined, using recombinant methods, by a synthetic linker that enables them to be made as a single protein chain in which the Vi_ and VH regions pair to form monovalent molecules (known as single chain Fv (scFv); see e.g., Bird et al. (1988) Science 242:423-426; and Huston et al. (1988) Proc. Natl. Acad. Sci. USA 85:5879-5883). Such single chain antibodies are also intended to be encompassed within the term "antigen-binding domain" of an antibody. Most preferably such antigen binding domains are provided in the form of an antigen binding domain of a V_{H}H, or an antigen binding fragment thereof, as described herein below.

A PBC, preferably an "antigen binding protein" ("ABP"), comprising such antigen binding domain of the invention, as used herein means a protein that specifically binds to a target antigen, such as to one or more epitope(s) displayed by or present on a target antigen. The antigen of the PBCs of the invention is a corona virus spike protein, preferably of SARS-CoV2, or an orthologue (or paralogue) or other variant thereof; and the PBC can, optionally bind to one or more extracellular domains of said S protein or variant (such as the epitope(s) can be displayed by or present on one or more domains of the S protein, or a multimer thereof. Typically, an antigen binding protein is an antibody (such as an V_{H}H) (or a fragment thereof), however other forms of antigen binding protein are also envisioned by the invention. For example, the PBC may be another (non-antibody) receptor protein derived from small and robust non-immunoglobulin "scaffolds", such as those equipped with binding functions for example by using methods of combinatorial protein design (Gebauer & Skerra, 2009; Curr Opin Chem Biol, 13:245). Particular examples of such non-antibody PBCs include: Affibody molecules based on the Z domain of Protein A (Nygren, 2008; FEBS J 275:2668); Affilins based on gamma-B crystalline and/or ubiquitin (Ebersbach et al, 2007; J Mo Biol, 372:172); Affimers based on cystatin (Johnson et al, 2012; Anal Chem 84:6553); Affitins based on Sac7d from Sulfolobus acidcaldarius (Krehenbrink et al, 2008; J Mol Biol 383:1058); Alphabodies based on a triple helix coiled coil (Desmet et al, 2014; Nature Comms 5:5237); Anticalins based on lipocalins (Skerra, 2008; FEBS J 275:2677); Avimers based on A domains of various membrane receptors (Silverman et al, 2005; Nat Biotechnol 23:1556); DARPins based on an ankyrin repeat motif (Strumpp et al, 2008; Drug Discov Today, 13:695); Fynomers based on an SH₃ domain of Fyn (Grabulovski et al, 2007; J Biol Chem 282:3196); Kunitz domain peptides based on Kunitz domains of various protease inhibitors (Nixon et al, Curr opin Drug Discov Devel, 9:261) and Centyrins and Monobodies based on a 10th type III domain of fibronectin (Diem et al., 2014; Protein Eng Des Sel 27:419 doi: 10-1093/protein/gzuo16; Koide & Koide, 2007; Methods Mol Biol 352:95).

The term "epitope" includes any determinant capable of being bound by an antigen binding protein, such as an antibody. An epitope is a region of an antigen, such as a spike protein, that is bound by an antigen binding protein that targets that antigen, and when the antigen is a protein, includes specific amino acids that bind the antigen binding protein (such as via an antigen binding domain of said protein). Epitope determinants can include chemically active surface groupings of molecules such as amino acids, sugar side chains, phosphoryl or sulfonyl groups, and can have specific three dimensional structural characteristics, and/or specific charge characteristics. Generally, antigen binding proteins specific for a particular target antigen will preferentially recognise an epitope on the target antigen in a complex mixture of proteins and/or macromolecules.

The term "identity" refers to a relationship between the sequences of two or more polypeptide molecules or two or more nucleic acid molecules, as determined by aligning and comparing the sequences. "Percent identity" means the percent of identical residues between the amino acids or nucleotides in the compared molecules and is calculated based on the size of the smallest of the molecules being compared. For these calculations, gaps in alignments (if any) are preferably addressed by a particular mathematical model or computer program (i.e., an "algorithm"). Methods that can be used to calculate the identity of the aligned nucleic acids or polypeptides include those described in Computational Molecular Biology, (Lesk, A. M., ed.), 1988, New York: Oxford University Press; Biocomputing Informatics and Genome Projects, (Smith, D. W., ed.), 1993, New York: Academic Press; Computer Analysis of Sequence Data, Part I, (Griffin, A. M., and Griffin, H. G., eds.), 1994, New Jersey: Humana Press; von Heinje, G., 1987, Sequence Analysis in Molecular Biology, New York: Academic Press; Sequence Analysis Primer, (Gribskov, M. and Devereux, J., eds.), 1991, New York: M. Stockton Press; and Carillo et al., 1988, SIAM J. Applied Math. 48:1073.

In calculating percent identity, the sequences being compared are typically aligned in a way that gives the largest match between the sequences. One example of a computer program that can be used to determine percent identity is the GCG program package, which includes GAP (Devereux et al., 1984, Nucl. Acid Res. 12:387; Genetics Computer Group, University of Wisconsin, Madison, WI). The computer algorithm GAP is used to align the two polypeptides or polynucleotides for which the percent sequence identity is to be determined. The sequences are aligned for optimal matching of their respective amino acid or nucleotide (the "matched span", as determined by the algorithm). A gap opening penalty (which is calculated as 3x the average diagonal, wherein the "average diagonal" is the average of the diagonal of the comparison matrix being used; the "diagonal" is the score or number assigned to each perfect amino acid match by the particular comparison matrix) and a gap extension penalty (which is usually 1/10 times the gap opening penalty), as well as a comparison matrix such as PAM 250 or BLOSUM 62 are used in conjunction with the algorithm.

A standard comparison matrix (see, Dayhoff et al., 1978, Atlas of Protein Sequence and Structure 5:345-352 for the PAM 250 comparison matrix; Henikoff et al., 1992, Proc. Natl. Acad. Sci. U.S.A. 89:10915-10919 for the BLOSUM 62 comparison matrix) may also be used by the algorithm.

Examples of parameters that can be employed in determining percent identity for polypeptides or nucleotide sequences using the GAP program are the following: (i) Algorithm: Needleman et al., 1970, J. Mol. Biol. 48:443-453; (ii) Comparison matrix: BLOSUM 62 from Henikoff et al., 1992, supra; (iii) Gap Penalty: 12 (but with no penalty for end gaps); (iv) Gap Length Penalty: 4; (v) Threshold of Similarity: o.

A preferred method of determining similarity between a protein or nucleic acid is that provided by the Blast searches supported at Uniprot supra (e.g., http://www.uniprot.org/uniprot/Q5DX21); in particular for amino acid identity, those using the following parameters: Program: blastp; Matrix: blosum62; Threshold: 10; Filtered: false; Gapped: true; Maximum number of hits reported: 250.

Certain alignment schemes for aligning two amino acid sequences may result in matching of only a short region of the two sequences, and this small aligned region may have very high sequence identity even though there is no significant relationship between the two full-length sequences. Accordingly, the selected alignment method (GAP program) can be adjusted if so desired to result in an alignment that spans at least about 10, 15, 20, 25, 30, 35, 40, 45, 50 or other number of contiguous amino acids of the target polypeptide or region thereof.

In certain embodiments of the invention the herein disclosed compounds or compositions comprise a second antigen binding domain binding to a second epitope of the spike protein, wherein the first and second epitope are (i) identical, (ii) overlapping, or (iii) distinct. Accordingly, the second antigen binding domain may, in the case of (i), be identical, in the cases of (ii) and (iii) are not identical. The first antigen binding domain and the second antigen binding domain may be comprised within one protein complex or are provided in separate protein complexes. In context of the present invention such combinations of antigen binding domains either in one complex or in separate complexes but in one composition (e.g. as two distinct antigen binding proteins), was found to significantly increase therapeutic efficacy against viral infections. A binding of the second antigen binding domain to the second epitope of the spike protein in accordance with the invention may (further) stabilize and/or induce a post fusion conformation of the trimeric spike protein and/or an up-conformation of a receptor binding domain (RBD) of the spike protein, and preferably wherein a binding of the second antigen binding domain to a trimeric spike protein stabilizes and/or induces a 2-up, more preferably a 3-up, conformation of the trimeric spike protein.

In embodiments wherein the first and the second antigen binding domains are covalently connected within one molecule, such connection is realized by using a linker moiety that covalently or non-covalently connects the first and the second antigen binding domains. Such linker moiety may be any molecule suitable to stably link the first and the second antigen binding domain to each other. The linker of the invention may be a synthetic amino acid sequence, or a naturally occurring polypeptide sequence, such as a polypeptide having the function of a hinge region. Such linker polypeptides are well known in the art (see, for example, Holliger, P. et al. (1993) Proc. Natl. Acad. Sci. USA 90:6444-6448; Poljak, R J et al. (1994) Structure 2:1121-1123)). Exemplary linkers include simple peptide linkers, or complex protein domain linkers, such as antibody constant domains (such as human CHi and/or CH3). The peptide linkers utilized in certain embodiments of the invention are used to link the antigen binding domains of two or more antibodies, antigen-binding sites, and/or antibody fragments comprising the different antigen-binding sites (e.g. scFv, full length antibody or V_{H}H) together to form a multispecific antibody according to the invention. Preferably, the peptide linkers are glycine-rich peptides with at least 5 amino acids, preferably of at least 10 amino acids, more preferably between 10 and 50 amino acids. In some embodiments of the present invention, said glycine-rich peptide linker is (GₓS)ₙ with G=glycine, S=serine, (x=3 and n=3, 4, 5 or 6) or (x=4 and n=2, 3, 4 or 5).

As used herein, the term "specifically binds" refers to a non-random binding reaction between two molecules, such as a reaction between an antigen binding domain, or the PBC in which such domain is comprised, and the antigen to which it is directed. In certain embodiments, a PBC that specifically binds to an antigen (or an antibody that is specific for an antigen) means that the antibody binds to the antigen with an affinity (K_{D}) of less than about 10⁻⁵ M, for example less than about 10⁻⁶ M, 10⁻⁷ M, 10⁻⁸ M, 10⁻⁹ M or 10⁻¹⁰ M or lesser. In the present invention, the term "KD" refers to the dissociation equilibrium constant of a particular antibody-antigen interaction, which is used to describe the binding affinity between an antibody and an antigen. The smaller the dissociation equilibrium constant, the tighter the antibody-antigen binding and the higher the affinity between the antibody and the antigen. Typically, an antibody (e.g., a VHH of the present invention) binds to an antigen (e.g., S protein) with a dissociation equilibrium constant (K_{D}) of less than about 10⁻⁵ M, for example less than about 10⁻⁶ M, 10⁻⁷ M, 5x10⁻⁸ M or less affinity. The term "KD", as used herein, is intended to refer to the dissociation constant, which is obtained from the ratio of Kd to Ka (i. e., Kd/Ka) and is expressed as a molar concentration (M). KD values for antibodies can be determined using methods well established in the art such as surface plasmon resonance (BIAcore^{®}), ELISA and KINEXA). A preferred method for determining the KD of an antibody is by using surface plasmon resonance, preferably using a biosensor system such as a BIAcore^{®} system or by ELISA. "Ka" (or "K-assoc"), as used herein, refers broadly to the association rate of a particular antibody-antigen interaction, whereas the term "Kd" (or "K-diss"), as used herein, refers to the dissociation rate of a particular antibody-antigen interaction.

A PBC of the invention in certain embodiments is a compound selected from a polypeptide, peptide, glycoprotein, a peptidomimetic, ubiquitin-like protein, an antibody or antibody-like molecule; a nucleic acid such as a DNA or RNA, including variants or derivatives thereof such as a peptide nucleic acid (PNA); a carbohydrate such as a polysaccharide or oligosaccharide and the like, including variants or derivatives thereof; a lipid such as a fatty acid and the like, including variants or derivatives thereof; or a small organic molecules including but not limited to small molecule ligands, small cell-permeable molecules, and peptidomimetic compounds.

In further particularly preferred embodiments of the invention, the first antigen binding domain specifically binds to an epitope of the S protein which involves at least one, preferably at least 2, 3, 4, 5, 6, 7, 8, 9, 10, or most preferably all of the amino acid residues of the S protein selected from E484, T470, I468, F490, Y351, L492, L455, Y453, Q493, S494, L452, R403, Y449, G447, G446 and K444 (as compared to SEQ ID NO: 93). In further preferred embodiments where the invention involves a second antigen binding domain such second antigen binding domain may not bind to such afore selected epitope.

### Antibodies and Antibody-like Proteins

Compounds and compositions in accordance with the present invention are preferably protein binding compounds (PBC) in accordance with the second aspect. In preferred embodiments of the invention such PBC can preferentially comprise at least one complementarity determining region (CDR), such as one from an antibody (in particular from a V_{H}H), and in particular embodiments the PBC can comprise a CDR having an amino acid sequence with at least 80%, 85%, 90% or 95% sequence identity to (preferably, at least 90% sequence identity to), or having no more than three or two, preferably no more than one amino acid substitution(s), deletion(s) or insertion(s) compared to, a CDR sequence set forth in Table 1 herein.

The term "complementarity determining region" (or "CDR" or "hypervariable region"), as used herein, refers broadly to one or more of the hyper-variable or complementarily determining regions (CDRs) found in the variable regions of light or heavy chains of an antibody. See, for example: "IMGT", Lefranc et al, 20003, Dev Comp Immunol 27:55; Honegger & Plückthun, 2001, J Mol Biol 309:657, Abhinandan & Martin, 2008, Mol Immunol 45:3832, Kabat, et al. (1987): Sequences of Proteins of Immunological Interest National Institutes of Health, Bethesda, Md. These expressions include the hypervariable regions as defined by Kabat et al (1983) Sequences of Proteins of Immunological Interest, US Dept of Health and Human Services, or the hypervariable loops in 3-dimensional structures of antibodies (Chothia and Lesk, 1987; J Mol Biol 196:901). The CDRs in each chain are held in close proximity by framework regions and, with the CDRs from the other chain, contribute to the formation of the antigen-binding site. Within the CDRs there are select amino acids that have been described as the selectivity determining regions (SDRs) which represent the critical contact residues used by the CDR in the antibody-antigen interaction. (Kashmiri, 2005; Methods 36:25).

As described above, in particular embodiments of the invention, a PBC can comprise at least one complementarity determining region (CDR). In certain of such embodiments, a PBC of the invention comprises at least one complementarity determining region 3 (CDR3), such as one having an amino acid sequence with at least 80%, 85%, 90% or 95% (preferably at least 90%) sequence identity to, or having no more than three or two, preferably no more than one amino acid substitution(s), deletion(s) or insertion(s) compared to, a sequence selected from those heavy and light chain CDR3 sequences shown in Table 1, e.g. an amino acid sequence selected from SEQ ID Nos 3, 7, 11, 15, 19, 23, 27, 31, 35, 39, 43, 47, 51, 55, 59, 63, 67, 71, 75, 79, 83, 87, and 91, or having an amino acid sequence having no more than three, preferably no more than two or one, amino acid substitution(s), deletion(s), insertion(s) or addition(s) compared to these sequences.

A PBC of the invention may, alternatively or as well as a CDR3 sequence, comprise at least one CDR1, and/or at least one CDR2 (such as one from an antibody, in particular from a V_{H}H). Preferably, a PBC of the invention comprises at least one such CDR3, as well as at least one such CDRi and at least one such CDR2, more preferably where each of such CDRs having an amino acid sequence with at least 80%, 85%, 90% or 95% (preferably at least 90%) sequence identity to, or having no more than three or two, preferably no more than one amino acid substitution(s), deletion(s) or insertion(s) compared to, a sequence selected from the corresponding (heavy and light chain) CDR1, CDR2 and CDR3 sequences shown in Table 1.

In particular embodiments, a PBC of the invention can be an antibody (such as an VHH) or an antigen binding fragment thereof.

As used herein, the term "antibody" may be understood in the broadest sense as any immunoglobulin (Ig) that enables binding to its epitope. An antibody as such is a species of a PBC. Full length "antibodies" or "immunoglobulins" are generally heterotetrameric glycoproteins of about 150 kDa, composed of two identical light and two identical heavy chains. Each light chain is linked to a heavy chain by one covalent disulphide bond, while the number of disulphide linkages varies between the heavy chain of different immunoglobulin isotypes. Each heavy and light chain also has regularly spaced intrachain disulphide bridges. Each heavy chain has an amino terminal variable domain (VH) followed by three carboxy terminal constant domains (CH). Each light chain has a variable N-terminal domain (VL) and a single C-terminal constant domain (CL). The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FRi, CDR1, FR2, CDR2, FR3, CDR3, FR4. The variable regions of the heavy and light chains contain a binding domain that interacts with an antigen. The constant regions of the antibodies may mediate the binding of the immunoglobulin to cells or factors, including various cells of the immune system (e.g., effector cells) and the first component (C1q) of the classical complement system.

A preferred form of an antibody of the invention includes a heavy-chain antibody or V_{H}H, being those which consist only of heavy chains and lack the two light chains usually found in antibodies. Heavy-chain antibodies include the hcIgG (IgG-like) antibodies of camelids such as dromedaries, camels, llamas and alpacas, and the IgNAR antibodies of cartilaginous fishes (for example sharks). And yet other forms of antibodies include single-domain antibodies (sdAb, called Nanobody by Ablynx, the developer) being an antibody fragment consisting of a single monomeric variable antibody domain. Single-domain antibodies are typically produced from heavy-chain antibodies, but may also be derived from conventional antibodies. In preferred embodiments the antibody of the invention is a VHH comprising an antibody heavy chain variable region, or an antigen binding fragment thereof, and not comprising an antibody light chain variable region, nor an antigen binding fragment thereof.

Antibodies (or those from which fragments thereof can be isolated) can include, for instance, chimeric, humanized, (fully) human, or hybrid antibodies with dual or multiple antigen or epitope specificities, antibody fragments and antibody sub-fragments, e.g., Fab, Fab' or F(ab')2 fragments, single chain antibodies (scFv) and the like (described below), including hybrid fragments of any immunoglobulin or any natural, synthetic or genetically engineered protein that acts like an antibody by binding to a specific antigen to form a complex.

Accordingly, in certain embodiments a PBC of the invention can comprise an antibody heavy chain, or an antigen binding fragment thereof, and/or an antibody light chain, or an antigen binding fragment thereof.

In further embodiments, a PBC of the invention can comprise an antibody heavy chain variable region, or an antigen binding fragment thereof, and/or an antibody light chain variable region, or an antigen binding fragment thereof, and in yet further embodiments, a PBC of the invention can comprise an antibody heavy chain variable region CDR1, CDR2, and CDR3, and/or an antibody light chain variable region CDR1, CDR2, and CDR3.

In particular embodiments of the invention, when the PBC comprises an antibody heavy chain sequence, or the fragment thereof, comprising a CDR1 having at least 80% sequence identity to an amino acid sequence selected from SEQ ID Nos. 1, 5, 9, 13, 17, 21, 25, 29, 33, 37, 41, 45, 49, 53, 57, 61, 65, 69, 73, 77, 81, 85 and 89, or having no more than three, preferably no more than two or one, amino acid substitution(s), deletion(s), insertion(s) or addition(s) compared to these sequences; and/or a CDR2 having at 80% sequence identity to an amino acid sequence selected from SEQ ID Nos. 2, 6, 10, 14, 18, 22, 26, 30, 34, 38, 42, 46, 50, 54, 58, 62, 66, 70, 74, 78, 82, 86 and 90, or having no more than three, preferably no more than two or one, amino acid substitution(s), deletion(s), insertion(s) or addition(s) compared to these sequences.

In preferred of such embodiments, the PBC may be an antibody, preferably a V_{H}H, or an antigen binding fragment thereof, composed of the first antigen binding domain comprising an antibody heavy chain sequence comprising CDR1 to CDR3 sequences in the following combination:

| | **Heavy Chain CDR1 to CDR3 (SEQ ID NO)** | | |
|---|---|---|---|
| **V_{H}H -A** | 1 | 2 | 3 |
| **V_{H}H -B** | 5 | 6 | 7 |
| **V_{H}H -C** | 9 | 10 | 11 |
| **V_{H}H -D** | 13 | 14 | 15 |
| **V_{H}H -E** | 17 | 18 | 19 |
| **V_{H}H -F** | 21 | 22 | 23 |
| **V_{H}H -G** | 25 | 26 | 27 |
| **V_{H}H -H** | 29 | 30 | 31 |
| **V_{H}H -I** | 33 | 34 | 35 |
| **V_{H}H -J** | 37 | 38 | 39 |
| **V_{H}H -K** | 41 | 42 | 43 |
| **V_{H}H -L** | 45 | 46 | 47 |
| **V_{H}H -M** | 49 | 50 | 51 |
| **V_{H}H -N** | 53 | 54 | 55 |
| **V_{H}H -O** | 57 | 58 | 59 |
| **V_{H}H -P** | 61 | 62 | 63 |
| **V_{H}H -Q** | 65 | 66 | 67 |
| **V_{H}H -R** | 69 | 70 | 71 |
| **V_{H}H -S** | 73 | 74 | 75 |
| **V_{H}H -T** | 77 | 78 | 79 |
| **V_{H}H -U** | 81 | 82 | 83 |
| **V_{H}H -V** | 85 | 86 | 87 |
| **V_{H}H-W** | 89 | 90 | 91 |

in each case independently, optionally with not more than three or two, preferably one, amino acid substitution(s), insertion(s) or deletion(s) compared to these sequences.

Preferred embodiments and aspects of the present invention pertain to the antibodies, or the herein described variants thereof, designated as V_{H}H-E, V_{H}H-N, V_{H}H-U, V_{H}H-V, and V_{H}H-W. Particular, referring to the first and second aspects, the such first antigen binding domain is preferably derived from V_{H}H-E, or at least binds specifically to the same or overlapping epitope, and wherein the optional second antigen binding domain binds specifically to the same, or overlapping, epitope of any of V_{H}H-U, V_{H}H-V, and V_{H}H-W.

In other embodiments of the present invention, an PBC of the invention can comprise an antibody heavy chain sequence having at least 80%, 85%, 90%; or 95% (preferably at least 90%) sequence identity to, or having no more than ten, nine, eight, seven, six, five, four, three, two or one, preferably no more than three, two or one amino acid substitution(s), deletion(s) or insertion(s) compared to, a sequence selected from SEQ ID NOs. 4, 8, 12, 16, 20, 24, 28, 32, 36, 40, 44, 48, 52, 56, 60, 64, 68, 72, 76, 80, 84, 88, or 92 (eg, a full length sequence disclosed in table 1 - preferred are sequences 20, 56, 84, 88, or 92).

As mentioned above, one preferred embodiment of the invention pertains to multimeric PBCs, comprising more than one (or more) antigen binding domains, preferably wherein such antigen binding domains may bind different antigenic epitopes, such as different epitopes on the S protein. Such multimeric PBCs of the invention are preferably bispecific molecules, for examples having a first and a second antigen binding site which are derived from any combination of binding sites (and their herein disclosed variants) of the VHH disclosed in table 1. Preferred combinations for such multi- or bispecific PBCs are derived from a combination of one or more antigen binding domains of V_{H}H-E, V_{H}H-N, V_{H}H-U, V_{H}H-V, and V_{H}H-W. Such preferred PBCs comprise a first antigen binding domain derived from V_{H}H-E, or any of its fragments or variants, and a second antigen binding domain derived from any of the other VHH of table 1, preferably of an antigen binding domain selected from any one of V_{H}H-N, V_{H}H-U, V_{H}H-V, and V_{H}H-W.

The present invention therefore, in some particular preferred aspects and embodiments pertains to a PBC, comprising a first and a second antigen binding domain, wherein the first antigen binding domain comprises optionally a CDRi having an amino acid sequence shown in SEQ ID NO: 17, optionally a CDR2 having an amino acid sequence shown in SEQ ID NO: 18, and a CDR3 having an amino acid sequence shown in SEQ ID NO: 19; in each case independently, optionally with not more than three or two, preferably one, amino acid substitution(s), insertion(s) or deletion(s) compared to these sequences; and preferably wherein the first antigen binding domain comprises said CDR1, CDR2 and CDR3; and wherein said second antigen binding domain comprises:
(a) a CDR1 having an amino acid sequence shown in SEQ ID NO: 53, optionally a CDR2 having an amino acid sequence shown in SEQ ID NO: 54, and a CDR3 having an amino acid sequence shown in SEQ ID NO: 55; in each case independently, optionally with not more than three or two, preferably one, amino acid substitution(s), insertion(s) or deletion(s) compared to these sequences; and preferably wherein the first antigen binding domain comprises said CDR1, CDR2 and CDR3; or
(b) a CDR1 having an amino acid sequence shown in SEQ ID NO: 81, optionally a CDR2 having an amino acid sequence shown in SEQ ID NO: 82, and a CDR3 having an amino acid sequence shown in SEQ ID NO: 83; in each case independently, optionally with not more than three or two, preferably one, amino acid substitution(s), insertion(s) or deletion(s) compared to these sequences; and preferably wherein the first antigen binding domain comprises said CDR1, CDR2 and CDR3; or
(c) a CDR1 having an amino acid sequence shown in SEQ ID NO: 85, optionally a CDR2 having an amino acid sequence shown in SEQ ID NO: 86, and a CDR3 having an amino acid sequence shown in SEQ ID NO: 87; in each case independently, optionally with not more than three or two, preferably one, amino acid substitution(s), insertion(s) or deletion(s) compared to these sequences; and preferably wherein the first antigen binding domain comprises said CDR1, CDR2 and CDR3; or
(d) a CDR1 having an amino acid sequence shown in SEQ ID NO: 89, optionally a CDR2 having an amino acid sequence shown in SEQ ID NO: 90, and a CDR3 having an amino acid sequence shown in SEQ ID NO: 91; in each case independently, optionally with not more than three or two, preferably one, amino acid substitution(s), insertion(s) or deletion(s) compared to these sequences; and preferably wherein the first antigen binding domain comprises said CDR1, CDR2 and CDR3.

The antigen binding domains of such PBC may be connected via a linker as described herein elsewhere. Further the first and/or second antigen binding domain is monovalent or divalent or multivalent.

In certain embodiments, the PBC of the invention may have an additional further antigen binding domain, specifically binding to for example albumin. Such further antigen binding domain may be provided as an antibody specifically binding albumin.

In view of the structural information of the binding of the VHH of the invention to the spike protein in particular the following amino acid positions were identified in the CDR regions of the VHH to be of less relevance for antibody binding.

A PBC of the invention which is a variant of V_{H}H-E may have preferably an amino acid substitution, addition or deletion in any of the following amino acid positions of S105; G106, S53, S54, and/or D55 SEQ ID NO: 20. According to the structural data obtained in context of the invention, the selected amino acid positions are not relevant for V_{H}H antigen binding.

A PBC of the invention which is a variant of V_{H}H-U/W may have preferably an amino acid substitution, addition or deletion in any of the following amino acid positions G107; S108, G55, G56, and/or S57 of SEQ ID NO: 84 or 92 (respectively). According to the structural data obtained in context of the invention, the selected amino acid positions are not relevant for V_{H}H antigen binding.

A PBC of the invention which is a variant of V_{H}H-V may have preferably an amino acid substitution, addition or deletion in any of the following amino acid positions A96; T97, S53, D54 and/or G55 of SEQ ID NO: 88 (respectively). According to the structural data obtained in context of the invention, the selected amino acid positions are not relevant for V_{H}H antigen binding.

Preferred for the such variants are amino acid substitutions, preferably of amino acid residues with similar properties such as exchanges between S - T, G - A, and D - E.

The term "overlapping epitope" in the context of the present specification refers to an epitope that is partially identical to a certain epitope.

In **a third aspect,** the invention pertains to a protein binding compound (PBC), which is an isolated PBC comprising an amino acid sequence at least 80% identical to a sequence according to any one of SEQ ID NO: 1 to 92.

In **a fourth aspect,** the invention pertains to an isolated PBC, which competes with a PBC as recited in the second or third aspect for binding to the epitope of the spike protein.

The term "compete" when used in the context of PBCs (e.g., antigen binding domain) that compete for binding for the same antigen (or epitope displayed by such antigen) means competition between PBCs as may be determined by an assay in which the PBC (e.g., antibody or binding fragment thereof) being tested prevents or inhibits (e.g., reduces) binding of a reference PBC (e.g., a ligand, or a reference antibody) to a common antigen (e.g., SARS- CoV2 Spike protein).

In preferred embodiments of all PBCs of the invention, the PBC is isolated and/or substantially pure.

The term "isolated" as used herein in the context of a protein, such as a PBC (an example of which could be an antibody), refers to a protein that is purified from proteins or polypeptides or other contaminants that would interfere with its therapeutic, diagnostic, prophylactic, research or other use. An isolated PBC according to the invention may be a recombinant, synthetic or modified (non-natural) PBC. The term "isolated" as used herein in the context of a nucleic acid or cells refers to a nucleic acid or cells that is/are purified from DNA, RNA, proteins or polypeptides or other contaminants (such as other cells) that would interfere with its therapeutic, diagnostic, prophylactic, research or other use, or it refers to a recombinant, synthetic or modified (non-natural) nucleic acid. Preferably an isolated PBC or nucleic acid or cells is/are substantially pure. In this context, a "recombinant" protein or nucleic acid is one made using recombinant techniques. Methods and techniques for the production of recombinant nucleic acids and proteins are well known in the art.

In some embodiments, an PBC of the invention may bind to (e.g., via one or more epitope(s) displayed by one or more EC domain(s) of) a corona virus S protein or a paralogue, orthologue or other variant thereof with a K_{D} that is less than 1000 nM, such as less than about 100 nM, or in particular, less than about 30 nM). In a preferred embodiment, the PBC of the invention will bind (e.g. said epitope(s) of) said S protein or variant with a K_{D} that is less than 10 nM, such as an K_{D} of about 2 nM.

In an alternative, and preferred, embodiment of all PBCs of the invention, the PBC is an antibody or an antigen binding fragment thereof, and the antibody is a monoclonal antibody, or wherein the antigen binding fragment is a fragment of a monoclonal antibody. Such antibodies may also non V_{H}H antibodies.

The term "monoclonal antibody" or "mAb" as used herein refers to an antibody obtained from a population of substantially identical antibodies based on their amino acid sequence. Monoclonal antibodies are typically highly specific. Furthermore, in contrast to conventional (polyclonal) antibody preparations which typically include different antibodies directed against different determinants (e.g. epitopes) of an antigen, each mAb is typically directed against a single determinant on the antigen. In addition to their specificity, mAbs are advantageous in that they can be synthesized by cell culture (hybridomas, recombinant cells or the like) uncontaminated by other immunoglobulins. The mAbs herein include for example chimeric, humanized or human antibodies or antibody fragments.

Monoclonal antibodies in accordance with the present invention may be prepared by methods well known to those skilled in the art. For example, mice, rats or rabbits may be immunized with an antigen of interest together with adjuvant. Splenocytes are harvested as a pool from the animals that are administered several immunisations at certain intervals with test bleeds performed to assess for serum antibody titers. Splenocytes are prepared that are either used immediately in fusion experiments or stored in liquid nitrogen for use in future fusions. Fusion experiments are then performed according to the procedure of Stewart & Fuller, J. Immunol. Methods 1989, 123:45-53. Supernatants from wells with growing hybrids are screened by e.g. enzyme-linked immunosorbent assay (ELISA) for mAb secretors. ELISA-positive cultures are cloned either by limiting dilutions or fluorescence-activated cell sorting, typically resulting in hybridomas established from single colonies. The ability of an antibody, including an antibody fragment or sub-fragment, to bind to a specific antigen can be determined by binding assays known in the art, for example, using the antigen of interest as the binding partner.

In a further preferred embodiment, a PBC of the invention is an antibody or an antigen binding fragment thereof, wherein the antibody is a human antibody a humanised antibody or a chimeric-human antibody, or wherein the antigen binding fragment is a fragment of a human antibody a humanised antibody or a chimeric-human antibody.

Human antibodies can also be derived by *in vitro* methods. Suitable examples include but are not limited to phage display (CAT, Morphosys, Dyax, Biosite/Medarex, Xoma, Yumab, Symphogen, Alexion, Affimed) and the like. In phage display, a polynucleotide encoding a single Fab or Fv antibody fragment is expressed on the surface of a phage particle (see e.g., Hoogenboom et al., J. Mol. Biol., 227: 381 (1991); Marks et al., J Mol Biol 222: 581 (1991); U.S. Patent No. 5,885,793). Phage are "screened" to identify those antibody fragments having affinity for target. Thus, certain such processes mimic immune selection through the display of antibody fragment repertoires on the surface of filamentous bacteriophage, and subsequent selection of phage by their binding to target. In certain such procedures, high affinity functional neutralizing antibody fragments are isolated. A complete repertoire of human antibody genes may thus be created by cloning naturally rearranged human V genes from peripheral blood lymphocytes (see, e.g., Mullinax et al., Proc Natl Acad Sci (USA), 87: 8095-8099 (1990)) or by generating fully synthetic or semi-synthetic phage display libraries with human antibody sequences (see Knappik et al 2000; J Mol Biol 296:57; de Kruif et al, 1995; J Mol Biol 248):97).

The antibodies described herein may alternatively be prepared through the utilization of the XenoMouse^{®} technology. Such mice are capable of producing human immunoglobulin molecules and antibodies and are deficient in the production of murine immunoglobulin molecules and antibodies. In particular, a preferred embodiment of transgenic production of mice and antibodies is disclosed in U.S. Patent Application Serial No. 08/759,620, filed December 3, 1996 and International Patent Application Nos. WO 98/24893, published June 11, 1998 and WO 00/76310, published December 21, 2000. See also Mendez et al., Nature Genetics, 15:146-156 (1997). Through the use of such technology, fully human monoclonal antibodies to a variety of antigens have been produced. Essentially, XenoMouse^{®} lines of mice are immunized with an antigen of interest. e.g.an epitope of the S protein as described herein), lymphatic cells (such as B-cells) are recovered from the hyper-immunized mice, and the recovered lymphocytes are fused with a myeloid-type cell line to prepare immortal hybridoma cell lines. These hybridoma cell lines are screened and selected to identify hybridoma cell lines that produce antibodies specific to the antigen of interest. Other "humanised" mice are also commercially available: eg, Medarex - HuMab mouse, Kymab - Kymouse, Regeneron - Velocimmune mouse, Kirin - TC mouse, Trianni - Trianni mouse, OmniAb - OmniMouse, Harbour Antibodies - H2L2 mouse, Merus - MeMo mouse. Also are available are "humanised" other species: rats: OmniAb - OmniRat, OMT - UniRat. Chicken: OmniAb - OmniChicken.

The term "humanised antibody" according to the present invention refers to immunoglobulin chains or fragments thereof (such as Fab, Fab', F(ab')2, Fv, or other antigen-binding sub-sequences of antibodies), which contain minimal sequence (but typically, still at least a portion) derived from non-human immunoglobulin. For the most part, humanised antibodies are human immunoglobulins (the recipient antibody) in which CDR residues of the recipient antibody are replaced by CDR residues from a non-human species immunoglobulin (the donor antibody) such as a mouse, rat or rabbit having the desired specificity, affinity and capacity. As such, at least a portion of the framework sequence of said antibody or fragment thereof may be a human consensus framework sequence. In some instances, Fv framework residues of the human immunoglobulin need to be replaced by the corresponding non-human residues to increase specificity or affinity. Furthermore, humanised antibodies can comprise residues which are found neither in the recipient antibody nor in the imported CDR or framework sequences. These modifications are made to further refine and maximise antibody performance. In general, the humanised antibody will comprise substantially all of at least one, and typically at least two, variable domains, in which all or substantially all of the CDR regions correspond to those of a non-human immunoglobulin and all or substantially all of the framework regions are those of a human immunoglobulin consensus sequence. The humanised antibody optimally also will comprise at least a portion of an immunoglobulin constant region, typically that of a human immunoglobulin, which (eg human) immunoglobulin constant region may be modified (eg by mutations or glycoengineering) to optimise one or more properties of such region and/or to improve the function of the (eg therapeutic) antibody, such as to increase or reduce Fc effector functions or to increase serum half-life. Exemplary such Fc modification (for example, Fc engineering or Fc enhancement) are described elsewhere herein.

The term "chimeric antibody" according to the present invention refers to an antibody whose light and/or heavy chain genes have been constructed, typically by genetic engineering, from immunoglobulin variable and constant regions which are identical to, or homologous to, corresponding sequences of different species, such as mouse and human. Alternatively, variable region genes derive from a particular antibody class or subclass while the remainder of the chain derives from another antibody class or subclass of the same or a different species. It covers also fragments of such antibodies. For example, a typical therapeutic chimeric antibody is a hybrid protein composed of the variable or antigen-binding domain from a mouse antibody and the constant or effector domain from a human antibody, although other mammalian species may be used.

In particular of such embodiments, a PBC of the invention comprises an antigen binding domain of an antibody wherein the antigen binding domain is of a human antibody. Preferably, PBC comprises an antigen binding domain of an antibody or an antigen binding fragment thereof, which is a human antigen binding domain; (ii) the antibody is a monoclonal antibody, or wherein the antigen binding fragment is a fragment of a monoclonal antibody; and (iii) the antibody is a human antibody or a humanised antibody, or wherein the antigen binding fragment is a fragment of a human antibody, a humanised antibody or a chimeric-human antibody.

Light chains of human antibodies generally are classified as kappa and lambda light chains, and each of these contains one variable region and one constant domain. Heavy chains are typically classified as mu, delta, gamma, alpha, or epsilon chains, and these define the antibody's isotype as IgM, IgD, IgG, IgA, and IgE, respectively. Human IgG has several subtypes, including, but not limited to, lgG1, lgG2, lgG3, and lgG4. Human IgM subtypes include IgM, and lgM2. Human IgA subtypes include lgA1 and lgA2. In humans, the IgA and IgD isotypes contain four heavy chains and four light chains; the IgG and IgE isotypes contain two heavy chains and two light chains; and the IgM isotype contains ten or twelve heavy chains and ten or twelve light chains. Antibodies according to the invention may be IgG, IgE, IgD, IgA, or IgM immunoglobulins, in the case of camelid antibodies or their fragments, such antibodies are IgG 2b/2c (otherwise known as IgG 2/3).

In some embodiments, the PBC of the invention is an IgG antibody or fragment thereof. In some embodiments, the PBC of the invention is an IgE antibody or fragment thereof. In some embodiments, the PBC of the invention is an IgD antibody or fragment thereof. In some embodiments, the PBC of the invention is an IgA antibody or fragment thereof. In some embodiments, the PBC of the invention is an IgM antibody or fragment thereof. Preferably the PBC of the invention is, comprises or is derived from an IgG immunoglobulin or fragment thereof; such as a human, human-derived IgG immunoglobulin, or a rabbit- or rat-derived IgG, and/or an IgG2 immunoglobulin, or fragment thereof. When the PBC of the invention is, comprises or is derived from a rat-derived IgG, then preferably, the PBC is, comprises or is derived from, a rat IgG2a or IgG2b immunoglobulin. When the PBC of the invention is, comprises or is derived from a human-derived IgG, then more preferably, the PBC of the invention is, comprises or is derived from a human IgGi, IgG2 or IgG4, most preferably, the PBC of the invention is, comprises or is derived from a human IgGi or IgG2

Accordingly, in particular embodiments of the invention, a PBC is an antibody wherein the antibody is an IgG, IgE, IgD, IgA, or IgM immunoglobulin; preferably an IgG immunoglobulin.

An PBC of the invention, where comprising at least a portion of an immunoglobulin constant region (typically that of a human immunoglobulin) may have such (eg human) immunoglobulin constant region modified - for example eg by glycoengineering or mutations - to optimise one or more properties of such region and/or to improve the function of the (eg therapeutic) antibody, such as to increase or reduce Fc effector functions or to increase serum half-life.

Antibody fragments include "Fab fragments", which are composed of one constant and one variable domain of each of the heavy and the light chains, held together by the adjacent constant region of the light chain and the first constant domain (CHi) of the heavy chain. These may be formed by protease digestion, e.g. with papain, from conventional antibodies, but similar Fab fragments may also be produced by genetic engineering. Fab fragments include Fab', Fab and "Fab-SH" (which are Fab fragments containing at least one free sulfhydryl group).

Fab' fragments differ from Fab fragments in that they contain additional residues at the carboxy terminus of the first constant domain of the heavy chain including one or more cysteines from the antibody hinge region. Fab' fragments include "Fab'-SH" (which are Fab' fragments containing at least one free sulfhydryl group).

Further, antibody fragments include F(ab')2 fragments, which contain two light chains and two heavy chains containing a portion of the constant region between the CH1 and CH2 domains ("hinge region"), such that an interchain disulphide bond is formed between the two heavy chains. A F(ab')2 fragment thus is composed of two Fab' fragments that are held together by a disulphide bond between the two heavy chains. F(ab')2 fragments may be prepared from conventional antibodies by proteolytic cleavage with an enzyme that cleaves below the hinge region, e.g. with pepsin, or by genetic engineering.

An "Fv region" comprises the variable regions from both the heavy and light chains, but lacks the constant regions. "Single-chain antibodies" or "scFv" are Fv molecules in which the heavy and light chain variable regions have been connected by a flexible linker to form a single polypeptide chain, which forms an antigen binding region.

An "Fc region" comprises two heavy chain fragments comprising the CH2 and CH3 domains of an antibody. The two heavy chain fragments are held together by two or more disulphide bonds and by hydrophobic interactions of the CH3 domains.

Accordingly, in some embodiments, the PBC of the invention is an antibody fragment selected from the list consisting of: Fab', Fab, Fab'-SH, Fab-SH, Fv, scFv and F(ab')2.

A PBC of the herein disclosed invention may be provided as a fusion protein of the PBC (for example a V_{H}H, or multi-specific V_{H}H as described) fused to a heterologous polypeptide chain, such as Fc immunoglobulin domains, albumin binding proteins, or tags or labels, and/or the PBC conjugated to another chemical moiety such as an effector group or a labelling group. In particular interesting for the context of the present invention are fusion proteins that have an increased half life compared to the V_{H}H. Fusion proteins that are known to increase half life of antibodies are fusions to albumin, albumin antibodies, albumin binding peptides or a fusion to albumin binding domains.

### Nucleic Acids and Host Cells

In a **fifth aspect,** the invention pertains to a nucleic acid encoding for a PBC which is a protein or polypeptide according to any one of the preceding aspects, hence preferably such PBC is an antigen binding protein (ABP). For example, the component encoded by a nucleic acid of the invention may be all or part of one chain of an antibody of the invention; or the component may be a binding domain of a V_{H}H of said ABP. The component encoded by such a nucleic acid may be all or part of one or other of the chains of an antibody of the invention; for example, the component encoded by such a nucleic acid may be an ABD of the invention. The nucleic acids of the invention may also encode a fragment, derivative, mutant, or variant of an ABP of the invention, and/or represent components that are polynucleotides suitable and/or sufficient for use as hybridisation probes, polymerase chain reaction (PCR) primers or sequencing primers for identifying, analyzing, mutating or amplifying a polynucleotide encoding a polypeptide, anti-sense or inhibitory nucleic acids (such as RNAi/siRNA/shRNA or gRNA molecules) for inhibiting expression of a polynucleotide, and complementary sequences of the foregoing.

In particular embodiments of the invention, a nucleic acid of the invention comprises a nucleic acid having a sequence encoding a heavy or light chain CDR, a combination of heavy and/or light chain CDR1, CDR2 and CDR3 or a heavy or light chain variable domain, in each case as displayed in Table 1, or a functional fragment thereof.

The nucleic acid according to the invention may be a DNA or RNA of genomic, mRNA, cDNA, or synthetic origin or some combination thereof, optionally linked to a polynucleotide to which it is not linked in nature. In some embodiments, such nucleic acid may comprise one or more (such as 2, 3, 4, 5, 6, 7, 8, 9, 10 or more than 20, in particular between 1 and about 5, or preferably all instances of a particular nucleotide in the sequence) unnatural (e.g. synthetic) nucleotides; and/or such nucleic acid may comprise (e.g. is conjugated to) another chemical moiety, such as a labelling group or an effector group; for example, a labelling group or an effector group as described elsewhere herein.

In one embodiment, the nucleic acid of the invention may be isolated or substantially pure. In another embodiment, the nucleic acid of the invention may be recombinant, synthetic and/or modified, or in any other way non-natural. For example, a nucleic acid of the invention may contain at least one nucleic acid substitution (or deletion) modification (such as 2, 3, 4, 5, 6, 7, 8, 9, 10 or more than 10 such modifications, in particular between 1 and about 5 such modifications, preferably 2 or 3 such modifications) relative to a product of nature, such as a human nucleic acid.

Nucleic acids encoding antibody polypeptides (e.g., heavy or light chain, variable domain only, or full length) may be isolated from B-cells of mice, rats, all camelid species, such as llamas and alpacas, sharks, chicken or rabbits that have been immunized with an S protein antigen or fragment thereof, such as one or more epitopes of the S protein (such as of an RBD thereof). The nucleic acid may be isolated by conventional procedures such as PCR.

Changes can be introduced by mutation into the sequence of a nucleic acid of the invention. Such changes, depending on their nature and location in a codon, can lead to changes in the amino acid sequence of a polypeptide (e.g., an antigen binding protein) that it encodes. Mutations can be introduced using any technique known in the art.

In one embodiment, one or more particular amino acid residues may be changed using, for example, a site-directed mutagenesis protocol. In another embodiment, one or more randomly selected residues may be changed using, for example, a random mutagenesis protocol. However, it is made, a mutant polypeptide can be expressed and screened for a desired property. Mutations can be introduced into a nucleic acid without significantly altering the biological activity of a polypeptide that it encodes. For example, one can make nucleotide substitutions leading to amino acid substitutions at non-essential amino acid residues.

Other changes that may be made (e.g. by mutation) to the sequence of a nucleic acid of the invention may not alter the amino acid sequence of the encoded polypeptide, but may lead to changes to its stability and/or effectiveness of expression of the encoded polypeptide. For example, by codon optimisation, the expression of a given polypeptide sequence may be improved by utilising the more common codons for a given amino acid that are found for the species in which the nucleotide is to be expressed. Methods of codon optimisation, and alternative methods (such as optimisation of CpG and G/C content), are described in, for example, Hass et al, 1996 (Current Biology 6:315); WO1996/09378; WO2006/015789 and WO 2002/098443).

In one related aspect, the invention relates to a nucleic acid construct (NAC) comprising at least one nucleic acid of the invention (such as described above). Such an NAC can comprise one or more additional features permitting the expression of the encoded ABP or component of said ABP (eg the ABD) in a cell (such as in a host cell). Examples of NACs of the invention include, but are not limited to, plasmid vectors, viral vectors, mRNA, non-episomal mammalian vectors and expression vectors, for example, recombinant expression vectors. The nucleic acid constructs of the invention can comprise a nucleic acid of the invention in a form suitable for expression of the nucleic acid in a cell, such as a host cell, (see below). The nucleic acid constructs of the invention will be, typically, recombinant nucleic acids, and/or may be isolated and/or substantially pure. Recombinant nucleic acids will, typically, be non-natural; particularly if they comprise portions that are derived from different species and/or synthetic, in-vitro or mutagenic methods.

Such nucleic acid or NAC of the invention may, as further described herein elsewhere, be used in therapy to deliver such nucleic acid or NAC by administration to a subject to cells involved with the disorder to be treated. Delivery systems for targeting a nucleic acid or NAC to a specific cell or tissue are well known in the art and include viral delivery systems where such nucleic acid or NAC of the invention is introduced into or combined with a viral vector.

In another related aspect, the invention relates to a cell (such as a host cell and/or a recombinant host cell) comprising one or more nucleic acid or NAC of the invention. Preferably, such cell is capable of expressing the ABP (or component thereof) encoded by said NAC(s). For example, if an ABP of the invention comprises two separate polypeptide chains (e.g. a heavy and light chain of an IgG), then the cell of the invention may comprise a first NAC that encodes (and can express) the heavy chain of such ABP as well as a second NAC that encodes (and can express) the light chain of such ABP; alternatively, the cell may comprise a single NAC that encodes both chains of such ABP. In these ways, such a cell of the invention would be capable of expressing a functional (e.g. binding and/or inhibitory) ABP of the invention. A (host) cell of invention may be one of the mammalian, prokaryotic or eukaryotic host cells as described elsewhere herein, in particularly where the cell is a E. coli cell, a Komagataella phaffii cell or a Chinese hamster ovary (CHO) cell.

In certain embodiments of such aspect, the (host) cell is a human cell; in particular it may be a human cell that has been sampled from a specific individual (eg an autologous human cell). In such embodiments, such human cell can be propagated and/or manipulated in-vitro so as to introduce a NAC of the present invention. The utility of a manipulated human cell from a specific individual can be to produce an ABP of the invention, including to reintroduce a population of such manipulated human cells into a human subject, such as for use in therapy. In certain of such uses, the manipulated human cell may be introduced into the same human individual from which it was first sampled; for example, as an autologous human cell.

The human cell that is subject to such manipulation can be of any germ cell or somatic cell type in the body. For example, the donor cell can be a germ cell or a somatic cell selected from the group consisting of fibroblasts, B cells, T cells, dendritic cells, keratinocytes, adipose cells, epithelial cells, epidermal cells, chondrocytes, cumulus cells, neural cells, glial cells, astrocytes, cardiac cells, oesophageal cells, muscle cells, melanocytes, hematopoietic cells, macrophages, monocytes, and mononuclear cells. The donor cell can be obtained from any organ or tissue in the body; for example, it can be a cell from an organ selected from the group consisting of liver, stomach, intestines, lung, pancreas, cornea, skin, gallbladder, ovary, testes, kidneys, heart, bladder, and urethra.

### Pharmaceutical Compositions

To be used in therapy, the PBCs, ABPs, nucleic acids or NACs (or the cells, such as host cells) of the invention may be formulated into a pharmaceutical composition appropriate to facilitate administration to animals or humans. The term "pharmaceutical composition" means a mixture of substances including a therapeutically active substance (such as a PBC of the invention) for pharmaceutical use.

In **a seventh aspect,** the invention pertains to a pharmaceutical composition comprising a PBC recited in any of the second, third or fourth aspects, an isolated nucleic acid recited in the fifth aspect, a recombinant host cell recited in the sixth aspect, together with a pharmaceutically acceptable carrier and/or excipient.

By way of example, the pharmaceutical composition of the invention may comprise between 0.1% and 100% (w/w) active ingredient (for example, a PBC of the invention), such as about 0.5%, 1%, 2%, 3%, 4%, 5%, 6%, 8% 10%, 15%, 20%, 25%, 30%, 40%, 50%, 60%, 70%, 80%, 90%, 95%, 96%, 97%, 98% or 99%, preferably between about 1% and about 20%, between about 10% and 50% or between about 40% and 90%.

As used herein the language "pharmaceutically acceptable" excipient, stabiliser or carrier is intended to include any and all solvents, solubilisers, fillers, stabilisers, binders, absorbents, bases, buffering agents, lubricants, controlled release vehicles, diluents, emulsifying agents, humectants, dispersion media, coatings, antibacterial or antifungal agents, isotonic and absorption delaying agents, and the like, compatible with pharmaceutical administration. The use of such media and agents for pharmaceutically active substances is well-known in the art. Except insofar as any conventional media or agent is incompatible with the active compound, use thereof in the compositions is contemplated. Supplementary agents can also be incorporated into the compositions.

The pharmaceutical composition of (or for use with) the invention is, typically, formulated to be compatible with its intended route of administration. Examples of routes of administration include oral, parenteral, e.g., intrathecal, intra-arterial, intravenous, intradermal, intramuscular, subcutaneous, oral, transdermal (topical) and transmucosal administration.

For administration by inhalation, which for the treatment of a respiratory disease such as COVID may be preferred in context of the invention, the compounds of (or for use with) the invention (eg a PBC of the invention) are typically delivered in the form of an aerosol spray from pressured container or dispenser which contains a suitable propellant, e.g., a gas such as carbon dioxide, or a nebuliser.

### Treatment and Prevention of Diseases using the Compounds or Compositions

In **an eighth aspect,** the invention pertains to a compound or composition of the preceding aspects for use in, or pertains to, a method of treatment of a disease

The terms "treatment," "treating," "treat," and the like are used herein to generally refer to obtaining a desired pharmacologic and/or physiologic effect. The effect may be prophylactic (also referred to as "prevention" or "preventing") in terms of completely or partially preventing a disease or symptom thereof and/or may be therapeutic in terms of a partial or complete stabilization or cure for a disease and/or adverse effect attributable to the disease. "Treatment" as used herein covers any treatment of a disease in a mammal, particularly a human, and includes: (a) preventing the disease or symptom from occurring in a subject who may be predisposed to the disease or symptom but has not yet been diagnosed as having it; (b) inhibiting the disease symptom, i.e., arresting its development; (c) relieving the disease symptom, i.e., causing regression of the disease or symptom; (d) limiting spread of a virus from one cell to another within an individual, e.g., limiting spread of a virus from an infected epithelial cell to other, uninfected, epithelial cells within an individual (which is preferable in accordance with the invention, which without being bound to theory, provides means and methods for reducing viral entry into a host cell); (e) limiting replication of a virus within an individual; (f) limiting entry of a virus into a cell in an individual; and (g) reducing the number of viruses in an individual or in a target tissue or target biological sample in an individual.

The terms "subject," "individual," "host," and "patient" are used interchangeably herein to refer to a bird or mammal, including, but not limited to, chicken, murines (rats, mice), felines, pholidota, chiroptera, non-human primates (e.g., simians), humans, canines, ungulates, etc. In some embodiments, a "subject" is a human, and can also be referred to as a "patient."

A disease to be treated in the various aspects and embodiments of the invention are selected from diseases caused by a virus, preferably a virus that causes a respiratory disease. In context of the present invention, the virus is preferably a virus that is characterized by a host cell entry mechanism that uses a spike protein, or variants thereof. Preferably such diseases include Corona virus caused primary or secondary diseases.

In context of the present invention, if a compound or composition is administered prior to exposure to the virus, the treatment is preventive or prophylactic (i.e., it protects the patient against infection), whereas if the administration is performed after infection or initiation of the disease, the treatment is therapeutic (i.e., it combats the existing infection). Preferably, the compounds or composition of the invention are used in a therapeutic aspect. However, the compounds or compositions of the invention may in addition be used to prevent a virally caused disease in a subject that is suspected of developing the disease, or who to a subject that is a member risk group. Such risk group is characterized by having an increased risk for a fatal event or outcome in the case of an infection with the virus. Elderly subjects, such as subjects of 50 years or older (preferably 60 years or older, most preferably 70 years or older) are risk groups, or subjects having another immune compromising condition, or being generally of poor health.

A therapeutic or preventive use in context of the present invention is preferably a use in the treatment or prevention of COVID19, or any later (subsequent to 2019) variant of the disease.

Accordingly, in such eighth aspect, the invention relates to a method for the treatment of a disease, disorder or condition in a mammalian subject by administering a product to the subject wherein the product is an PBC of the invention, or of a variant thereof. In a related aspect, the invention relates to a product for use in medicine, wherein the product is a compound that is PBC of the invention, or of a variant thereof. In particular embodiments, of these medical/treatment claims, the modulating compound (such as an PBC) stabilizes (favors or induces) a S protein up-conformation (such as of SARS-CoV2- S protein; and/or wherein the product is selected from the list consisting of a mono or multi specific PBC, nucleic acid, NAC or recombinant host cell of the invention, in particular a PBC of the invention.

In a related aspect, the invention also relates to method of treating or preventing a disease, disorder or condition in a mammalian subject in need thereof, comprising administering to said subject at least once an effective amount of modulating compound as desired above, or, and in particular administering to said subject at least once an effective amount of the PBC, the NAC, the (host) cells, or the pharmaceutical composition as described above.

In another related aspect, the invention also relates to the use of a product of the invention as describe above, or a modulating compound as described above (in particular an PBC of the invention) for the manufacture of a medicament, in particular for the treatment of a disease, disorder or condition in a mammalian subject, in particular where the disease, disorder or condition is one as set out herein.

In particular embodiments of these aspects, the modulating compound is one described above, and/or is an PBC, NAC, a (host) cell, or a pharmaceutical composition of the present invention; in particular is an PBC of the invention.

### Diagnosis of Diseases

In **a ninths aspect,** the invention pertains to a compound or composition of the preceding aspects for use in, or pertains to, a method of treatment of a disease.

A compound or composition of the invention, in particular one or more of the herein disclosed PBC of the invention, or a variant thereof, can be used for diagnostic purposes to detect, diagnose, or monitor diseases, disorders and/or conditions associated with the undesired presence of a viral S protein. Viral S-protein comprising viral particles, positive cells for such viral particles or proteins, or cells positive for a virus or a viral s protein, or a variant thereof, and/or associated with a viral infection can be so detected. The disease, disorder and/or conditions so detected, diagnosed, or monitored, can be one of those described elsewhere herein. In preferred embodiments of the detection and diagnosis methods of the invention, the diseases, disorders or conditions is a disorder caused by a viral infection, such as corona virus infection.

In such aspect, the invention relates to a method for determining the presence or an amount of an S protein or of a virus or virus particle comprising the s protein in a biological sample from a subject or in or on any environmental sample (such as a sample taken from any surface), the method comprising the steps of:
- contacting said sample with a PBC capable of the invention; and
- detecting binding between the s protein, or the virus suspected to display such S protein, and the PBC.

In certain embodiments, a biological sample will (preferably) comprise solid material, liquid, cells or tissue of the subject, or an extract of such cells or tissue.

In particular embodiments of such aspect, the method will also comprise a step of:

providing (such as by obtaining) the biological sample from the subject, in particular where such step is conducted prior to the detection step.

In particular embodiments, such detection and/or determination methods can be practiced as a method of diagnosis, such as a method of diagnosis whether a mammalian subject (such as a human subject or patient) has a disease or disorder associated with a corona virus infection.

In certain embodiments, the biological sample is one obtained from a mammalian subject like a human patient. The term "biological sample" is used in its broadest sense and can refer to a bodily sample obtained from the subject (eg, a human patient). For example, the biological sample can include a clinical sample, i.e., a sample derived from a subject. Such samples can include, but are not limited to: peripheral bodily fluids, which may or may not contain cells, e.g., blood, urine, plasma, mucous, bile pancreatic juice, supernatant fluid, and serum; tissue or fine needle biopsy samples; lung biopsy samples or sections (or cells thereof), and archival samples with known diagnosis, treatment and/or outcome history. Biological samples may also include sections of tissues, such as frozen sections taken for histological purposes. The term "biological sample" can also encompass any material derived by processing the sample. Derived materials can include, but are not limited to, cells (or their progeny) isolated from the biological sample, nucleic acids and/or proteins extracted from the sample. Processing of the biological sample may involve one or more of, filtration, distillation, extraction, amplification, concentration, fixation, inactivation of interfering components, addition of reagents, and the like. In one embodiment the biological sample is a plasma (or serum) sample (previously taken) from the subject.

Such detection, determination and/or diagnosis methods can be conducted as an in-vitro method, and can be, for example, practiced using the kit of the present invention (or components thereof).

In some embodiments of these detection, determination and/or diagnosis methods, the biological sample is a tissue sample from the subject, such as a sample of a respiratory system of the subject. Such sample can be taken from the lung, or the upper respiratory tract, such as the mouth, nose, or the trachea.

In some embodiments, determination and/or diagnosis method of the invention can comprise, such as in a further step, comparing the detected amount (or activity of) of (eg protein) the applicable biomarker (ie viral S protein or a variant thereof) with a standard or cut-off value; wherein a detected amount greater than the standard or cut-off value indicates a phenotype (or a risk of developing a phenotype) that is associated with the presence or infection of the virus. Such a standard or cut-off value may be determined from the use of a control assay, or may be pre-determined from one or more values obtained from a study or a plurality of samples having known phenotypes. For example, a cut-off value for a diagnostic test may be determined by the analysis of samples taken from patients in the context of a controlled clinical study, and determination of a cut-off depending on the desired (or obtained) sensitivity and/or specificity of the test.

Examples of methods useful in the detection of (such as the presence or absence of, or an amount of) the virus or viral s protein) include immunoassays, such as the enzyme linked immunosorbent assay (ELISA) and the radioimmunoassay (RIA), which employ PBC (eg of the present invention) such as an antibody or an antigen-binding fragment thereof, preferably a VHH, that specifically binds to such virus or viral s protein.

For such detection, determination or diagnostic applications, the PBC, typically, will be labelled with a detectable labelling group. In general, labelling groups fall into a variety of classes, depending on the assay in which they are to be detected: a) isotopic labels, which may be radioactive or heavy isotopes; b) magnetic labels (e.g., magnetic particles); c) redox active moieties; d) optical dyes; enzymatic groups (e.g. horseradish peroxidase, beta-galactosidase, luciferase, alkaline phosphatase); e) biotinylated groups; and f) predetermined polypeptide epitopes recognised by a secondary reporter (e.g., leucine zipper pair sequences, binding sites for secondary antibodies, metal binding domains, epitope tags, etc.).Suitable labelling groups include, but are not limited to, the following: radioisotopes or radionuclides, fluorescent groups (e.g., FITC, rhodamine, lanthanide phosphors), enzymatic groups (e.g., horseradish peroxidase, beta-galactosidase, luciferase, alkaline phosphatase), chemiluminescent groups, biotinyl groups, or predetermined polypeptide epitopes recognised by a secondary reporter (eg, leucine zipper pair sequences, binding sites for secondary antibodies, metal binding domains, epitope tags). In some embodiments, the labelling group is coupled to the PBC via spacer arms of various lengths to reduce potential steric hindrance. Various methods for labelling proteins are known in the art and may be used. For example, the PBC may be labelled with a secondary reporter (e.g., leucine zipper pair sequences, binding sites for secondary antibodies, metal binding domains, epitope tags, etc.).

For such diagnostic purposes, any one of the herein disclosed compounds or compositions, preferably such PBC of the invention are provided as kits for use in the diagnostic methods of the invention.

### Screening Methods for Therapeutics

In **a tenth aspect,** the invention pertains to a method for identifying and/or characterising a compound suitable for the treatment of a disease, disorder or condition that is associated with, or caused by, a virus expressing a viral spike (S) protein, the method comprising the steps of:
- bringing into contact a candidate compound and a spike protein (or a variant or fragment thereof); and
- detecting and/or quantifying an induction and/or stabilization of an up-conformation of the spike protein (or the variant or fragment thereof) upon binding of the candidate compound to the spike protein (or the variant or fragment thereof),
wherein the induction and/or stabilization of an up-conformation of the spike protein (or the variant or fragment thereof) upon binding of the candidate compound to the spike protein (or the variant or fragment thereof)) compared to a control indicates that the compound is suitable for the treatment of a disease, disorder or condition that is associated with, or caused by, a virus expressing the spike protein.

In **a further aspect,** the invention pertains to a method for identifying and/or characterising a compound suitable for the treatment of a disease, disorder or condition that is associated with, or caused by, a virus expressing a viral spike (S) protein, the method comprising the steps of:
- Providing at least two cells each expressing the viral spike protein or a truncation thereof on the cell surface, preferably wherein the at least two cells do not express a spike protein receptor (such as angiotensin converting enzyme 2 (ACE2);
- bringing into contact the at least two cells, a candidate compound, and a protein binding compound (PBC) of the present invention; and
- detecting and/or quantifying an induction of a cellular fusion between the at least two cells induced by the PBC of the invention,
wherein a reduced induction of a cellular fusion between the at least two cells compared to a control without the candidate compound indicates that the candidate compound is suitable for the treatment of a disease, disorder or condition that is associated with, or caused by, a virus expressing the spike protein.

Preferably, the PBC used in the screening method is a V_{H}H-E, V_{H}H-N, V_{H}H-U, V_{H}H-V, or V_{H}H-W, or a functional variant thereof. The screening method of the invention is predicated on the observation that the inventive PBC stabilize an up-conformation of the RBD.

In some embodiments the at least two cells express a viral spike protein.

A truncation of the SARS-CoV2 spike protein is preferably a C terminal truncation of 10 to 30 amino acids, preferably of about 20 amino acids, more preferably of 18 amino acids.

In a preferred embodiment the at least two cells are composed of a first cell comprising a first detectable label and a second cell comprising a second detectable label, wherein the first and the second detectable label are different. Using detectable labels allows a detection and/or quantification of cell fusion of the first and the second cell, for example by microscopy. Such detectable labels are exemplary shown herein elsewhere. Preferred examples of detectable labels for such uses comprise the expression of fluorescent proteins within the at least two cells, such as GFP (and other fluorescent, for example EGFP, RFP, RFP-t, DsRed, tagged versions of proteins etc).

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES AND SEQUENCES

The figures show:

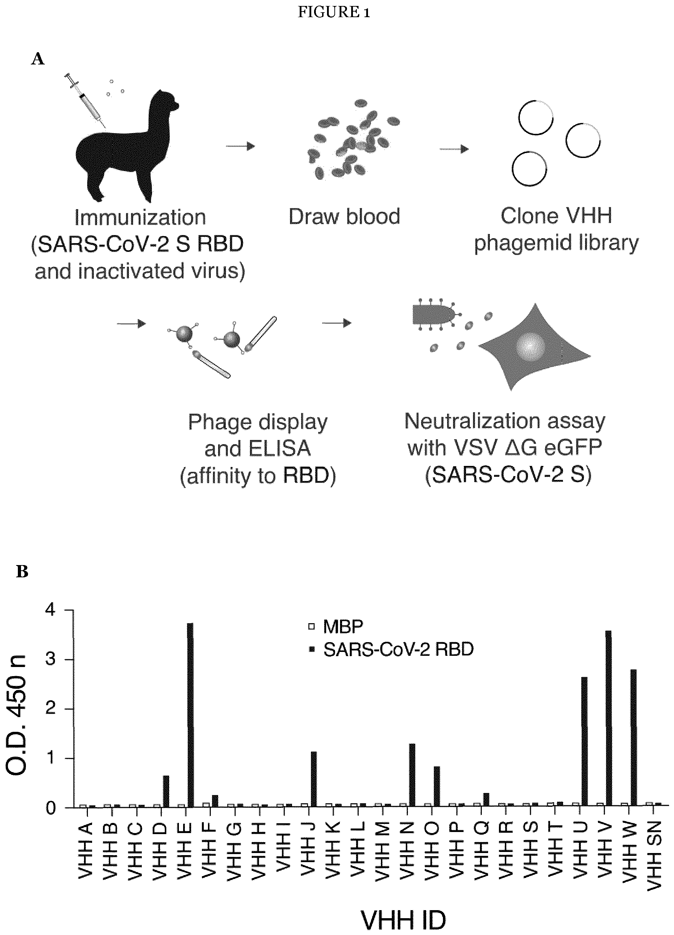

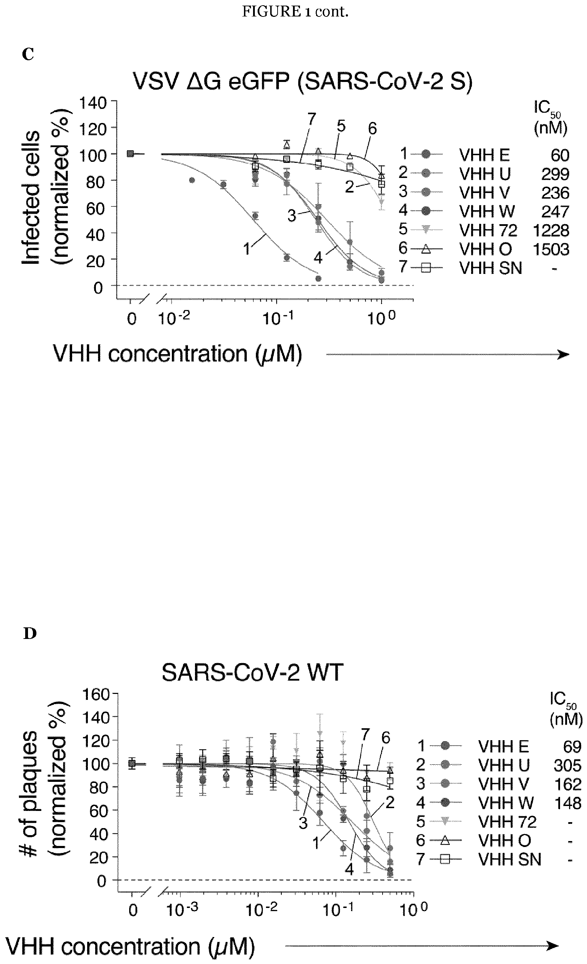

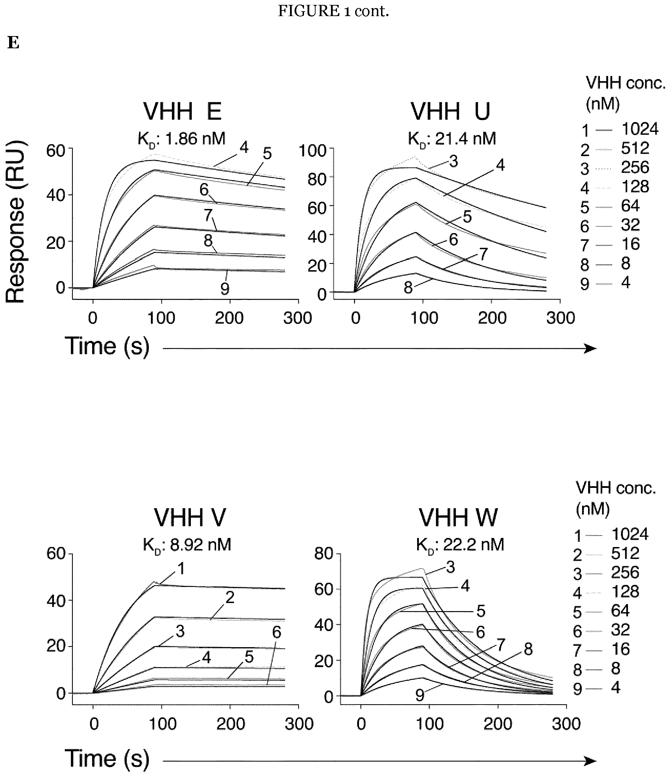

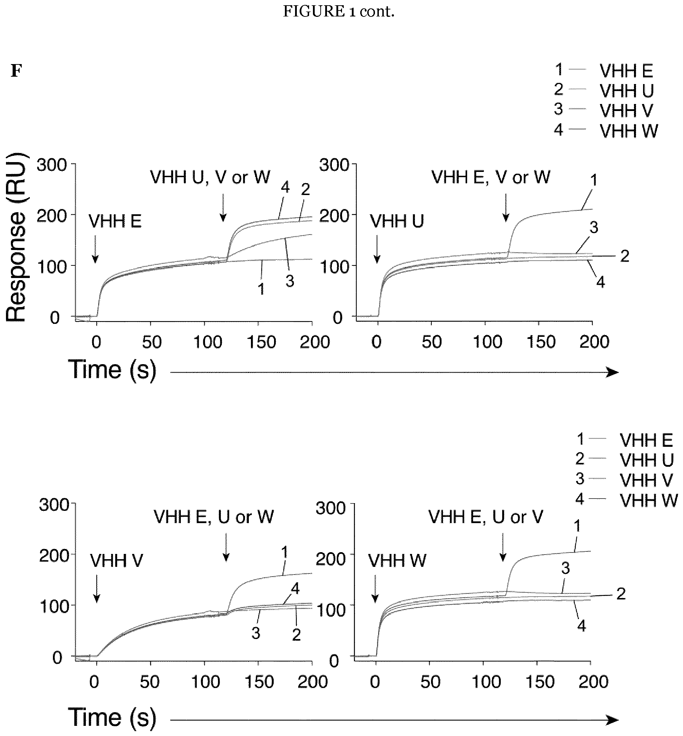

**Figure 1****:** shows that camelid VHHs against two epitopes on SARS-CoV-2 Spike RBD neutralize infection. (A) Strategy to identify VHHs against SARS-CoV-2 spike RBD. (B) Binding of 100 nM HA-tagged VHHs to immobilized SARS-CoV-2 spike RBD or control protein MBP was quantified by ELISA with HRP-coupled anti-HA. Unrelated VHH SN was used as a negative control. (C) SARS-CoV-2 spike-pseudotyped VSV ΔG eGFP was incubated with the indicated concentrations of HA-tagged VHHs at 37° C for 1h, followed by infection of Vero E6 cells for 8 h. eGFP-positive cells were measured by flow cytometry to quantify infection. Normalized values from three independent experiments ± SEM and IC50 values are plotted. (D) SARS-CoV-2 was incubated with the indicated concentrations of HA-tagged VHHs as in (C), followed by plaque assay on Vero E6 cells. Plaques were enumerated 3 days post infection and normalized values of three independent experiments ± SEM and IC50 values are plotted. (E, F) Biotinylated SARS-CoV-2 spike RBD was immobilized on surface plasmon resonance spectroscopy chips. (E) Indicated HA-tagged VHHs were injected for 90 s, followed by dissociation for 120 s. Dissociation constants (KD) were determined based on fits applying a 1:1 interaction model. (F) Epitope binning was performed by first injecting a single VHH for 120 s, followed by injection of 1:1 mixtures of the first VHH in combination with VHH E, U, V, or W for 80 s.

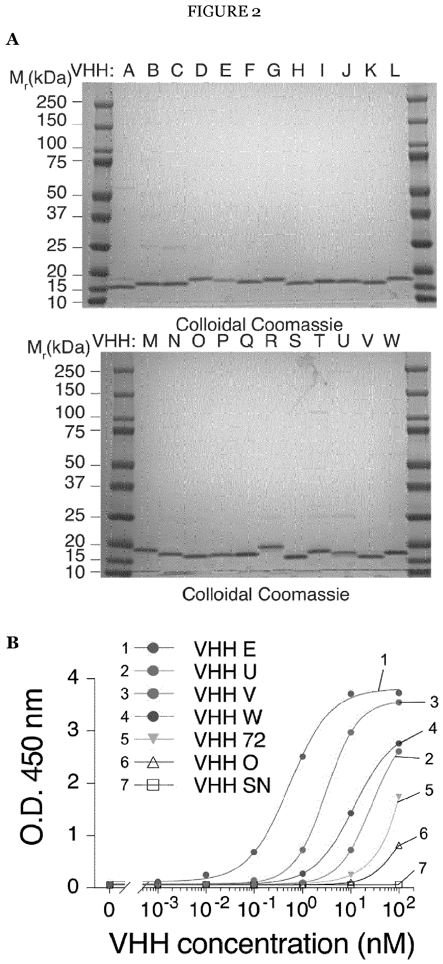

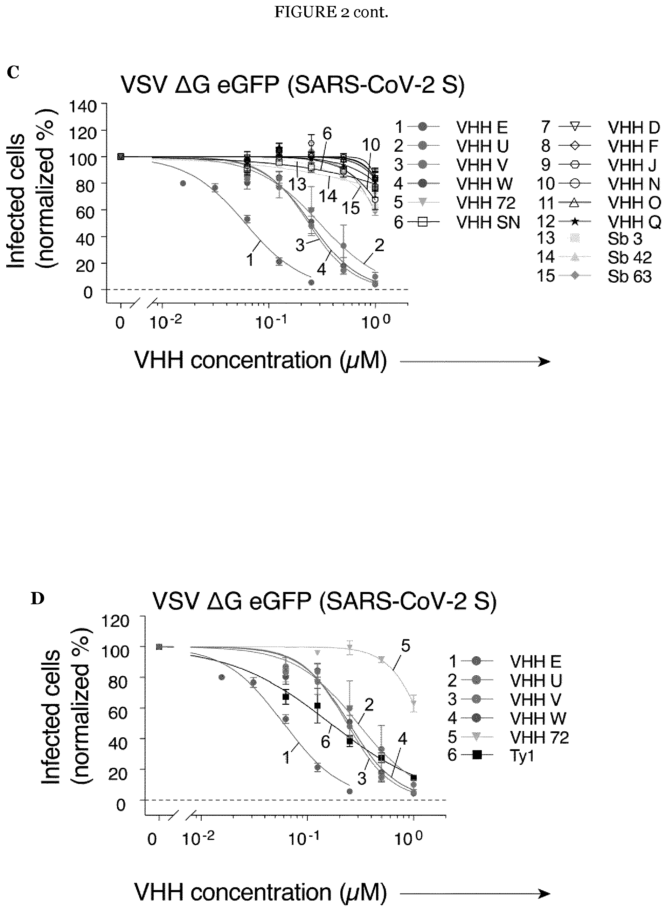

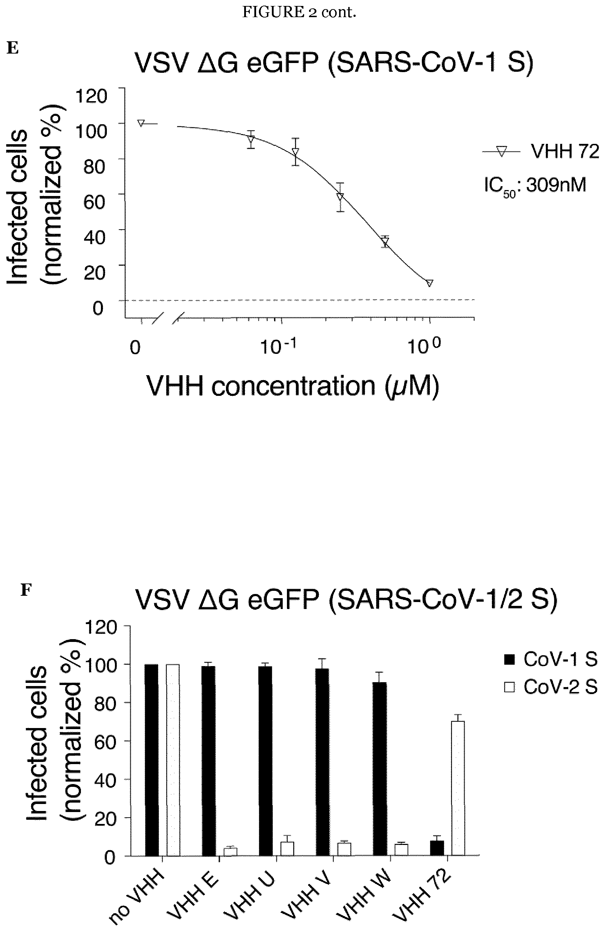

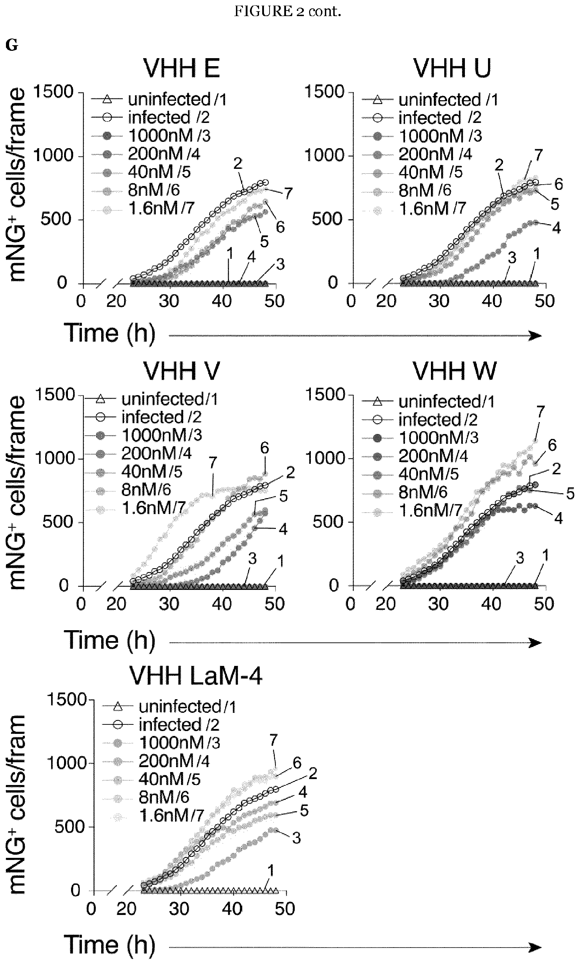

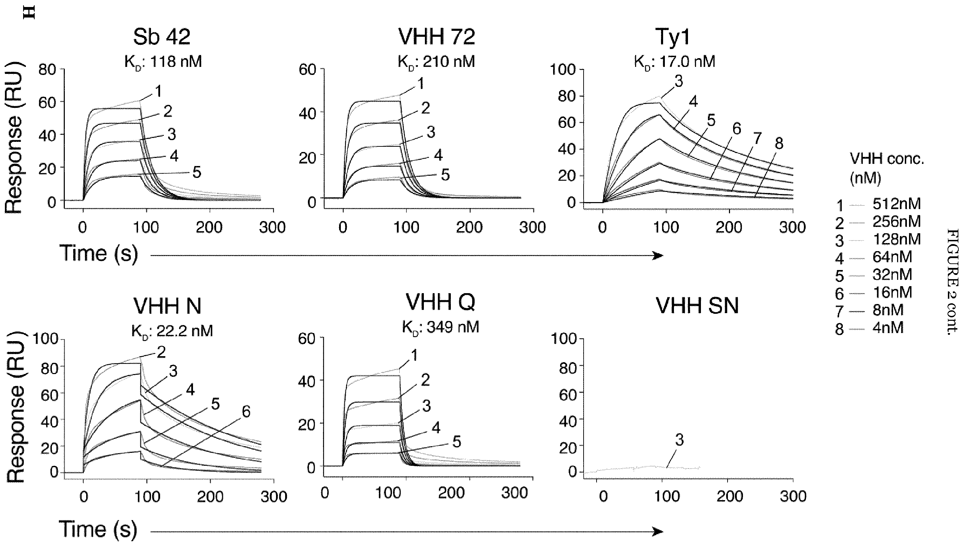

**Figure 2****:** shows that camelid VHHs against two epitopes on SARS-CoV-2 Spike RBD neutralize infection. (A) Colloidal Coomassie stained SDS-PAGE gel of 2 µg HA-tagged VHHs used in Fig. 1B-F, Fig. 7A, Fig. 2B-C, and Fig. 8A. (B) Binding of indicated concentration of HA-tagged VHHs to immobilized SARS-CoV-2 spike RBD or control protein MBP was quantified by ELISA with HRP-coupled anti-HA. (C, D) SARS-CoV-2 spike-pseudotyped VSV ΔG eGFP was incubated with the indicated concentrations of HA-tagged or LPETG-tagged (VHH 72 and Ty1) VHHs at 37°C for 1h, followed by infection of Vero E6 cells for 8h. eGFP-positive cells were measured by flow cytometry to quantify infection. Normalized values from three independent experiments ± SEM are plotted. (E) SARS-CoV-1 spike-pseudotyped VSV ΔG eGFP was incubated with the indicated concentrations of LPETG-tagged VHH 72 and infection of Vero E6 cells quantified as in C. Normalized values from three independent experiments ± SEM are plotted. (F) SARS-CoV-1 or SARS-CoV-2 spike-pseudotyped VSV ΔG eGFP was incubated with 1 µM of LPETG-tagged VHHs and infection of Vero E6 cells quantified as in C. Normalized values from three independent experiments ± SEM are plotted. (G) Caco-2 cells were infected with mNeonGreen-expressing icSARS-CoV-2-mNG in the presence of the indicated VHH concentrations. Infection was microscopically followed over time. (H) SARS-CoV-2 spike RBD was immobilized on SPR chips as in Fig. 1E and the indicated VHHs were injected for 90s, followed by dissociation for 210s. Dissociation constants (KD) were determined as before.

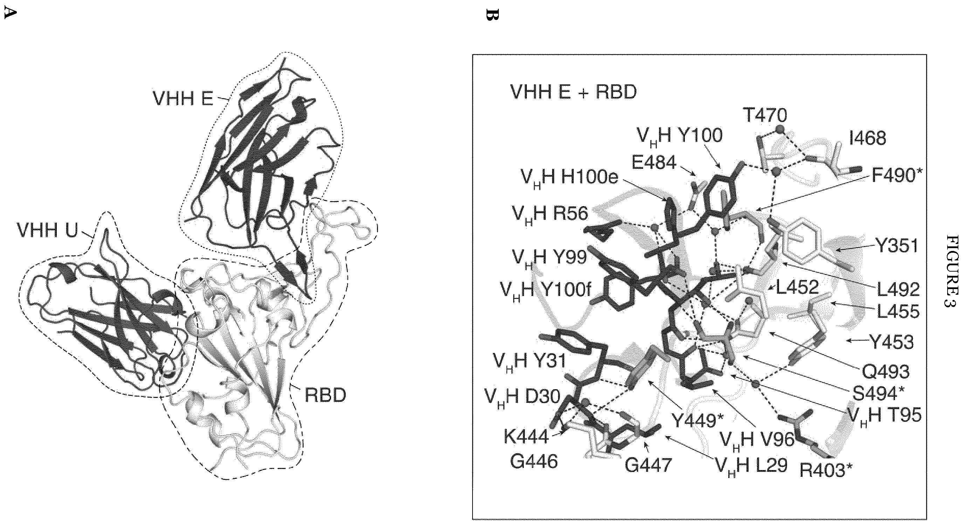

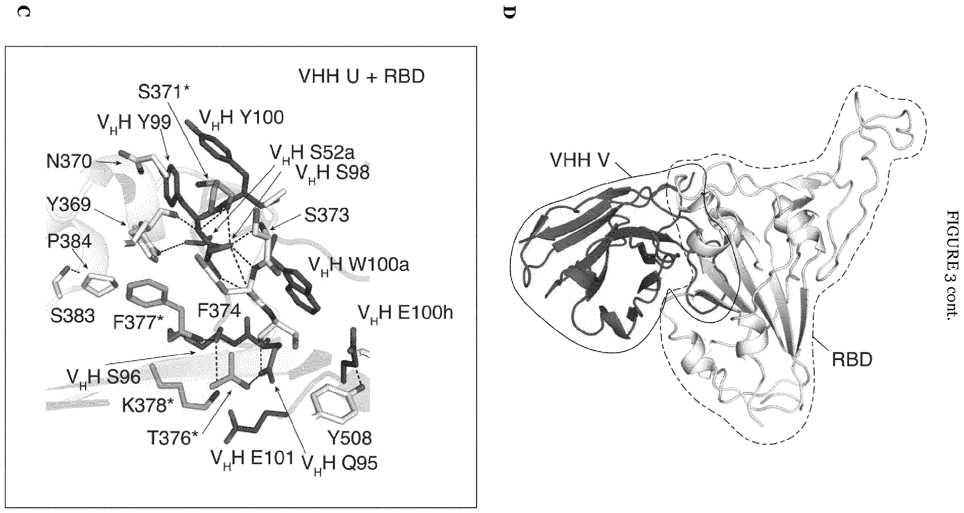

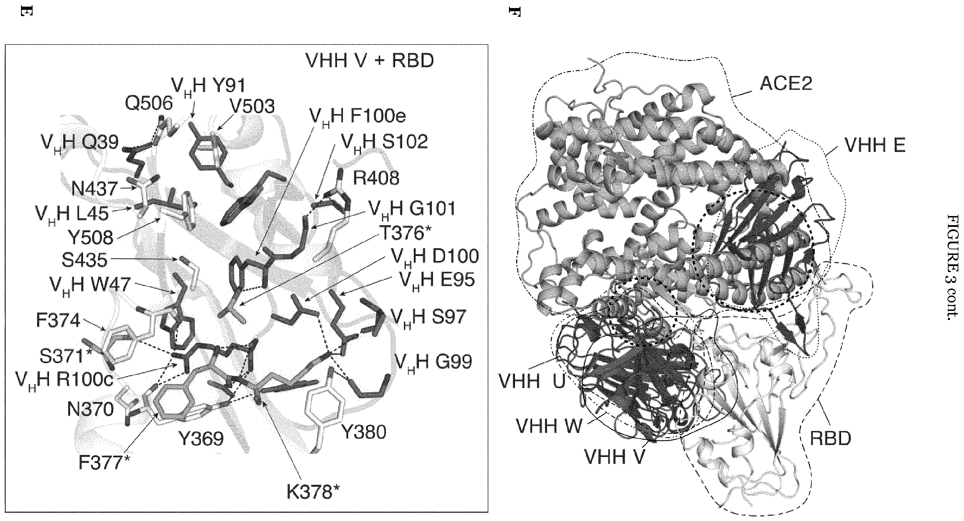

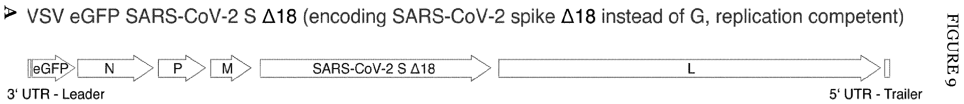

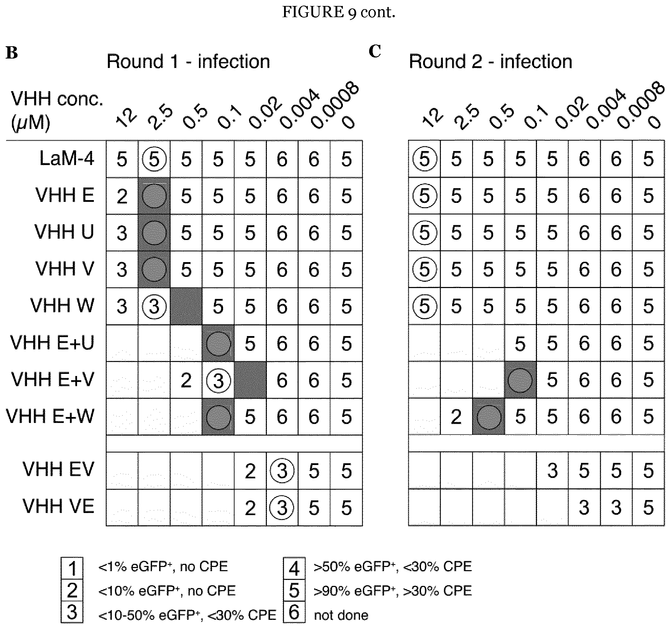

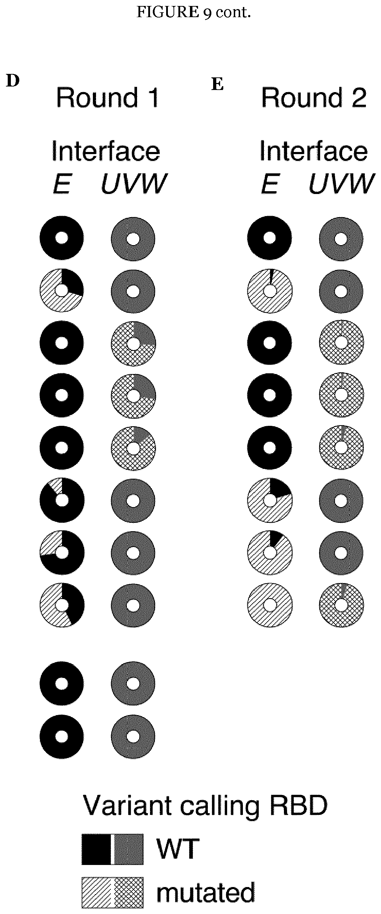

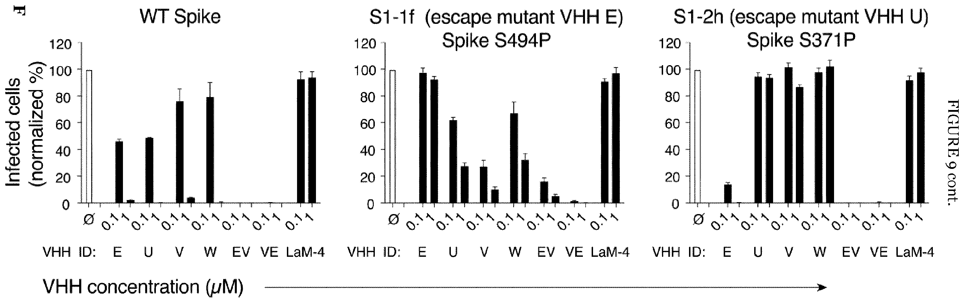

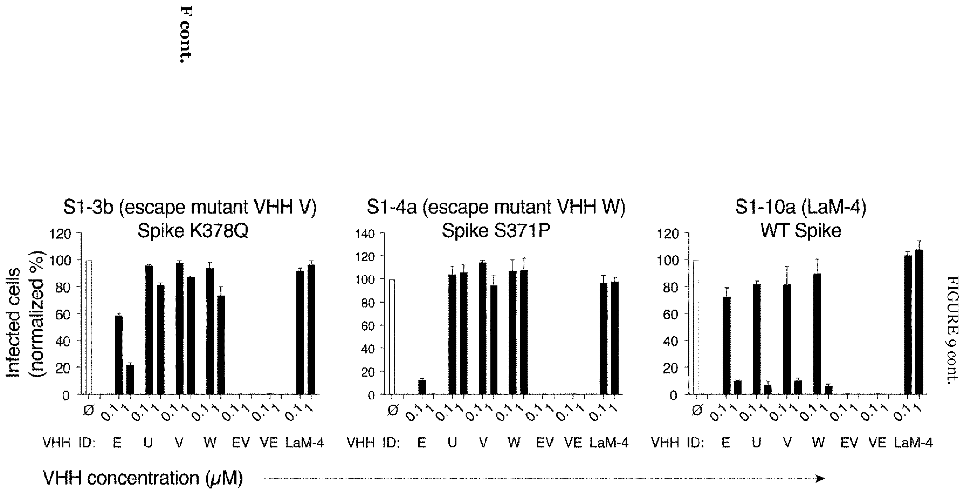

**Figure 3****:** shows that X-ray crystallography defines binding sites of neutralizing VHHs on the SARS-CoV-2 RBD. (A-C) Crystal structure of SARS-CoV-2 spike RBD in complex with VHH E and VHH U at 1.87 Å (A) and detailed interaction interface of RBD (in white) with VHH E (B) and RBD with VHH U (C), respectively. (D-E) Crystal structure of SARS-CoV-2 spike RBD in complex with VHH V at 2.55 Å (D) and detailed interaction interface of RBD with VHH V (E). Escape mutants (see Fig. 9) in the RBD are labeled with asterisks. (F) Overview of binding sites of all four neutralizing VHHs on the RBD and their overlap with ACE2 based on pdb 6MOJ (38). Steric clashes are indicated with dashed circles.

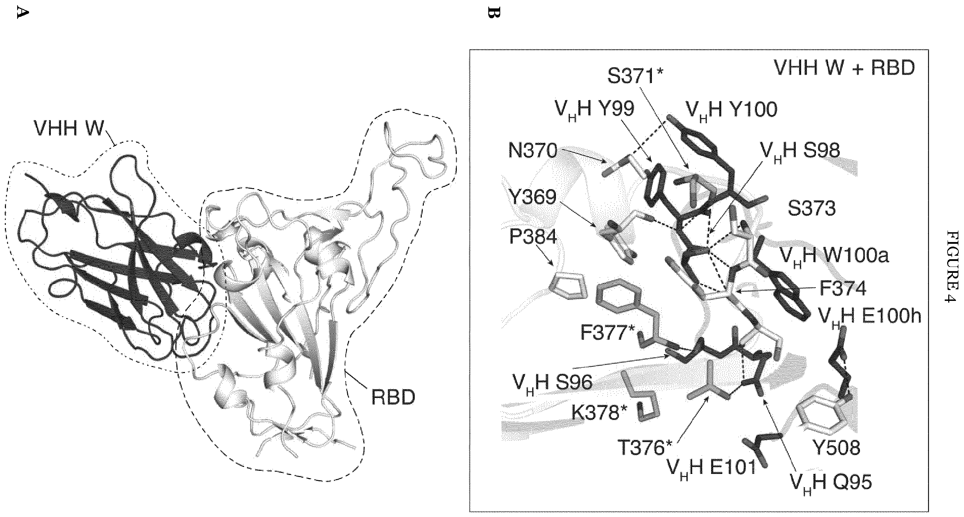

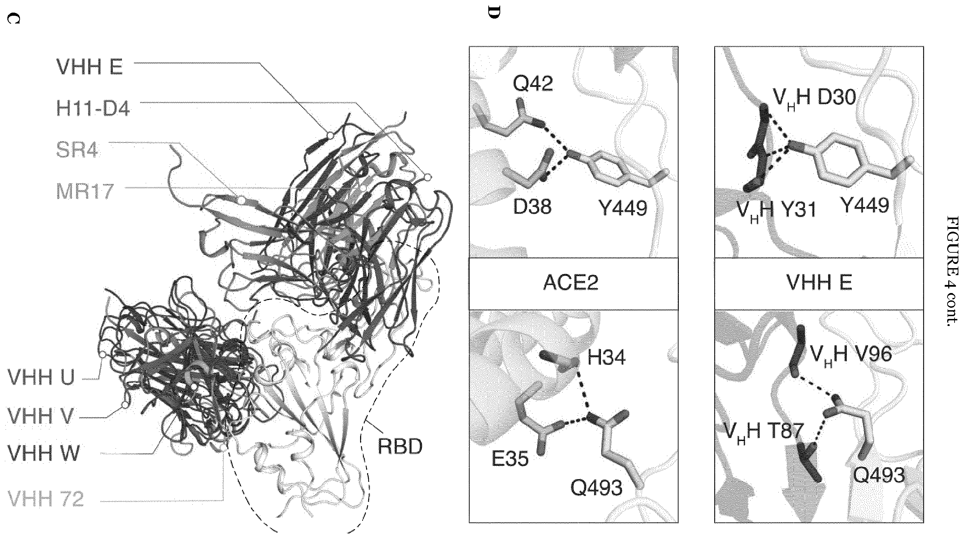

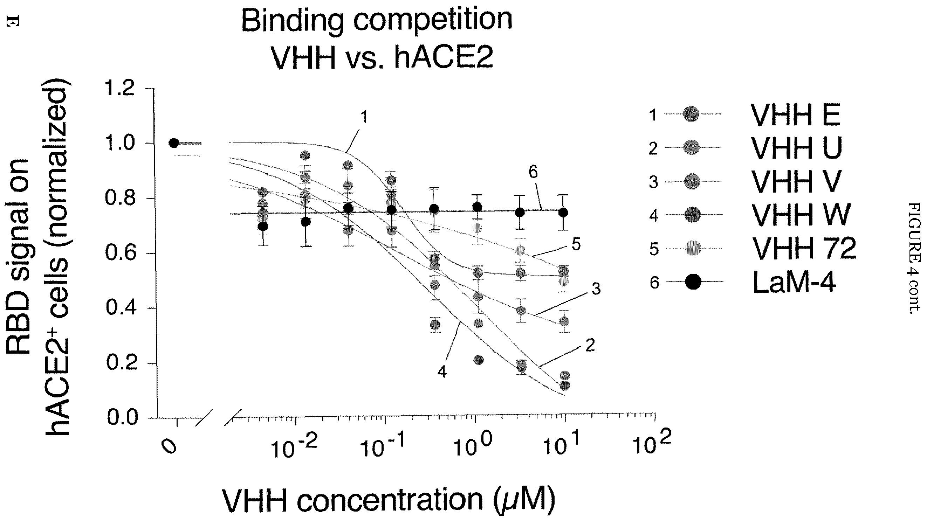

**Figure 4****:** shows that X-ray crystallography defines binding sites of neutralizing VHHs on the SARS-CoV-2 RBD. (A-B) Crystal structure of SARS-CoV-2 spike RBD in complex with VHH W at 2.73 Å (A) and detailed interaction interface of RBD with VHH W with view focusing on epitope residues involved in escape mutations (B). (C) Overview of binding sites of all four neutralizing VHHs on the RBD and their overlap with VHH H11-D4 (pdb 6YZ5) (13), VHH MR17 (pdb 7C8W), and VHH SR4 (pdb 7C8V) (14). (D) Comparison of interaction interface of RBD (in white) with ACE2 (left) or VHH E (right), respectively. (E) HEK 293T cells transiently expressing ACE2-tagRFP-t were incubated with DyLight 488-labeled SARS-CoV-2 spike RBD in the presence of the indicated concentrations of VHHs. RBD bound to ACE2-positive cells was quantified by flow cytometry. Normalized data from three independent experiments ± SEM is plotted.

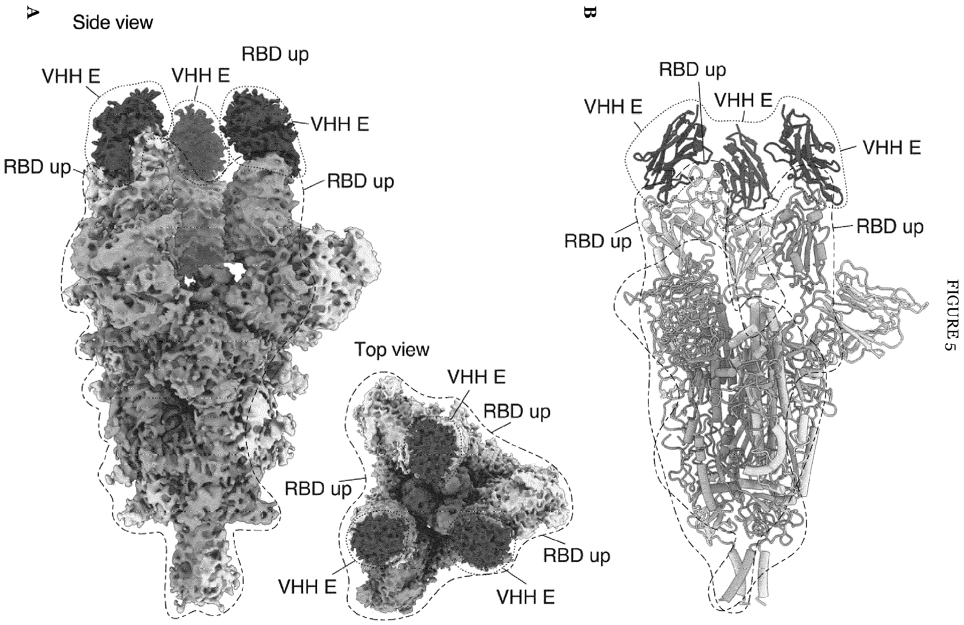

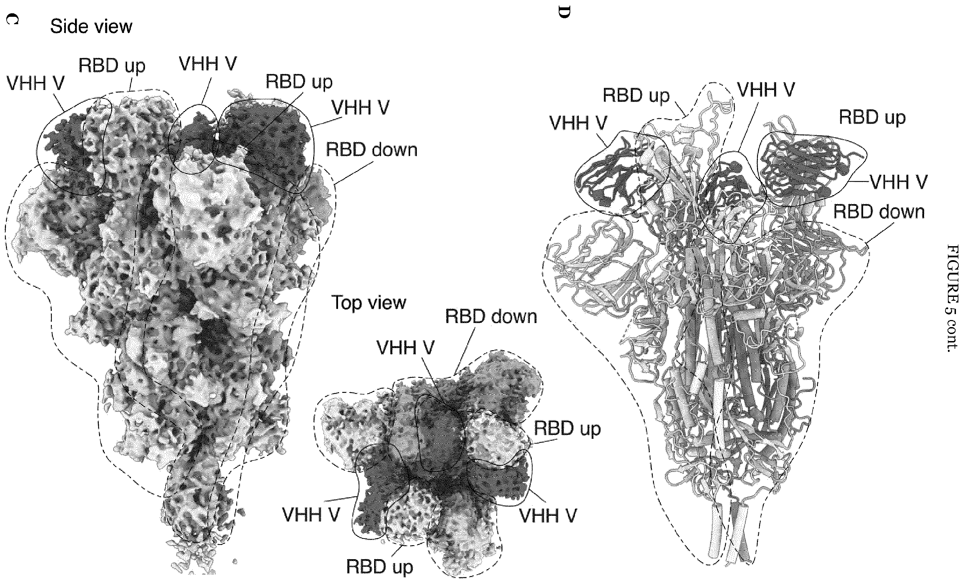

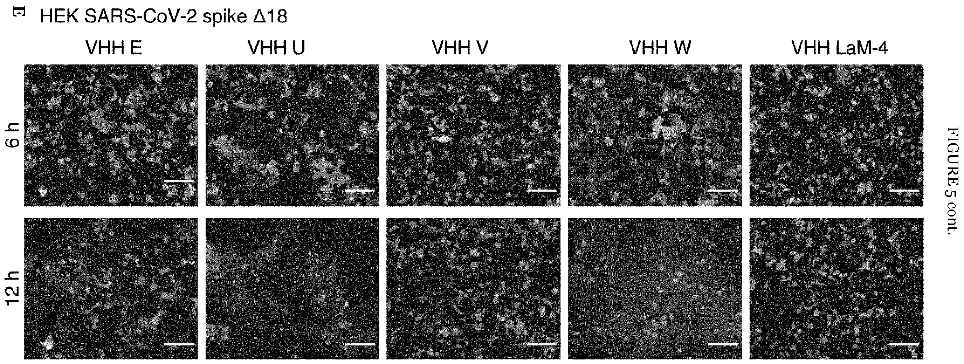

**Figure 5****:** shows that cryo-electron microscopy structures reveal that VHHs stabilize SARS-CoV-2 spike trimers with RBDs in the up conformation. Cryo-EM reconstructions (A, C) and atomic models (B, D) of VHH E (A, B) and VHH V (C, D) in complex with trimeric SARS-CoV-2 spike. (A, B) VHH E binds to SARS-CoV-2 in a 3-up conformation; the resolution is 2.95 Å (0.143 FSC). (C, D) VHH V binds to SARS-CoV-2 in a 2-up conformation with all VHH binding sites occupied at a resolution of 3.14 Å, and VHH V binding to the RBD in the up- or the down-conformation. (E) Flp-In 293 T-REx cell lines inducible expressing SARS-CoV-2 S Δ18 were separately transfected with eGFP (bright grey) or mCherry (medium grey) expression vectors, seeded into microscopy-grade 96-well plates, induced with 1 µg/mL doxycycline for 14 h, and treated with 1 µM of the indicated VHHs. Images representative of three independent experiments were recorded after 6 h and after fixation 12 h post treatment. Scale bars represent 100 µm.

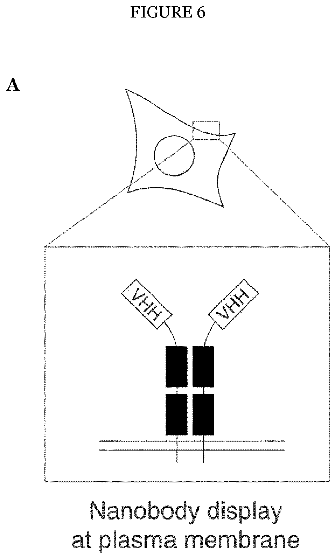

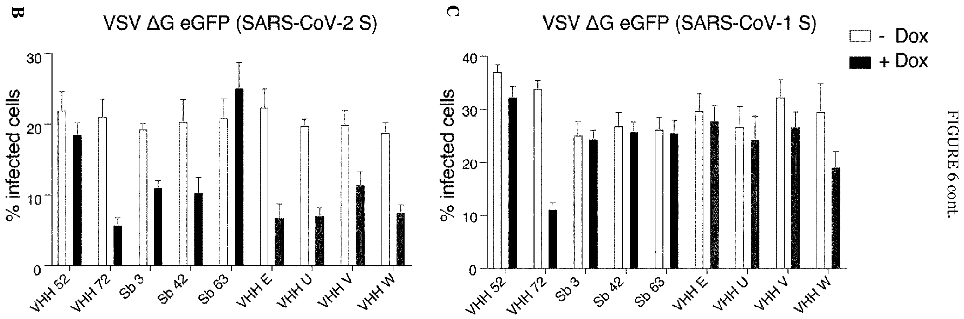

**Figure 6****:** shows that cell surface-displayed VHHs reduce infection. Vero cells doxycycline-inducibly expressing the indicated VHHs as a cell surface-displayed fusion of VHH-mIgG2a Fc-PDGFRβ TM were cultivated in the presence of 1 µg/mL doxycycline for 24h, followed by infection with VSV ΔG eGFP pseudotyped with SARS-CoV-2 spike (A) or SARS-CoV-1 spike (B). 8h post infection, eGFP-positive cells were measured by flow cytometry to quantify infection. Average values from three independent experiments ± SEM are plotted.

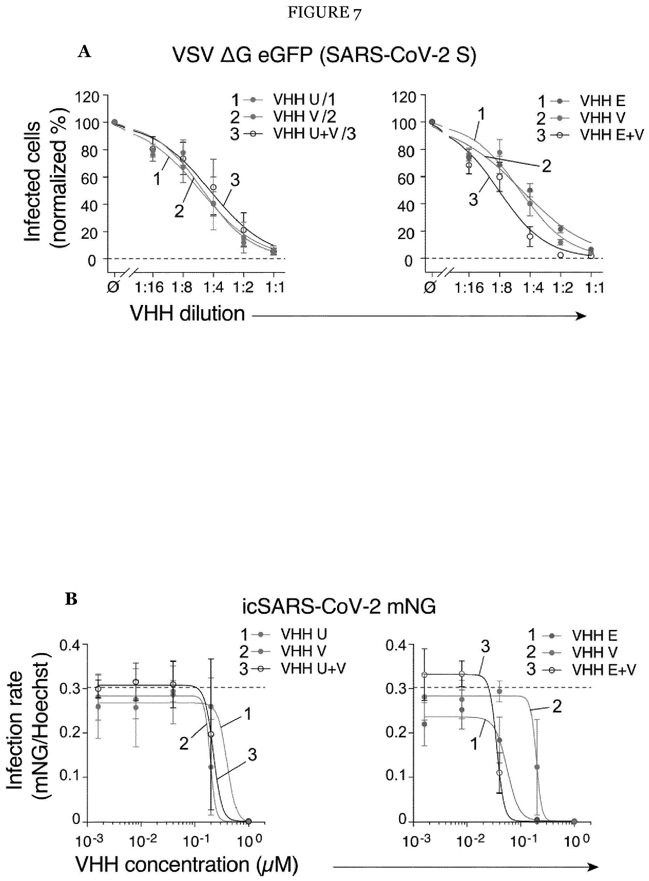

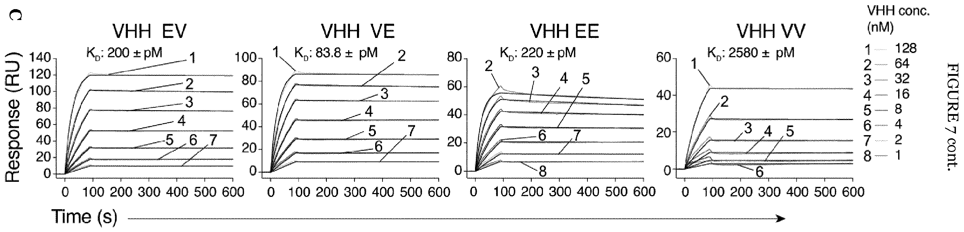

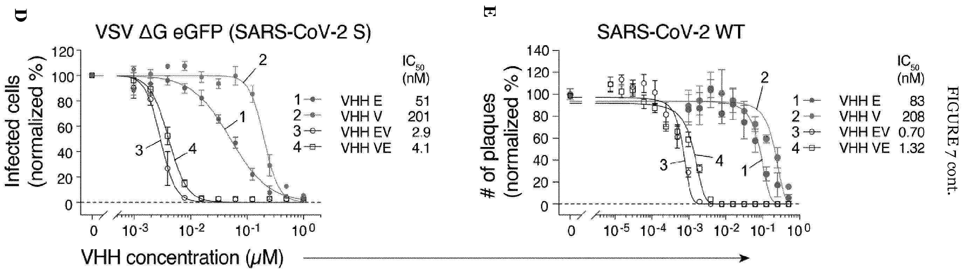

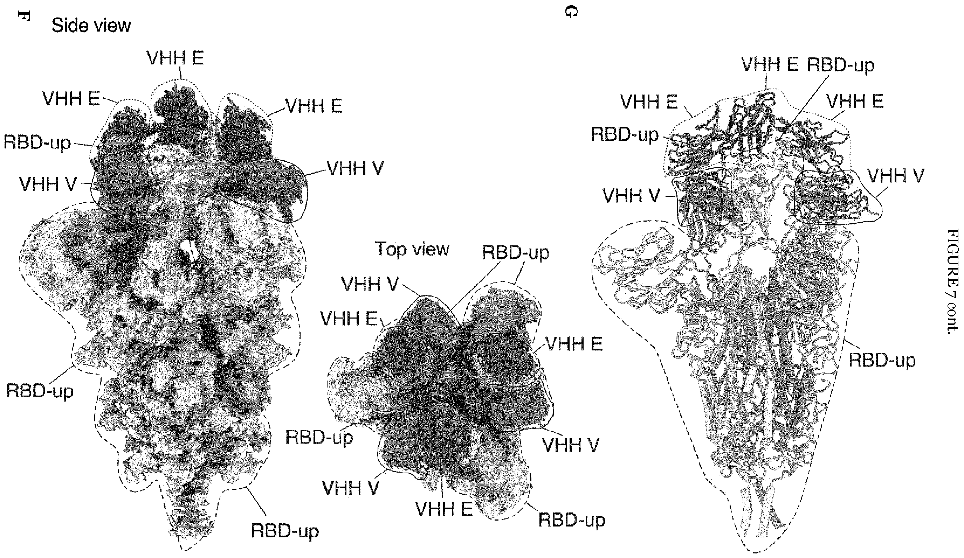

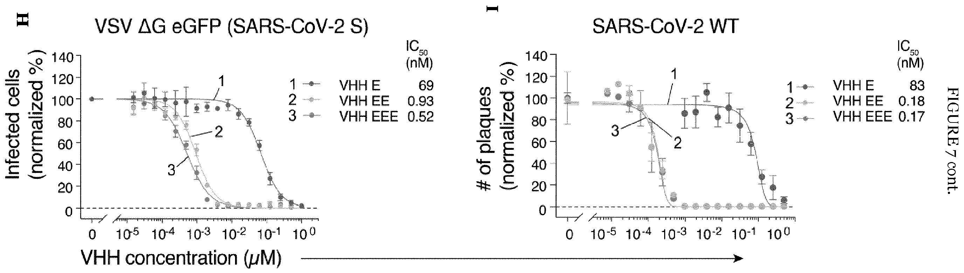

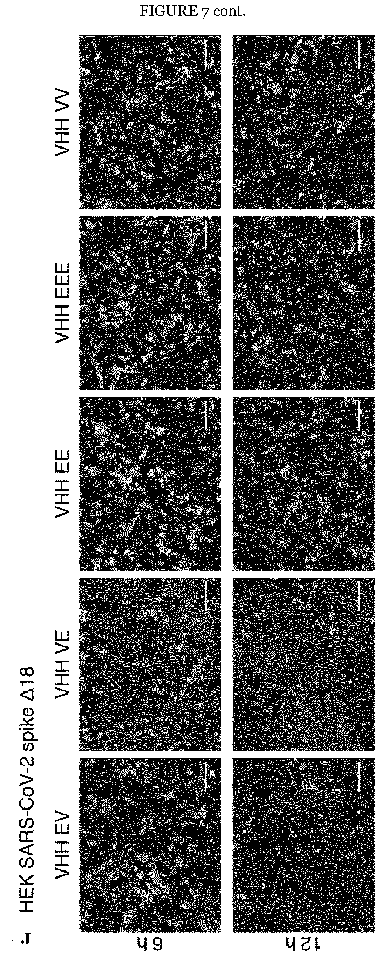

**Figure 7****:** shows that multivalent VHH fusions potentiate neutralization of SARS-CoV-2. (A) SARS-CoV-2 spike-pseudotyped VSV ΔG eGFP was incubated with 2-fold serial dilutions of 0.25 µM VHH E, 1 µM VHH U, 1 µM VHH V or the indicated combinations containing 50% of each VHH at 37° C for 1h. Vero E6 cells were subsequently incubated with the mixtures and infection quantified as in Fig. 1B. Normalized values from three independent experiments ± SEM are plotted. (B) Caco-2 cells were infected with mNeonGreen-expressing icSARS-CoV-2-mNG in the presence of the indicated VHH concentrations. Cells were fixed 48 h p.i., stained for DNA, and infection quantified by microscopy. (C) SARS-CoV-2 spike RBD was immobilized on SPR chips as in Fig. 1E and the indicated HA-tagged bivalent VHHs were injected for 90s, followed by dissociation for 510s. Dissociation constants (KD) were determined as before. (D, H) SARS-CoV-2 spike-pseudotyped VSV ΔG eGFP was incubated with the indicated concentrations of HA-tagged single, bivalent, or trivalent VHHs at 37°C for 1h, followed by infection of Vero E6 cells as in Fig. 1C. Normalized values from three independent experiments ± SEM and IC50 values are plotted. (E, I) SARS-CoV-2 was incubated with the indicated concentrations of HA-tagged VHHs, followed by plaque assay on Vero E6 cells as in Fig. 1D. Normalized values of three independent experiments ± SEM and IC50 values are plotted. (F, G) Cryo-EM reconstruction (F) and atomic models (G) of VHH VE in complex with trimeric SARS-CoV-2 spike. The biparatopic VHH binds to SARS-CoV-2 in the 3-up conformation; the resolution is 2.53 Å (0.143 FSC). (J) Flp-In 293 T-REx cell lines expressing SARS-CoV-2 S Δ18 as well as either eGFP or mCherry were treated with 1 µM of the indicated VHHs. Representative images were recorded after 6h or 12h as in Fig. 5E, and were from the same data set as those in Fig. 5E. Scale bars represent 100 µm.

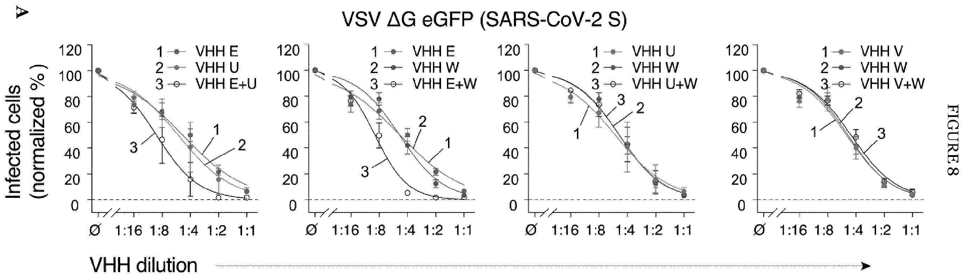

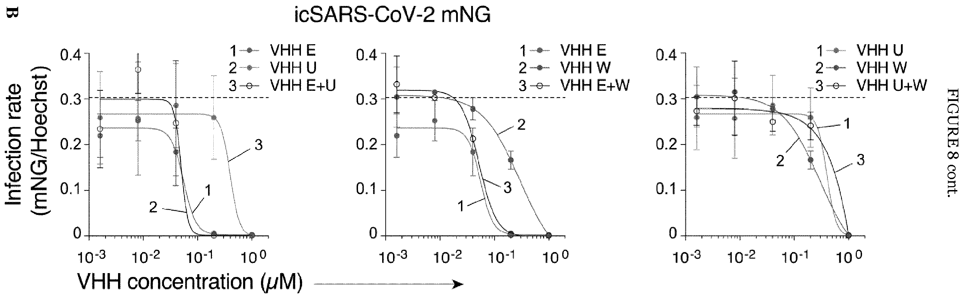

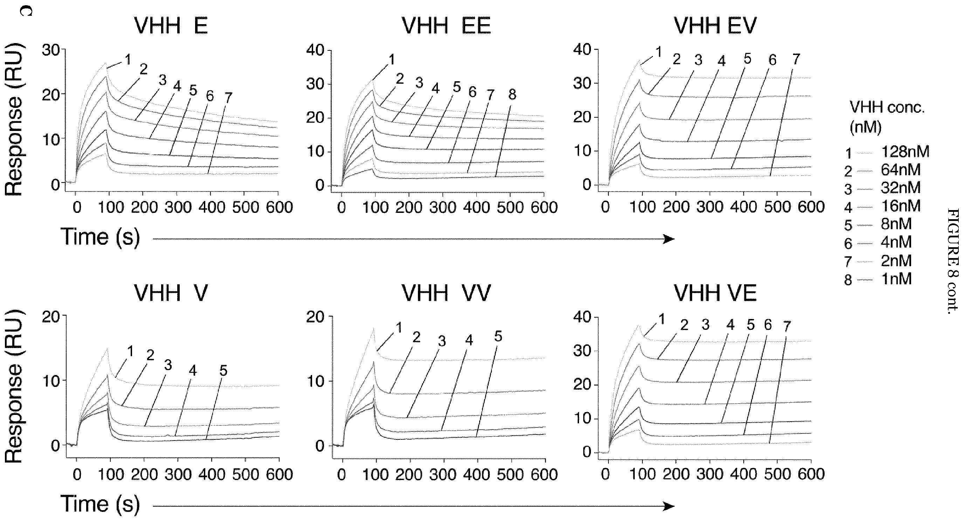

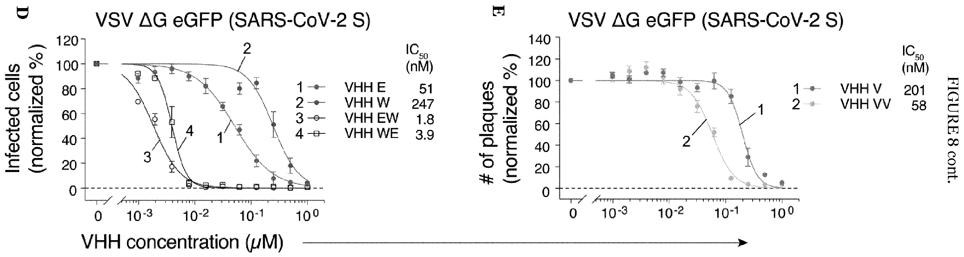

**Figure 8****:** shows that multivalent VHH fusions potentiate neutralization of SARS-CoV-2. (A) SARS-CoV-2 spike-pseudotyped VSV ΔG eGFP was incubated with 2-fold serial dilutions of 0.25 µM VHH E, 1 µM VHH U, 1 µM VHH W or the indicated combinations containing 50% of each VHH at 37° C for 1h. Vero E6 cells were subsequently infected and infection quantified as in Fig. 1B. Normalized values from three independent experiments ± SEM are plotted. (B) Caco-2 cells were infected with mNeonGreen-expressing icSARS-CoV-2-mNG in the presence of the indicated VHH combinations. 48h post infection, cells were fixed, stained for DNA, and infected cells quantified microscopically. One representative data set of three independent experiments is shown. (C) Biotinylated trimeric, soluble SARS-CoV-2 spike was immobilized on SPR chips and the indicated HA-tagged VHHs were injected for 90 s, followed by dissociation for 510 s. (D/E) SARS-CoV-2 spike-pseudotyped VSV ΔG eGFP was incubated with the indicated concentrations of HA-tagged monovalent or bivalent VHHs at 37° C for 1h, followed by infection of Vero E6 cells as in Fig. 1C. Normalized values from three independent experiments ± SEM and IC50 values are plotted.
**Figure 9****:** shows that simultaneous targeting of two independent epitopes with neutralizing VHHs prevents viral escape. (A) Genome structure of VSV SARS-CoV-2 S Δ18 eGFP. (B-E) Evolution experiment. (B) Replication-competent VSV SARS-CoV-2 S Δ18 eGFP at an MOI of 0.5 was incubated with different concentrations of the indicated VHHs and allowed to replicate on Vero E6 cells in 12-wells for 4 days. The fraction of infected (eGFP positive) cells and CPE was estimated microscopically and is plotted according to the indicated grey tone code. (C) Cleared supernatants from the wells indicated with a light circle in (B) were diluted 1:5 and used for a second round of replication on Vero E6 cells in the presence of the indicated VHH concentrations. Cleared supernatants were harvested as in (B). (D, E) Cell lysates from the wells indicated by circles in light color in B (D) and C (E) were subjected to next generation sequencing of the RBD to determine and quantify variants that had emerged at the interfaces to VHH E (Interface E) or to VHH U, V, or W (Interface UVW). (F) Wildtype VSV SARS-CoV-2 spike eGFP or plaque-purified escape mutants of VHH E (Si-if, Spike S494P), VHH U (S1-2h, Spike S371P), VHH V (S1-3b, Spike K378Q), VHH W (S1-4a, Spike S371P), and VHH LaM-4 (S1-10a, wt spike) at an MOI of 0.5 were incubated with the indicated VHH concentrations, and used for Vero E6 infection experiments as described in Fig. 1B. Infection was quantified by flow cytometry and normalized data from three independent experiments ± SEM is plotted.

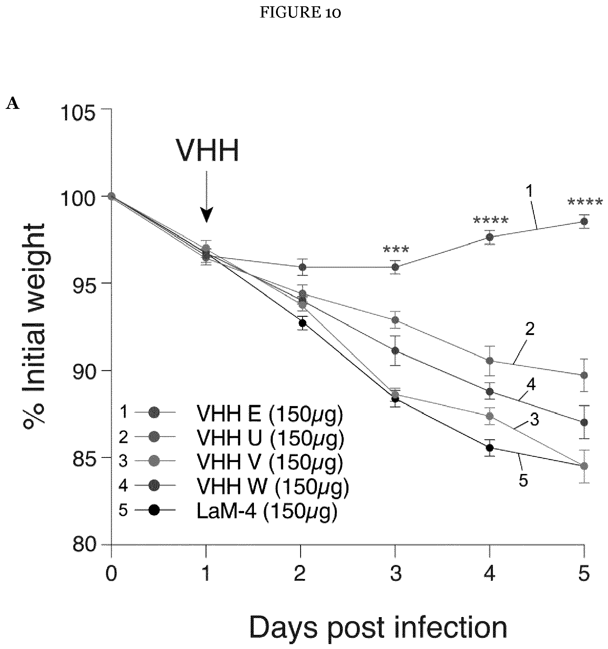

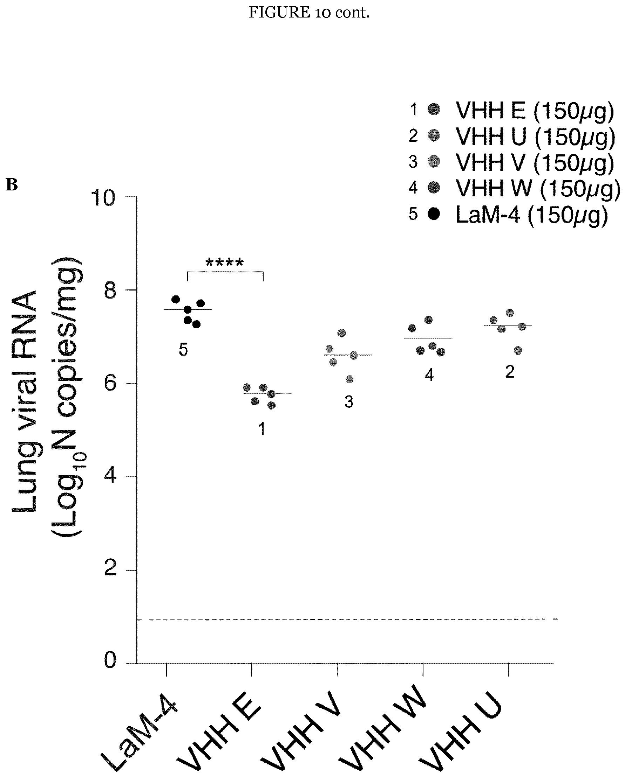

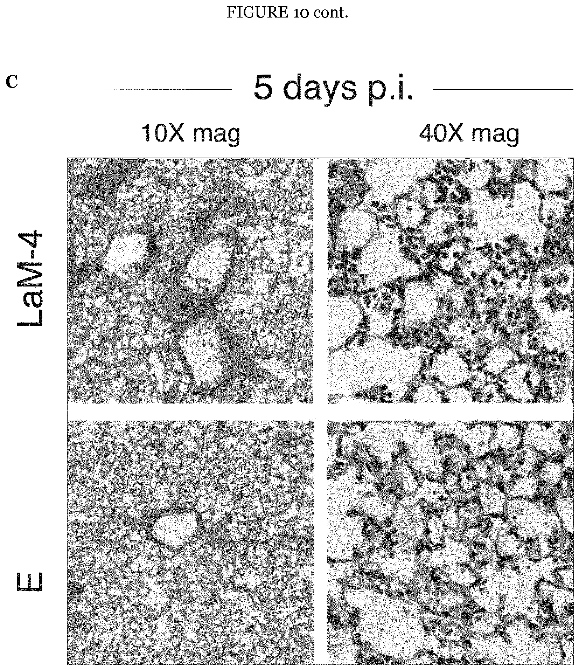

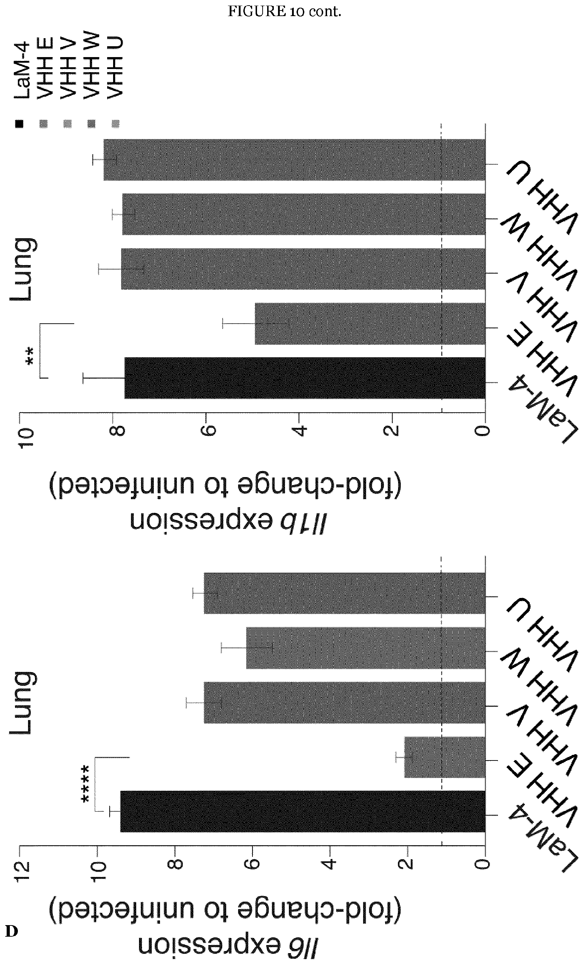

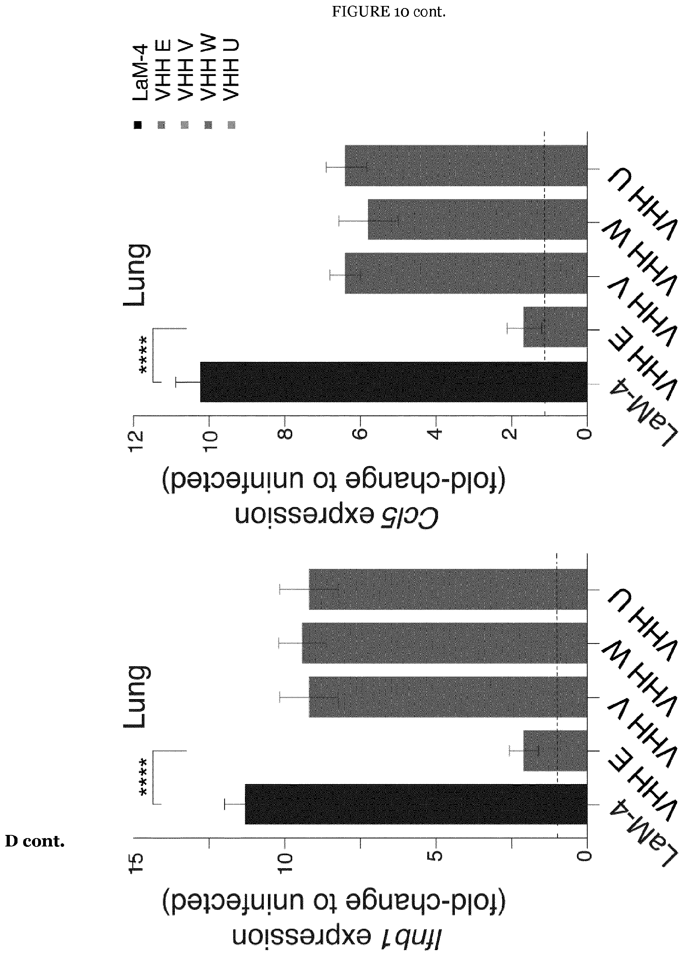

**Figure 10****:** shows that VHH E provides therapeutic protection in a mouse model of SARS-CoV-2 infection. 3-to-4-week-old male and female BALB/c mice intranasally (i.n) received hACE2-AdV (3.10⁸ PFU per mouse). 5 days post transduction, mice were i.n. infected with 2.105 FFU SARS-CoV-2. 1 day post infection (dpi), 150 µg VHH as indicated were intraperitoneally (i.p.) injected into each mouse. (A) Weight change was monitored daily for 5 days; (B) viral burden in the lungs was analyzed at 5 dpi by quantitative RT-PCR assay. Error bars indicate SD. (C) Hematoxylin and eosin staining of lung sections from 4-week-old BALB/c mice at 5 dpi after treatment with VHH E or control VHH (LaM-4). Images show low-magnification (left, scale bars: 100 µm), and high-power magnification (right, scale bar: 25 µm). Each image is representative of a group of 3 mice. (D) Fold change in gene expression of indicated cytokines and chemokines was determined by qRT-PCR, normalized to *Gapdh,* and compared with naive controls in lung homogenates at 5 dpi from indicated VHH or control VHH-treated mice (three experiments, n = 6-8 per group, Mann-Whitney test: *p < 0.05, ***p < 0.001, ****p < 0.0001). Dashed line indicates the average level of cytokine or chemokine transcript in naive mice.

The sequences show:

**Table 1: Sequences of the VHHs of the invention**

| **VHH-Designation** | **SEQ ID NO:** | **Domain** | **Amino Acid Sequence** |
|---|---|---|---|
| VHH-A | **1** | CDR1 | GFTLDDYA |
| VHH-A | **2** | CDR2 | ISSSDGST |
| VHH-A | **3** | CDR3 | AATYWSGSVWYMDAEEYDY |
| VHH-A | **4** | Full length | QVQLVETGGGLVQAGGSLRLSCAASGFTLDDYAI GWFRQAPGKEREGVSSISSSDGSTYYADSVKARF AISSDNAKNVVYLQMNSLKPEDTAVYYCAATYW SGSVWYMDAEEYDYWGQGTQVTVSA |
| VHH-B | **5** | CDR1 | GFGYDDHV |
| VHH-B | **6** | CDR2 | ISTDDGST |
| VHH-B | **7** | CDR3 | AATKGWHTEYDLKRLRDFDFET |
| VHH-B | **8** | Full length | QVQLVETGGGLVQPGGSLILSCQVDGFGYDDHV IGWFRQAPGKEREGVSYISTDDDRSTWYAESVK GRFAISKDSAEDTTYLQMNNLKAEDTAVYYCAA TKGWHTEYDLKRLRDFDFETWGQGTQVTVSS |
| VHH-C | **9** | CDR1 | GFTLDYYA |
| VHH-C | **10** | CDR2 | VSSSDGST |
| VHH-C | **11** | CDR3 | AHKQRDICSGYEFLYDY |
| VHH-C | **12** | Full length | QLQLVETGGGLVQPGGSLRLSCAASGFTLDYYAI GWFRQAPGKEREGVSCVSSSDGSTYYADSVKGR FTISRDSAKNTVYLQMNSLKPEDTAVYYCAHKQ RDICSGYEFLYDYWGQGTQVTVSS |
| VHH-D | **13** | CDR1 | GRTFGSYA |
| VHH-D | **14** | CDR2 | MTWSSMSP |
| VHH-D | **15** | CDR3 | AAVGLSVPFIETHEHEYDY |
| VHH-D | **16** | Full length | QVQLVETGGGRVQAGDSLRLSCVASGRTFGSYA VAWFRQAPGKEREFVAAMTWSSMSPNYADAVK GRFTISRDNAKNTAYLQMNRLAPEDTAVYYCAA VGLSVPFIETHEHEYDYWGQGTQVTVSS |
| VHH-E | **17** | CDR1 | GVTLDYYA |
| VHH-E | **18** | CDR2 | IGSSDGRT |
| VHH-E | **19** | CDR3 | ALTVGTYYSGNYHYTCSDDMDY |
| VHH-E | **20** | Full length | QVQLVETGGGFVQPGGSLRLSCAASGVTLDYYAI GWFRQAPGKEREGVSCIGSSDGRTYYSDSVKGR FTISRDNAKNTVYLQMNSLKPEDTAVYYCALTV GTYYSGNYHYTCSDDMDYWGKGTLVTVSS |
| VHH-F | **21** | CDR1 | GRTFSDYA |
| VHH-F | **22** | CDR2 | IGGGSI |
| VHH-F | **23** | CDR3 | AVAVGATTRPSWRSRVAEVYYDY |
| VHH-F | **24** | Full length | QVQLVETGGGLVQAGDSLRLSCTASGRTFSDYA LGWFRQAPGQEREFVAVIGGGSIYYAESVEGRF AISRDNARNTAYLQMNALKPEDTADYYCAVAVG ATTRPSWRSRVAEVYYDYWGQGTRVTVTT |
| VHH-G | **25** | CDR1 | EDTDA |
| VHH-G | **26** | CDR2 | ITWNADRT |
| VHH-G | **27** | CDR3 | AAAVGGSYYPPVPSEYDY |
| VHH-G | **28** | Full length | QLQLVETGGGLVQAGDSLRLSCAASEDTDAMG WFRQAPGKEREFVATITWNADRTYYADSVKGR FAISRDNAKSTVYLQMNSLKPEDTAVYYCAAAV GGSYYPPVPSEYDYWGQGTQVTVSS |
| VHH-H | **29** | CDR1 | ENIVNFGS |
| VHH-H | **30** | CDR2 | ITDGGST |
| VHH-H | **31** | CDR3 | VAARSNLAGWGEDFDS |
| VHH-H | **32** | Full length | QVQLVETGGGLVQTGDSLRLSCTASENIVNFGS MGWYRQAPGKQRELVAAITDGGSTIYTDSVKGR FIISRDNAKNTVFMQMNSLRPADSATYYCVAAR SNLAGWGEDFDSWGQGTQVTVSS |
| VHH-I | **33** | CDR1 | GSIFSIQA |
| VHH-I | **34** | CDR2 | IGASGNT |
| VHH-I | **35** | CDR3 | YALTRLPLPDGMDY |
| VHH-I | **36** | Full length | QLQLVETGGGLVQAGDSLRLSCVASGSIFSIQAM GWYRQAPGNERELVAGIGASGNTKYGDSVRGR FTISRDDEENKVYVQMNDLKTEDTAVYRCYALT RLPLPDGMDYWGEGTPVTVSS |
| VHH-J | **37** | CDR1 | GRAYA |
| VHH-J | **38** | CDR2 | ILWSGAS |
| VHH-J | **39** | CDR3 | AADLQFPLTTPQFHEYDV |
| VHH-J | **40** | Full length | QLQLVETGGGRVQAGDSLRLSCTASGRAYAMG WFRQAPGKEREFVAAILWSGASYADSVKDRFTI SRDNAKNTVYLQMNSLKPEDTAVYYCAADLQFP LTTPQFHEYDVWGQGTQVTVSS |
| VHH-K | **41** | CDR1 | GRAFNNNG |
| VHH-K | **42** | CDR2 | INWSGYST |
| VHH-K | **43** | CDR3 | AASLGSSWYSHDANDYPY |
| VHH-K | **44** | Full length | QLQLVETGGGLVQAGDSLRLSCAASGRAFNNNG IAWFRQAPGQEREFVAVINWSGYSTDYADSVKG RFTISRDNVKSVVYLQMNSLESEDTAVYYCAASL GSSWYSHDANDYPYWGQGTQVTVSS |
| VHH-L | **45** | CDR1 | AFTFGYYA |
| VHH-L | **46** | CDR2 | INYNGRDT |
| VHH-L | **47** | CDR3 | AAERASICTLQAD PYEY |
| VHH-L | **48** | Full length | QLQLVETGGGLVQPGGSLRLSCASSAFTFGYYAI GWFRQAPGKEREGIACINYNGRDTNFADSVKG RFTISRDDAKKTVTLQMNSLKPEDTANYYCAAE RASICTLQADPYEYWGQGTHVTVSS |
| VHH-M | **49** | CDR1 | GILFDYFA |
| VHH-M | **50** | CDR2 | INYNGRDT |
| VHH-M | **51** | CDR3 | AAERASICTLQEDPYEY |
| VHH-M | **52** | Full length | QLQLVETGGGLVQPGGSLTISCASSGILFDYFAIG WFRQAPGKEREGISCINYNGRDTNFADSVEGRF TISRDDAKKTVHLQMKDLKPEDTGTYFCAAERA SICTLQEDPYEYWGQGTQVTVSS |
| VHH-N | **53** | CDR1 | GDTLDYYS |
| VHH-N | **54** | CDR2 | MTRNGRI |
| VHH-N | **55** | CDR3 | AAPERFRLCTLRSLTDFGA |
| VHH-N | **56** | Full length | QLQLVETGGGLVQPGGSLRLTCTASGDTLDYYSI VWFRRSPGKEREGVSCMTRNGRIEYGDSVKGR FTISRDNAKRTVYLQMSNLKPEDTAVYYCAAPE RFRLCTLRSLTDFGAWGQGTQVTVSS |
| VHH-O | **57** | CDR1 | GFTLDYYA |
| VHH-O | **58** | CDR2 | ISSSGGST |
| VHH-O | **59** | CDR3 | AADRGIHSECSSWSADFAS |
| VHH-O | **60** | Full length | QLQLVESGGGLVQPGGSLRLSCAASGFTLDYYAI GWFRQAPGKEREGVSCISSSGGSTNYADSVEGR FTISRDNAKNTVYLQIRSLKPEDTAVYYCAADRG IHSECSSWSADFASWGQGTQVTVSS |
| VHH-P | **61** | CDR1 | GFILDYYD |
| VHH-P | **62** | CDR2 | ISSSGSTT |
| VHH-P | **63** | CDR3 | VLDPPCHQSESALHYERAF |
| VHH-P | **64** | Full length | QVQLVESGGGLVQPGGSLRLSCVLSGFILDYYDI GWFRQAPGKEREGVSCISSSGSTTYYGGSVKGRF TISRDNAKNTVYLEMNSLKAEDTGRYYCVLDPP CHQSESALHYERAFWGQGTQVTVSS |
| VHH-Q | **65** | CDR1 | GRTTRNDA |
| VHH-Q | **66** | CDR2 | ISWNGRNT |
| VHH-Q | **67** | CDR3 | AIGINDYGVPSRPNMYDY |
| VHH-Q | **68** | Full length | QLQLVESGGGLVQAGGSLKLSCAASGRTTRNDA MGWFRQAPGKEREFVAAISWNGRNTAYADSIK GRFTISRDNAKNTGYLQMNRLKPDDTAAYYCAI GINDYGVPSRPNMYDYWGQGTQVTVSS |
| VHH-R | **69** | CDR1 | GFTLDYYA |
| VHH-R | **70** | CDR2 | ISSGGGYT |
| VHH-R | **71** | CDR3 | AADDPHPQGPDVCSDSWLEYRYREL |
| VHH-R | **72** | Full length | QVQLVETGGGLVQPGGSLRLSCAAPGFTLDYYAI GWFRQAPGAEREGVSCISSGGGYTKYADSVKGR FTISRDSAKNTVYLQMNGLKPEDTAVYYCAADD PHPQGPDVCSDSWLEYRYRELWGQGTLVTVSS |
| VHH-S | **73** | CDR1 | ESILENNN |
| VHH-S | **74** | CDR2 | ISNSGGA |
| VHH-S | **75** | CDR3 | LRAHITPSRCLDFGA |
| VHH-S | **76** | Full length | QVQLVETGGGLVQPGGSLTLSCVASESILENNNV GWFRQGPGEEHEAVSCISNSGGAFYYGSGEGRF TISRDTAKNMVYLHMRDLKHEDSGIYHCLRAHI TPSRCLDFGAVGQGTQVTVSS |
| VHH-T | **77** | CDR1 | GFSLDYYA |
| VHH-T | **78** | CDR2 | ISSSGGST |
| VHH-T | **79** | CDR3 | AAAPSTYYGGSYYHQCLEYDY |
| VHH-T | **80** | Full length | QVQLVESGGGLVQPGGSLRLSCAASGFSLDYYAI GWFRQAPGKEREGVSCISSSGGSTNYADSVKGR FTISRDNAKNTVYLQMNSLKPEDTAVYYCAAAP STYYGGSYYHQCLEYDYWGQGTQVTVSS |
| VHH-U | **81** | CDR1 | GFTLDYYA |
| VHH-U | **82** | CDR2 | ISSSGGST |
| VHH-U | **83** | CDR3 | AAQSGSYYWCGSDWHEYEY |
| VHH-U | **84** | Full length | QLQLVESGGGLVQPGGSLRLSCAASGFTLDYYAI GWFRQAPGKEREGVSCISSSGGSTHFADSVKGR FTISRDNAKNTVYLQMNSLIPEDTAVYYCAAQSG SYYWCGSDWHEYEYWGQGTQVTVSS |
| VHH-V | **85** | CDR1 | GFTFSSYA |
| VHH-V | **86** | CDR2 | IYSDGST |
| VHH-V | **87** | CDR3 | ATEGSLGGWGRDFGS |
| VHH-V | **88** | Full length | QVQLVETGGGLVQPGGSLRLSCAASGFTFSSYA MGWARQVPGKGLEWVSYIYSDGSTEYQDSVKG RFTISRDNAKSTVYLQMNSLKPEDTAVYYCATE GSLGGWGRDFGSWGQGTQVTVSS |
| VHH-W | **89** | CDR1 | GFTLDYYA |
| VHH-W | **90** | CDR2 | ISSSGDST |
| VHH-W | **91** | CDR3 | AAQSGSYYWCGSDWHEYDY |
| VHH-W | **92** | Full length | QVQLVESGGGLVQPGGSLRLSCAASGFTLDYYAI GWFRQAPGKEREGVSCISSSGDSTHYVDSVKGR FTISRDNAKNTVYLQMNSLKPEDTAVYYCAAQS GSYYWCGSDWHEYDYRGQGTQVTVSS |

**SEQ ID NO: 93** shows the amino acid sequence of SARS-CoV2 spike protein:

10 20 30 40 50

60 70 80 90 100

110 120 130 140 150

160 170 180 190 200

210 220 230 240 250

260 270 280 290 300

310 320 330 340 350

360 370 380 390 400

410 420 430 440 450

460 470 480 490 500

510 520 530 540 550

560 570 580 590 600

610 620 630 640 650

660 670 680 690 700

710 720 730 740 750

760 770 780 790 800

810 820 830 840 850

860 870 880 890 900

910 920 930 940 950

960 970 980 990 1000

1010 1020 1030 1040 1050

1060 1070 1080 1090 1100

1110 1120 1130 1140 1150

1160 1170 1180 1190 1200

1210 1220 1230 1240 1250


     1260       1270


### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Provision of VHHs against epitopes on SARS-CoV-2 spike RBD

Camelid VHHs that bind two different epitopes on the SARS-CoV-2 spike RBD neutralize infection: One alpaca and one llama were immunized with the receptor binding domain (RBD) of SARS-CoV-2 spike as well as formalin-inactivated SARS-CoV-2 to elicit neutralizing heavy chain-only antibodies (Fig. 1A). 23 candidate VHHs (Fig. 2A) were identified by phage display, and confirmed binding of 10 hits by ELISA (Fig. 1B and Fig. 2B). Their neutralizing activity was assessed in single round infections with replication-deficient vesicular stomatitis virus (VSV) ΔG eGFP pseudotyped with SARS-CoV-2 spike Δ18 (Fig. 1C and Fig. 2C). Four VHHs, VHHs E, U, V, and W, potently neutralized infection in a dose-dependent manner, with an IC50 value of 60 nM for the most potent VHH, VHH E. Three published VHHs from a synthetic library did not neutralize (9), while the published VHH Ty1 (10) neutralized infection at comparable levels, although no complete neutralization was observed. As reported, SARS-CoV-1-specific VHH VHH 72 (11) did not impair infection with SARS-CoV-2-pseudotyped virus as a monomer (Fig. 2D), but potently inhibited SARS-CoV-1-pseudotyped virus (Fig. 2E). None of the four VHH hits neutralized SARS-CoV-1-pseudotyped virus (Fig. 2F). Plaque reduction neutralization tests (PRNTs) with SARS-CoV-2 on Vero E6 cells confirmed the neutralizing activity, yielding IC50 values ranging from 69 to 305 nM (Fig. 1D). Virus neutralization was further microscopically validated by quantifying the replication of an independent mNeonGreen expressing clone of SARS-CoV-2 on human Caco2 cells in the presence of VHHs over time (fig. 2G). The binding affinities of the VHHs to the RBD were measured by surface plasmon resonance (SPR) (Fig. 1E, Fig 2H), and the dissociation constants of 2 nM (VHH E), 21 nM (VHH U), 9 nM (VHH V) and 22 nM (VHH W) in the kinetic mode were obtained. An SPR-based binding competition assay revealed that the VHHs bind to two distinct regions: U, V, and W competed for the same binding interface on RBD (interface UVW), while VHH E bound to an independent RBD interface (interface E), and could bind to the RBD at the same time as U, V, or W (Fig. 1F).

### Example 2: Structural analysis of the interaction of VHHs and RBD

Binding epitopes of neutralizing VHHs on the spike RBD: The x-ray crystal structures of complexes of VHH E and VHH U with the SARS-CoV-2 RBD at 1.87 Å (Fig. 3, A to C), VHH V with the RBD and Fab CC12.3 (6) at 2.55 Å (Fig 3, D to E) and VHH W with the RBD and Fab CC12.3 at 2.73 Å (fig. 4, A to B) were determined. VHH E and U bind to distinct epitope sites on the RBD (Fig. 3A). VHH E employs its complementarity determining region (CDR) 1 and its unusually long CDR3 (22 amino acids) to bind to the ACE2 interface of the RBD (Fig. 3B). The epitope of VHH E is similar to that of potent SARS-CoV-2 neutralizing antibodies, including CC12.3 (12), H11-D4 (13) as well as VHHs MR17 and SR4 (14) (Fig. 4C). Notwithstanding, VHH E binds at an orientation that markedly differs from that of other neutralizing antibodies (Fig. 3F and Fig. 4C). Its CDR₃ adopts an extended beta-hairpin conformation and binds to the receptor binding site (RBS) using polar and hydrophobic interactions (Fig. 3B). Interestingly, 16 of 27 epitope residues that bind VHH E (Fig. 3B) are also involved in ACE2 binding (15) (Fig. 4D). Although VHH U binds to a distinct epitope on RBD, it should also compete with ACE2 for binding to RBD due to steric hindrance (Fig. 3F). VHH U uses its three CDRs to bind the RBD. The epitope of VHH U overlaps with the binding site of SARS-CoV-1 neutralizing antibody CR3022 (12) (Fig. 4C) and SARS-CoV-1 neutralizing VHH VHH 72 also covers the equivalent interface in the SARS-CoV-1 RBD (11).

Although most residues involved in the interaction of the SARS-CoV-2 RBD with VHH U are identical in the SARS-CoV-1 RBD, VHH U does not neutralize SARS-CoV-1 (Fig. 2F), hinting either at differences in accessibility of the SARS-CoV-1 RBD in the context of the trimeric spike, or small differences in the epitope that reduce binding affinity to below the threshold for neutralization. Binding of both VHHs E and U does not substantially alter the overall RBD fold (Cα RMSD = 0.37 Å between RBDS bound by E and U, or ACE2). VHH W adopts a structure that is virtually identical to VHH U (Cα RMSD = 0.48 Å) and engages the RBD in the same way (Fig. 3A, Fig. 3F, fig. 4A), consistent with the finding that 6 of the 7 amino acid differences between the two VHHs occur in the framework and are not involved in binding. Although VHH V binds to a similar epitope to VHH U and W, VHH V approached the RBD with a markedly distinct angle. In this case, CDR3 was mainly involved in binding. Again, no major changes in the overall structure of the RBD were observed on VHH U binding. Similar to VHH U, binding of VHH V should also compete with ACE2 binding due to steric hindrance (Fig. 3F). To confirm whether VHH binding to the RBD does compete with ACE2 binding on the cell membrane surface, a flow cytometry-based competition assays was performed and binding of fluorescently labeled RBD to ACE2-expressing HEK293T cells was quantified (fig. 4E). ACE2 binding was indeed impaired by all neutralizing VHHs in a dose-dependent manner, although inhibition was incomplete.

Neutralizing VHHs stabilize the SARS-CoV-2 spike in the RBD-up conformation: RBDs exist in an equilibrium of up- and down- conformations in the context of the trimeric spike on virions. Most unperturbed spike trimers exist in the configuration with none or one RBD up, while the 3-up conformation is barely populated at all (7, 8, 16). Only the up conformation of the RBD is compatible with ACE2 (17) binding, which likely induces further conformational changes that favor secondary proteolytic cleavage, dissociation of the Si subdomain, and eventually conversion to the post-fusion conformation. However, it is unknown how many RBDs must be in the up conformation to permit these transitions. To further interrogate the mechanism of action of the identified neutralizing VHHs, cryo-electron microscopy structures of the soluble, trimeric spike complex bound to individual VHHs using cryo-EM (7, 18) were determined. The initial analysis of the inventors focused on one representative VHH binding each of the two identified epitopes. Ab-initio reconstruction of VHH E bound to the trimeric spike revealed that the predominant complex contained all RBDs in the up conformation (3-up) with all three VHH E binding sites populated (Fig. 5A to B). This suggests that binding of VHH E stabilizes (or induces) the 3-up conformation, perhaps by mimicking the engagement of multiple peptidase domains of ACE2. The cryo-EM structures of trimeric spike in complex with VHH V also showed full coverage of all VHH binding sites (Fig. 5C to D). The major population of VHH V:spike complexes contained two RBDs in the up-conformation and one in the down-conformation. Apart from the RBD state, the overall structure of the trimeric spike and the RBD was not substantially changed by any of the VHHs. Importantly, the VHHs altered the abundance of the spike trimer populations with a 2- or 3-up configuration and confirmed the binding mode to be similar to the binding modes identified by macromolecular crystallography described above.

### Example 3: In vitro studies of neutralizing effect of the VHHs on RBD

Neutralizing VHHs trigger activation of the fusion machinery: ACE2 can only bind to RBDs in the up-conformation and is expected to trigger conformational changes required for secondary proteolytic processing and fusion. Premature activation of these steps will inactivate the fusion machinery, as the energetically favored post-fusion conformation is irreversible. To test whether stabilization of the RBDs in the up-conformation by VHHs activates the fusion machinery, a HEK 293-based cell line was generated that inducibly expresses the SARS-CoV-2 spike protein on the cell surface. Lacking the expression of the cognate receptor ACE2, the SARS-CoV-2 spike is not expected to mediate fusion of these cells. These cells were transfected with expression vectors for either eGFP or mCherry, the cells were mixed at an equal ratio, and treated with VHHs (Fig. 5E). Cell-cell fusion as a macroscopic readout for the activation of viral fusion proteins would result in cells containing both eGFP and mCherry. Untreated cells, or cells treated with control VHHs did not fuse or exhibit any signs of toxicity. However, when spike-expressing cells were incubated with VHH E, U, V, or W, cell-cell fusion could be detected, which was revealed by mixing of eGFP and mCherry fluorescence (Fig. 3E). VHH-induced fusion was already obvious after 6 hours in cells treated with VHH U or W, while cells treated with VHH E or V showed clear signs of fusion after 12h.

These findings show that stabilization of the RBD up-conformation by neutralizing VHHs favored conformational changes that mediate fusion. It remains unclear whether VHH binding is sufficient to induce fusion directly, or whether it facilitates secondary proteolytic processing of spike. Mixtures of cells expressing SARS-CoV-2 spike and ACE2, as well as SARS-CoV-2-infected cells, undergo spontaneous fusion at similar kinetics as observed here (19, 20). Cell-cell fusion mediated by SARS-CoV-1 spike has been shown to be dependent on protease activity (21). It is thus possible that VHH binding mimics binding of ACE2 and exposes otherwise inaccessible protease cleavage sites.

Activation of the fusion machinery has two potential outcomes: Either it facilitates infection by inducing fusion of viral membranes with cellular membranes (requiring proximity of both membranes), or it prematurely induces conformational changes of the spike in the absence of suitable membranes, thus irreversibly inactivating the fusion capacity. To rule out that the identified VHHs enhance infection, even if artificially brought into the proximity of the membrane, SARS-CoV-2 susceptible Vero cells were generated exposing neutralizing VHHs at their plasma membrane as fusions to an Fc fragment and a transmembrane domain (Fig. 6 A to B). Cells expressing neutralizing VHHs E, U, V, and W on their surface were partially protected from infection by SARS-CoV-2, but not SARS-CoV-i-pseudotyped virus. In contrast VHH 72 inhibited both viruses. Importantly, no enhancement of infection was observed.

Binding of VHHs to the RBD partly competes with ACE2 attachment of virions (Fig. 4E). Yet, it was also observed that binding of VHHs to SARS-CoV-2 spike protein activates fusion activity. The inventors conclude that, in addition to interference with virus attachment, VHH binding to the spike protein on virions promotes premature activation of the fusion machinery, leading to irreversible inactivation of the spike protein. As the most potent neutralizing VHH VHH E did not induce fusion more efficiently than the other VHHs, it is possible that the observed fusogenic activity is more pronounced in case of VHH U and W, while the effect of VHH E is mainly mediated by inhibition of virus attachment by competing ACE2 binding, or by interfering with the loss of S1.

Multivalent VHH fusions potentiate neutralization of SARS-CoV-2: In the course of natural adaptive immune responses, viruses face a polyclonal antibody response, which may enhance neutralizing activity by additive or synergistic effects (22). To test whether the four neutralizing VHHs act synergistically, the neutralizing activity of combinations of individual VHHs was compared. For direct comparison, starting concentrations of the individual VHHs were established that led to similar dose-response curves in a two-fold dilution series (Fig. 7A and Fig. 8A). Then neutralizing activities of the individual VHHs were compared with mixtures of VHHs containing 50% of the concentration of each VHH combined. Combinations of VHHs competing for the same epitope (VHHs U and V, U and W, or V and W) exhibited additive inhibition, i.e. mixtures containing half the concentrations of two VHHs neutralized to the same extent as the individual VHHs alone at full concentration (Fig. 7A, Fig. 8A). However, when VHHs binding to different epitopes were combined (VHHs E and U, E and V, or E and W), mixtures containing 50% of the two VHHs neutralized more efficiently than 100% of each of the individual VHHs. Combinations of VHHs binding independent epitopes were also more potent in preventing SARS-CoV-2 mNeonGreen replication in human cells (Fig 7B, Fig. 8B).

These results suggested that targeting multiple epitopes may be beneficial to interfere with infection. Thus, multivalent VHH fusions linked by flexible linkers were produced, capitalizing on the relatively unrestrained N- and C-termini of VHHs. Based on the modeled structure of VHH E and VHH V bound to the same RBD, it was determined that the C-terminus of VHH V and the N-terminus of VHH E were close enough (33 Å) to allow fusion with a 15 aa (GGGGS)3 linker (VHH VE). The distance between the C-terminus of VHH E and the N-terminus of VHH V, in contrast, was estimated to be greater than the length of a 15 aa linker. The reverse construct (VHH EV) with a (GGGGS)3 linker was thus expected to either bind to two different RBDs within a trimeric complex, to only employ one of its binding sites, or to induce some strain or distortion on the targeted RBD. The biparatopic VHHs VHH VE and EV were produced in bacteria, purified and subsequently subjected to SPR analysis (Fig. 7C). With apparent dissociation constants of 84 pM (VE) and 200 pM (EV), the biparatopic VHHs bound to the RBD 22- and 9-fold stronger than the best individual VHH (VHH E). While binding to trimeric spike (Fig. 8C) was confirmed as well, the more complex binding behavior precluded the application of defined models required to calculate affinities. In neutralization assays with SARS-CoV-2 S pseudotyped VSV, it was found that VE or EV neutralized with IC50 values of 4.1 or 2.9 nM, i.e., 18 or 12 times better than VHH E alone, respectively (Fig. 7D). Similar values for the biparatopic fusions VHH EW and WE (Fig. 8D) were observed. Neutralizing activity of VHH fusions as measured by PRNT with wildtype SARS-CoV-2 was improved even more, reaching IC50 values of 0.7 nM for EV and 1.32 nM for VE, i.e. 119 and 63-fold higher neutralizing potential than VHH E alone (Fig. 7E). Through examination of the cryo-EM reconstructions of VE in complex with trimeric spike (Fig. 7, F-G), it was observed that the biparatopic VHH VE bound to the same spike monomer and stabilized all RBDs in the up conformation, and that all six VHH binding sites were occupied on the spike. Complexes of the spike trimer with VHH V alone (Fig. 5C to D) contained two RBDs in the up-conformation, hence the formation of a 3-up spike trimer is dependent on addition of VHH E in the biparatopic VHH VE complex.

The cryo-EM structures of VHH E in complex with the trimeric spike suggested that three RBDs in the up conformation could also be bound by multivalent fusions of VHH E. Thus VHH EE and VHH EEE connected by GS-rich linkers of 15 aa length were produced and similarly their neutralizing activity was tested. VHH EE and VHH EEE neutralized VSV ΔG eGFP (SARS-CoV-2 S) with IC50 values in the pM range (930 and 520 pM, respectively), improving neutralizing potential 73- or 133-fold, respectively, as compared to VHH E (Fig. 7H). IC50 values in PRNT with wildtype virus were even lower, reaching 180 and 170 pM for VHH EE and EEE (Fig. 7I). Also other bivalent fusions were tested, including VHH VV, which are not expected to be able to engage multiple subunits of the trimeric spike with both their binding interfaces for steric reasons. As the inventors expected, VHH VV exhibited only a modest gain in affinity (Fig. 7C) and neutralizing activity (Fig. 8E).

Monovalent, multivalent and biparatopic VHHs boost SARS-CoV-2 neutralization by distinct mechanisms: Next, the mechanism by which multivalent VHHs enhance the neutralizing activity of individual VHHs was explored. In the cell fusion assay as described above, it was tested whether incubation of SARS-CoV-2 spike expressing cells with multivalent VHHs triggers spike-mediated cell-cell fusion (Fig. 7J). Indeed, VHHs EV and VE triggered extensive cell-cell fusion after 6h, and VE clearly exceeded the fusogenic activity of all individual VHHs. In contrast, and unlike their monomeric counterparts, multivalent VHHs EE, EEE, and VV did not induce obvious cell fusion after 6 or 12h. These findings suggested that multivalent VHHs enhance neutralizing activity by at least two distinct mechanisms: Since VHH VE is able to bind to a single spike monomer, to stabilize the RBD in the up conformation, and to substantially enhance VHH-triggered cell fusion, it is conceivable that the simultaneous binding of E and V (and perhaps E and W) substantially facilitates premature initiation of the post-fusion conformation. As VHH E binding alone already stabilizes the RBD 3-up conformation, this may involve additional conformational changes, possibly facilitating proteolytic processing of S2 to S2', or the bona fide dissociation of S1. On the contrary, the homodimeric and -trimeric VHHs EE and EEE did not induce fusion despite a substantial boost in neutralizing activity. It is clear that binding of VHH EEE is likely more potent in competing with ACE2 binding of virions due to higher avidity. Yet, the marked difference from VHH E alone, which did induce activation of the spike, suggests that crosslinking the individual spike subunits by EEE may in addition prevent crucial conformational changes necessary for fusion by restraining the available conformational landscape. This may include rearrangements required to enhance proteolytic processing or the conformational changes that go along with loss of S1. Of note, unlike described for more distantly related coronaviruses, S1 subunits dissociating from SARS-CoV-1 spike after receptor engagement are monomeric (23). This implies that individual S1 subunits dissociate in the course of fusion activity, a step likely perturbed by homodimeric and -trimeric VHH E (and V).

Targeting two independent vulnerable epitopes on the RBD prevents viral escape: In the course of prolonged infections or therapeutic settings, viruses with escape mutants emerge, which evade recognition and thus neutralization by specific antibodies. In this process, simultaneous emergence of escape mutants for two independent epitopes is significantly less likely. Thus, it was experimentally tested if combinations of VHHs targeting distinct epitopes, or our biparatopic VHHs indeed increased resistance to escape mutants. As an experimental model to study the viral evolution of SARS-CoV-2 spike, a chimeric VSV strain was generated that expresses eGFP and encodes SARS-CoV-2 spike Δ18 instead of its own glycoprotein (VSV eGFP SARS-CoV-2 S, see Fig. 5A)(24-26). The error rate of VSV RNA polymerase is one to two orders of magnitude higher than that of coronaviruses, allowing more rapid mutational sampling of the evolutionary landscape. VSV eGFP SARS-CoV-2 S replicated well in ACE2-containing Vero cells but did not infect BHK cells lacking ACE2, as expected. VSV eGFP SARS-CoV-2 S was preincubated at an MOI of 0.5 with different concentrations of individual or combinations of VHHs before adding the mixture to Vero E6 cells (Fig. 9B). Virus treated with control VHH (LaM-4 (27)) infected and killed cells within 1 or 2 days. The administration of individual neutralizing VHHs at a concentration that completely prohibited infection in a single round infection experiment (2.5 µM, Fig. 1C) resulted in more than 50% of cells being infected cells after prolonged incubation for 4 days, suggesting or selection of escape mutants. The inventors hypothesize that the diversity of mutations present in the inoculum combined with ongoing replication over four days were sufficient to allow outgrowth of escape mutants. To continue selection and isolate escape mutants, the wells with the highest dose of VHH that still allowed substantial infection (>10% eGFP positive cells) with low CPE were selected, the supernatant was harvested, and cells were lysed for RNA extraction. Supernatants were diluted 1:5, treated with different concentrations of the same VHHs, and were allowed to replicate for another five days on fresh Vero E6 cells (Fig. 9C). Virus derived from wells with single VHHs rapidly replicated in the presence of high concentrations of up to 12.5 µM of the respective VHHs E, U, V, and W, and infected all cells within less than five days (Fig. 9C).

Virus incubated with combinations of VHHs E and U, E and V, or E and W were only able to substantially replicate in the presence of 0.1 µM total VHH in the first round, i.e. non-neutralizing concentrations as determined in single round infection experiments (Fig 7, A to B, and fig. 8, A to B). Together, this experiment suggested that combining two neutralizing VHHs targeting independent epitopes prevented rapid emergence of fully resistant mutants. In the second round of evolution, virions treated with cocktails of E and U, or E and V were inhibited by similar VHH concentrations as in the first round, suggesting that no mutants escaping both VHHs had emerged. In contrast, some virus replication in the presence of the combination of E and W occurred in the presence of 2.5 µM VHHs in the second round of selection, indicating the late emergence of escape mutants (Fig. 9C). In a separate evolution experiment, virus replication in the presence of VHH EV and VE was tested using identical conditions. To validate the comparability of the experiments, selection with individual VHHs was repeated with identical results (data not shown). Virus replication in the presence of EV and VE only occurred at 4 nM VHH in the first round of selection and no substantial improvement was detected in a second selection round, suggesting that no escape mutants targeting both interfaces had emerged.

To analyze the emerging escape mutants, the RBD sequences encoded in viral RNAs were determined, which were purified from infected cells after one or two rounds of evolution. Sequence variants using a customized analysis pipeline were found to be of high quality and that no mutations accumulated in virions replicating in the presence of control VHHs. However, for virus replicating in the presence of individual neutralizing VHHs, around 75% of all reads contained a single point mutation in the structurally defined E or UVW interfaces of the RBD after one round of selection, while almost 100% of all reads exhibited the same mutations after two rounds of evolution. Escape mutants selected with VHH E contained an S494P mutation located in the key site in the VHH E:RBD interface. The observed mutation not only eliminates two hydrogen bonds interacting with CDR₃ of VHH E, but would also prevent the CDR beta sheet from forming the neighbouring hydrogen bond network (Fig. 3B). Of note, the same mutation was also observed in detailed evolutionary studies of viral escape to monoclonal antibodies and is found in clinical insolates (28). Mutants escaping recognition by VHH U and VHH W contained point mutation S371P, located in a loop region where most of hydrogen bonds between RBD and VHHs are formed (Fig. 3C and Fig. 4B). Escape mutants for VHH V carried the point mutation K378Q, eliminating two salt bridges and two hydrogen bonds between CDR₃ of VHH V and the RBD (Fig. 3E). Virus replicating in the presence of the VHH combinations E and U, E and V, or E and W accumulated individual point mutations in the interface with VHH E in the first round of evolution, but no virus that was mutated in both interfaces emerged. In the second round of evolution, still no virus variants containing mutations in both interfaces developed in the presence of VHH E and U, or E and V, while some virus with mutations in the E and W interface emerged in the presence of VHH E and W. Virus in the presence of permissive concentrations of EV or VE did not accumulate any escape mutations in the first round of evolution (Fig. 9D).

To further analyze escape mutants that had emerged in the presence of individual neutralizing VHHs, individual escape mutants from supernatants of the first round were plaque-purified, the virus was amplified, and point mutations were confirmed by Sanger sequencing. Next, the parental virus, the isolated escape mutants, or control isolates were subjected to neutralization experiments with different VHHs and quantified infection by flow cytometry (Fig. 9F). Parental virus VSV eGFP SARS-CoV-2 S as well as control isolates (Fig. 9F) were sensitive to VHHs E, U, V, W, EV, and VE as previously observed for replication-deficient pseudotyped VSV ΔG eGFP (SARS-CoV-2 S) (Fig. 1 and 7). VHH E escape mutant 'S1-1f' (spike S494P) was no longer sensitive to VHH E, but was still neutralized by VHH U, V, and W. Of note, VHHs EV and VE neutralized the S494P escape mutant better than V alone, suggesting that the VHH E component of the bivalent VHHs still helped neutralize, perhaps because the escape mutant allowed residual binding of VHH E if stabilized by the additional VHH V interface. VHH U and W escape mutants 'S1-2h' and 'S1-4a' (both carrying spike S371P) were still sensitive to VHH E, but were completely resistant to VHHs U, V, and W, demonstrating that a single point mutation in this interface allows escape from different VHHs. This finding is also consistent with the identified role of S371 in the RBD:VHH V interface (Fig. 3E). Again, biparatopic VHHs EV and VE neutralized infection more stringently than E alone, pointing at some residual activity of the VHH V component of the fusion. The escape mutant of VHH V, 'S1-3b', was still resistant to VHH E, albeit at somewhat lower efficacy, but was likewise resistant to VHHs U, V and W. As before, biparatopic VHHs VE and EV were more neutralizing than VHH E alone.

Taken together, it was found that the combined treatment of virions with VHHs targeting more than one epitope potently suppresses the emergence of escape mutants. The combination of monomeric VHHs illustrates the additional effects particularly well, as protection was dramatically improved as compared to individual VHHs, where replication was observed at 25 to 125-fold higher concentrations. Fusions of VHHs targeting distinct epitopes (biparatopic VHHs) were even more potent, and, importantly, also performed better on escape mutants.

### Example 4: Demonstration of in vivo efficacy of VHH E

VHH E provides therapeutic protection in a mouse model of SARS-CoV-2 infection: An important prerequisite for the development of therapeutic VHHs as a treatment option for SARS-CoV-2 infections is neutralization efficacy *in vivo.* To address this issue, mice expressing human ACE2 in the lung due to transduction with recombinant Adenovirus 5 (Ad5-hACE2) were infected intranasally with SARS-CoV-2 (29). Mice were injected intraperitoneally with 150 µg of control VHH LaM-4, VHH E, U, V, or W 24 hours post infection. A single injection of VHH E prevented weight loss (Fig. 10A) and reduced viral titers by a factor of ∼100 (Fig. 10B). Five days post infection, mice treated with LaM-4 showed substantial immune cell infiltration in peribronchiolar, perivascular, and alveolar sites compared with those receiving VHH E (Fig. 10C). At 5 dpi, lower transcript levels of several pro-inflammatory cytokines and chemokines (IFN-β, IL-6, IL-1β, CCL5) were detected in lung homogenates of mice treated with VHH E compared with those receiving control VHH (Fig. 10D). The data shows that VHHs provide a viable therapeutic option to interfere with SARS-CoV-2 replication in clinical settings.

### Materials and Methods

### Animals

Animal studies were carried out in accordance with the UK Animals (Scientific Procedures) Act. Mice were housed in individually ventilated cage (IVCs) with constant temperature (20-24 °C) and humidity (45 - 65%) with lighting on a fixed light/dark cycle (12 hours/12 hours). All efforts were made to minimize animal suffering. BALB/c mice were purchased from Jackson Laboratories.

### Cell lines

Syrian baby hamster kidney (BHK)-21 cell clone BSR-T7/5 (a kind gift of Sean Whelan, Harvard Medical School), human embryonic kidney (HEK) 293T cells (ATCC), and African green monkey Vero E6 cells (a kind gift of Ralf Bartenschlager, University of Heidelberg) were cultivated in DMEM containing 10% FBS and GlutaMax; human colorectal adenocarcinoma Caco-2 cells were cultured in DMEM complemented with 10% FBS, 2 mM L-glutamine, 100 µg/mL penicillin/ streptomycin (PS), and 1% non-essential amino acids (NEAA). Flp-In 293 T-REx cells (Thermo Fisher Scientific) inducibly expressing SARS-CoV-2 Spike Δ18 with a C-terminal Strep2-HA tag were generated using the Flp-In system (Thermo Fisher Scientific) according to the manufacturer's recommendations, and cultivated in DMEM supplemented with 10% FBS, Glutamax, 4 µg/mL blasticidine S, and 50 µg/mL hygromycin B. Vero E6 cells inducibly expressing cell surface-displayed VHHs were generated using lentiviral transduction with plnducer20-based vectors and cultivated in DMEM supplemented with 10% FBS, Glutamax, and 500 µg/mL G418. VHHs were expressed as fusions of Igκ signal peptide-HA-VHH-mIgG2a Fc-myc-PDGFRβ(TM).

### Viruses

All experiments involving the authentic virus were conducted in Biosafety Level 3 laboratories. SARS-CoV-2 clinical isolate SARS-CoV-2/human/Germany/Heinsberg-01/2020 was isolated from a throat swap of an infected patient at the University of Bonn, Germany, and was used for plaque reduction assays (39). To prepare larger stocks of virus, Vero E6 cells were infected with SARS-CoV-2 at a multiplicity of infection (MOI) of 0.01. Supernatants were harvested 2 days post infection (p.i.), cleared by centrifugation, and quantified by plaque assay. To prepare inactivated virus for camelid immunizations, virus was produced in cells covered with Opti-MEM: Clarified supernatants containing virus were mixed with 37% formaldehyde to obtain a final concentration of 4% formaldehyde and incubated at 4°C for 4 h. The virus suspension was overlaid on 7 ml of a 30% sucrose cushion in 20 mM Hepes pH 7.4,150 mM NaCl and virions were sedimented by ultracentrifugation in a SW 32 TI rotor at 4° C, 30.000 rpm for 2.5 h. Inactivated virus pellets from 28 mL of virus-containing supernatants were resuspended in 100 µl of 20 mM Hepes pH 7.4, 150 mM NaCl and the virus suspensions from five ultracentrifuge tubes were pooled. Inactivation of the virus was verified by the lack of replication in a Vero E6 infection experiment. Isolate SARS-CoV-2/human/USA/WA-CDC-WA1/2020 for in vivo experiments was obtained from the CDC (Atlanta, GA, USA). Recombinant SARS-CoV-2 clone icSARS-CoV-2-mNG expressing mNeonGreen (40), derived from isolate SARS-CoV-2/human/USA/WA-CDC-WA1/2020, was obtained from the World Reference Center for Emerging Viruses and Arboviruses (WRCEVA) at the UTMB (University of Texas Medical Branch) and was used for microscopy-based replication assays. To generate icSARS-CoV-2-mNG stocks, 200,000 Caco-2 cells were infected with 50 µl of virus stock in a 6-well plate, the supernatant was harvested 48 hours p.i., centrifuged, and stored at -80°C. Viral titers were determined based on mNeonGreen expression after serial dilutions.

To generate replication-deficient pseudotyped VSV strains for single round infections, VSV ΔG eGFP pseudotyped with VSV G, VSV ΔG eGFP (VSV G), from BSR-T7/5 cells using T7-expressing vaccinia virus (VACV) strain vTF7.3, pVSV eGFP dG (a kind gift from Connie Cepko, Harvard Medical School, Addgene plasmid # 31842), and T7-based expression vectors for VSV polymerase (pL), phosphoprotein (pP), nucleoprotein (pN), and glycoprotein (pG) (all kind gifts from Sean Whelan, Harvard Medical School) were first recovered using published procedures (41). VSV ΔG eGFP (VSV G) was amplified in BSR-T7/5 cells transiently transfected with VSV G expression vector pMD2.G (a kind gift from Didier Trono, EPFL, Addgene plasmid # 12259) at 34° C. VSV ΔG eGFP (SARS-CoV-1SΔ18) and VSV ΔG eGFP (SARS-CoV-2 SΔ18) were produced in BSR-T7/5 cells transiently transfected with pCAGGS SARS-CoV-1 SΔ18 or pCAGGS SARS-CoV-2 SΔ18, respectively, followed by infection with VSV ΔG eGFP (VSV G) and cultivation at 34° C. pCAGGS SARS-CoV-1 SΔ18 encodes aa 1-1237 of SARS-CoV-1 strain Frankfurt 1 (cloned from a template kindly provided by Stephan Poehlmann, German Primate Center), while pCAGGS SARS-CoV-2 SΔ18 encodes aa 1-1255 of SARS-COV-2/human/China/Wuhan-Hu-1/2019 (cloned from a codon-optimized template kindly provided by Stephan Poehlmann). C-terminal truncations were introduced to avoid retention of spike proteins in the ER/Golgi and maximize exposure at the plasma membrane. Virus-containing supernatants were clarified by centrifugation and stored at -80° C. Viral titers were determined by infection of Vero E6 with dilution series of the virus for 8 hours, followed by quantification of green fluorescent cells by flow cytometry. Supernatants were shown to be free of VSV G-pseudotyped virus, as no infection of BSR-T7/5 cells was observed.

To generate replication competent chimeric VSV strains encoding eGFP and SARS-CoV-2 SΔ18 in place of the VSV glycoprotein, we inserted the coding sequence of SARS-CoV-2 SΔ18 (aa 1-1255 of SARS-CoV-2/human/China/Wuhan-Hu-1/2019) into pVSV eGFP dG. Replication-competent virus was recovered from BSR-T7/5 cells infected with VACV vTF7.3, transfected with pVSV eGFP SARS-CoV-2 SΔ18, pL, pP, pN, and pG, and cultivated at 34° C. Virus was amplified in Vero E6 cells at 34° C and virus-containing supernatants were clarified and stored at -80° C. Viral titers were determined by infection of Vero E6 with dilution series of the virus for 8 hours, followed by quantification of green fluorescent cells by flow cytometry. Chimeric virus strain VSV eGFP SARS-CoV-2 SΔ18 was confirmed to infect primate ACE2 expressing VeroE6 cells, but not BSR-T7/5 cells.

### Proteins

### Expression and purification of SARS-CoV-2S RBD

Cloning and expression of the receptor-binding domain (RBD) (residues 319-541) of spike protein from SARS-CoV-2/human/China/Wuhan-Hu-1/2019 (GenBank: QHD43416.1) was described earlier (12). In brief, the coding sequence was cloned into a customized pFastBac vector and fused with an N-terminal gp67 signal peptide and C-terminal His6 tag. A recombinant bacmid DNA was generated using the Bac-to-Bac system (Thermo Fisher Scientific). Baculovirus was generated by transfecting Sf9 cells with purified bacmid DNA using FuGENE HD (Promega), and subsequently used to infect suspension cultures of High Five cells (Thermo Fisher Scientific) at an MOI of 5 to 10. Infected High Five cells were cultivated at 28 °C for 72h, shaking at 110 r.p.m. for protein expression. The supernatant was then concentrated using a 10 kDa MW cutoff Centramate cassette (Pall Corporation). The RBD protein was purified by Ni-NTA, followed by size exclusion chromatography, and buffer exchanged into 20 mM Tris-HCl pH 7.4 and 150 mM NaCl. Fluorescent RBD was produced by reaction with DyLight 488 NHS ester (Thermo Fisher Scientific) in 100 mM sodium pohosphate pH 8.0, 150 mM NaCl, pH 8.0, followed by desalting with 7K MWCO Zeba spin desalting columns (Thermo Fisher Scientific).

### Expression and purification of VHHs

VHH coding sequences were cloned into pHEN6-based bacterial, periplasmic expression vectors with C-terminal HA-His6 or LPETG-His6 tags using Gibson cloning (New England Biolabs). Multivalent VHHs were either directly cloned into pHEN6, or first assembled in pBluescript II (KS) + cloning vectors lacking promoters by Gibson cloning, followed by subcloning into pHEN6 using conventional ligation with T4 ligase. VHHs were produced in Escherichia coli WK6 transformed with the respective expression vectors. Expression cultures were grown in Terrific Broth (TB), and expression induced with 1 mM IPTG at an OD600 of 0.6, followed by cultivation at 30° C for 16 h. Bacterial pellets were resuspended in TES buffer (200 mM Tris-HCl pH 8.0, 0.65 mM EDTA, 0.5 M sucrose), and periplasmic extracts generated by osmotic shock in 0.25x TES, followed by Ni-NTA purification and either desalting by PD MiniTrap G-25 columns (GE Healthcare Life Sciences) (small scale purifications), or gel filtration with Superdex 75 Increase 10/300 GL or HiLoad 16/600 Superdex 75 pg columns (medium or large scale purifications) in 20 mM Hepes pH 7.4, 150 mM NaCl. Protein was concentrated using Amicon concentration cells with 3 kDa or 10 kDa cutoff (Millipore).

### Expression and purification of CC12.3 Fab

CC12.3 Fab was produced as described before (12). In brief, the coding sequences of heavy and light chain of CC12.3 Fab were cloned into phCMV3. ExpiCHO cells were transiently co-transfected at a ratio of 2:1 (HC:LC) using ExpiFectamine^{™} CHO Reagent (Thermo Fisher Scientific) according to the manufacturer's instructions. The supernatant was collected at 10 days post-transfection. The Fabs were purified with a CaptureSelect^{™} CHi-XL Affinity Matrix (Thermo Fisher Scientific) followed by size exclusion chromatography.

### Expression and purification of SARS-CoV-2 Spike

Soluble, trimeric SARS-CoV-2 spike was expressed as a fusion of aa 1-1208 of SARS-CoV-2/human/China/Wuhan-Hu-1/2019 containing the mutations R682G, R683S, R685S, K986P, V987P (S2-P), a C-terminal T4 fibritin trimerization motif, an HRV3C protease cleavage site, a TwinStrepTag and an His8 tag from a mammalian expression vector based on paH as described before (7). For some cryo-EM experiments, the SARS-CoV-2 spike HexaPro mutant with additional proline mutations was used (42). In brief, protein was produced in FreeStyle 293F cells transfected with FreeStyle MAX reagent (Thermo Fisher Scientific) or Expi293F cells transfected with ExpiFectamine 293 (Thermo Fisher Scientific). The ectodomain was purified from filtered supernatant on Streptactin XT resin (IBA Lifesciences), followed by gel filtration on a HiLoad^{®} 16/600 Superdex 200 column in 50 mM Tris pH 8, 200 mM NaCl. Soluble spike protein was biotinylated using UV-traceable ChromaLink Biotin (SoluLink).

### VHH Library Generation

To raise heavy chain-only antibodies against SARS-CoV-2 spike, one llama (6 injections, each week), and one alpaca (4 injections, every two weeks) were immunized. The llama was immunized six times with 200 µg SARS-CoV-2 S RBD, complemented with 300 µL formaldehyde-inactivated virus (corresponding to ∼109 pfu) in the last two injections. The alpaca was immunized 4x with 200 µg SARS-CoV-2 S RBD. GERBU Adjuvant Fama (GERBU Biotechnik GmbH) was used as an adjuvant in all immunizations. In two (llama) or one (alpaca) injection, respectively, antigen and adjuvant were injected separately. VHH plasmid libraries in the M13 phagemid vector pD (pJSC) were generated as described before (43). In brief, RNA from peripheral blood lymphocytes was extracted and used as a template to generate cDNA using three sets of primers (random hexamers, oligo(dT), and primers specific for the constant region of the alpaca heavy chain gene). VHH coding sequences were amplified by PCR using VHH-specific primers, cut with AscI and NotI, and ligated into pJSC linearized with the same restriction enzymes. E. coli TG1 cells (Agilent) were electroporated with the ligation reactions and the obtained ampicillin-resistant colonies were harvested, pooled, and stored as glycerol stocks.

### VHH identification

SARS-CoV-2 spike RBD-specific VHHs were obtained by phage display and panning with a protocol modified from Schmidt et al. (43). E. coli TG1 cells containing the VHH library were infected with helper phage VCSM13 to produce phages displaying the encoded VHHs as pIII fusion proteins. Phages in the supernatant were purified and concentrated by precipitation. Phages presenting RBD-specific VHHs were enriched using enzymatically or chemically biotinylated RBDs immobilized to MyOne Streptavidin T1 Dynabeads (Thermo Fisher Scientific). The retained phages were used to infect E. coli ER2738 and subjected to a second round of panning. 96 E. coli ER2837 colonies yielded in the second panning were grown in 96-well plates and VHH expression induced with IPTG. VHHs leaked into the supernatant were tested for specificity using ELISA plates coated with control protein MBP or SARS-CoV-2 RBD. Bound VHHs were detected with HRP-coupled rabbit anti-E-Tag antibodies (Bethyl), HRP-coupled MonoRab^{™} Rabbit Anti-Camelid VHH Antibody (GenScript), and the chromogenic substrate TMB. Reactions were stopped with 1 M HCl and absorption at 450 nm was recorded. Positive candidates were sequenced, and representative VHHs cloned into bacterial expression vectors for further analysis.

### ELISA

To test VHH candidates, SARS-CoV-2 spike RBD or the control protein mannose binding protein (MBP) in PBS were immobilized on ELISA plates at a concentration of 1µg/mL over night. Serial 10-fold dilutions of HA-tagged VHHs in 10% FBS/PBS were incubated with the immobilized antigen, followed by incubation with HRP-coupled anti-HA (clone 6E2, 1:5000, Cell Signaling), and the chromogenic substrate TMB. Reactions were stopped with 1 M HCl and absorption measured at 450 nm.

### Surface plasmon resonance spectroscopy

Surface plasmon resonance experiments were performed using a Biacore 8K instrument (GE Healthcare). The flow system was cleaned using the maintenance "Desorb" function (Desorb Kit, GE Healthcare). The system was flushed with running buffer (20 mM HEPES pH 7.4, 150 mM NaCl, 0.05% Tween 20) and all steps were performed at 25°C chip temperature. Before immobilization, a streptavidin functionalized sensor chip (Series S Sensor Chip SA, GE Healthcare) was conditioned with three consecutive 1-minute injections of 1 M NaCl in 50 mM NaOH (10 µL/min). After immobilization of biotinylated SARS-CoV2-RBD (50 nM, 5 µL/min, 300 s) on the sensor chip flow cell 2, the flow system was washed using 50% isopropanol in 1 M NaCl and 50 mM NaOH. For kinetic binding measurements, various VHHs were injected (30 µL/min, association: 90 s, dissociation: 180 s) over both flows. For epitope binning, the VHHs were pairwise tested for competitive binding using the ABA-injection feature. After each cycle, the surfaces were regenerated with a 120 s injection step of 10 mM glycine pH 2.1. Data were collected at a rate of 10 Hz. The binding data were double referenced by blank cycle and reference flow cell subtraction. Processed data were fitted by the 1:1 interaction model using Biacore Insight Evaluation Software (version 2.0. 15.12933).

### Crystallization and structural determination

VHH E/VHH U/RBD, VHH V/CC12.3/RBD, and VHH W/CC12.3/RBD complexes were formed by mixing each of the protein components at an equimolar ratio and incubated overnight at 4°C. The final concentration for the complexes used for crystallization screening ranged from 13.5 to 17.8 mg/ml. Crystallization screening was set-up by the vapor diffusion method in sitting drops containing 0.1 µl of protein and 0.1 µl of reservoir solution with the 384 conditions of the JCSG Core Suite (Qiagen) using our custom-designed robotic CrystalMation system (Rigaku) at Scripps Research. Diffraction-quality crystals were obtained in the following conditions:
VHH E/VHH U/RBD complex (13.5 mg/mL): 20% PEG-3000, 0.1 M citrate pH 5.5 at 20°C,
VHH V/CC12.3/RBD complex (17.8 mg/mL): 20% PEG 3350, 0.2 M Na2HPO4, pH 9.1 at 20°C,
VHH W/CC12.3/RBD complex (17.6 mg/mL): 1.0 M Li-chloride, 10% PEG-6000, 0.1 M Bicine pH 9.0 at 20°C.

All crystals appeared within 7 days after tray set-up. Before flash cooling in liquid nitrogen for X-ray diffraction studies, crystals were equilibrated in reservoir solution supplemented with 10% ethylene glycol at day 7.

Diffraction data were collected at cryogenic temperature (100 K) at either Stanford Synchrotron Radiation Lightsource (SSRL) on the new Scripps/Stanford beamline 12-1 with a beam wavelength of 0.97946 Å or Advanced Photon Source (APS) on the beamline 23ID-B with a beam wavelength of 1.03317 Å, and processed with HKL2000 (44). Structures were solved by molecular replacement using PHASER (45) with template derived from PDB 6XC7 (12), 7JMW (46) and 6WAQ (11). Iterative model building and refinement were carried out in COOT (47) and PHENIX (48) respectively. Epitope and paratope residues, as well as their interactions, were identified by accessing PISA at the European Bioinformatics Institute (http://www.ebi.ac.uk/pdbe/prot_int/pistart.html) (49).

### Cryo-EM sample preparation and imaging

Spike trimer (2.4 mg/ml S2-P (7) for VHH E and VHH V; HexaPro (42) for VHH VE) and VHH were mixed in a 1:6 molar ratio for VHH E and VHH V, and 1:4 for VHH VE, followed by incubation on ice for 10 minutes. A 3-µl aliquot of the sample solution was applied to glow-discharged UltrAuFoil Gold 200 mesh (R 2/2 geometry) grids (Quantifoil Micro Tools GmbH) or CryoMatrix^{®} holey grids with amorphous alloy film (Zhenjiang Lehua Technology Co.,Ltd) in a Vitrobot Mk IV (Thermo Fisher Scientific) at 4 °C and 100% humidity (blot 10 s, blot force 3). Cryo-EM data collection was performed with EPU Thermo Fisher Scientific) using a Krios G3i transmission-electron microscope (Thermo Fisher Scientific) operated at 300 keV in the Karolinska Institutet 3D-EM facility. Images were acquired in nanoprobe EFTEM mode with a slit width of 20 eV using a GIF 967 energy filter (Ametek) and a K3 detector (Ametek) during 1.5 seconds with a dose rate of 66.7 e-/px/s resulting in a total dose of 0.81 e-/Å2 fractionated into 60 movie frames. Motion correction, CTF-estimation, fourier binning (to 1.02 Å/px), picking and extraction in 600 pixel boxes were performed on the fly using Warp (50).

A total of 6,468 (VHH E), 9,861 (VHH V), and 12,606 (VHH VE) micrographs were selected based on an estimated resolution cutoff of 4 Å and defocus below 2 microns and 245,000 (VHH E), 509,302 (VHH V), and 9821,221 (VHH VE), particles were picked by Warp (50). Extracted particles were imported into cryoSPARC V2.15.0 (51) for 2D classification, 3D classification and non-uniform 3D refinement. The particles were processed with C1 symmetry. For VHH E and VHH VE last homogenous refinement step was done with C3 symmetry. After 2D classification clean classes with high resolution features were retained and used to build ab-initio 3D reconstructions. These were further processed for heterogeneous refinement that resulted in reconstructions showing high-resolution structural features in the core of the spike. One round of homogenous refinement followed by non-uniform refinement resulted in a final reconstruction to an overall resolution of 2.95 Å for spike VHH E, (0.143 FSC) using 115,034 particles, 3.14 Å for spike VHH V using 166,568 particles, and 2.47 Å for spike VHH VE using 380,925 particles. For the VHH VE dataset the particles from refinement job containing angular information was migrated from cryoSPARC to Relion3.1 (52) for 3D classification without alignment and classified into four classes using reconstruction in cryoSPARC as reference map (low-pass filtered to 25A). One class containing 92,938 particles where densities for all six VHHs were visible was refined in cryoSPARC to a final overall resolution of 2.53Å.

### Cryo-EM model building and structure refinement

The structure of the spike protein trimer PDB:6ZXN (53) was used as a starting model for model building. The VHH structures bound to RBD were determined using models built with the crystallography data from this study. Structure refinement and manual model building were performed using COOT and PHENIX (48) in interspersed cycles with secondary structure and geometry restrained. All structure figures and all EM density-map figures were generated with UCSF ChimeraX (54).

### ACE2-RBD binding assay

HEK 293T cells were transfected with expression vectors for ACE2-tagRFP-t and detached in PBS with 1 mM EDTA. 0.04 µM DyLight 488-labeled SARS-CoV-2 RBD was incubated with different concentrations of VHHs at room temperature (RT) for 20 min. ACE2-expressing cells were subsequently incubated with RBD-VHH mixtures on ice for 20 min. ACE2-tagRFP-t and DyLight 488 positive cells were subsequently quantified using a MACS Quant VYB flow cytometer.

### SARS-CoV-2 spike fusion assay

Flp-In 293 T-REx cells inducibly expressing SARS-CoV-2 spike Δ18-Strep2-HA were reversely transfected with pCAGGS eGFP or pCAGGS mCherry using Lipofectamine 2000 (Thermo Fisher Scientific). 4 h post transfection, cells were detached by scraping, washed, and seeded in tissue culture-treated CellCarrier-96 Ultra Microplates (Perkin Elmer) at a density of 100,000 cells per well in phenol red-free full medium without antibiotics. 2 h later, SARS-CoV-2 spike Δ18 expression was induced by the additions of doxycycline at a final concentration of 1 µg/mL. Cells were treated with the indicated concentrations of VHHs 20 h post transfection. Representative images were recorded using the 10x objective of a Leica SP8 confocal microscope after 6 h. Cells were fixed 12 h post treatment and representative images recorded accordingly.

### Neutralization assay with VSV ΔG eGFP (SARS-CoV-1/2) (infection assay)

Single round infection experiments with replication-deficient VSV ΔG eGFP (SARS-C0V-1) and VSV ΔG eGFP (SARS-CoV-2) were performed in Vero E6 cells. 48-well plates were seeded with 3.5-104 Vero E6 cells per well. The next day, 104 infectious units (IU) of VSV ΔG eGFP (SARS-CoV-1/2) in 50 uL DMEM (without FBS) (titered to achieve ∼30% infection) were mixed with 50 uL VHH dilution in DMEM (without FBS), yielding the indicated final VHH concentrations. Virus mixtures were incubated at 37° C for 1h, and subsequently used to infect Vero E6 cells. The inoculum was removed after 1h and cells were covered with 0.5 mL full DMEM (FBS). After 7h at 37° C (8h p.i.), cells were trypsinized, fixed, and eGFP-positive cells quantified using a MACS Quant VYB flow cytometer. Inhibitory concentration 50 (IC50) values were calculated using the 'log(inhibitor) vs. normalized response' equation in GraphPad Prism.

### Neutralization assays with SARS-CoV-2 wt (Plaque reduction neutralization test - PRNT)

Plaque reduction assays with SARS-CoV-2/human/Germany/Heinsberg-01/2020 were performed in Vero E6 cells. 24-well plates were seeded with 1.5-105 Vero E6 cells per well. The next day, VHHs at a concentration of 1 µM were subjected to a two-fold dilution series. 120 µl of each VHH dilution was mixed with 120 µl of OptiPROTM SFM cell culture media (Thermo Fisher Scientific) containing 80 pfu of SARS-CoV-2. After 1 h at 37°C, 200 µl of each mixture was added to 24-well plates. After 1 h at 37°C, the inoculum was removed and cells were overlaid with a 1:1 mixture of 1.5% carboxymethylcellulose (Sigma Aldrich) in 2x MEM (Biochrom) with 4% FBS (Thermo Fisher Scientific). After a 3-day incubation at 37°C, cells were fixed with 6% formaldehyde and stained with 1% crystal violet in 20% ethanol. Plaques were counted manually. Inhibitory concentration 50 (IC50) values were calculated using the 'log(inhibitor) vs. normalized response' equation in GraphPad Prism.

### Neutralization assays with SARS-CoV-2 mNeonGreen (replication assay)

Microscopy-based neutralization experiments with SARS-CoV-2 clone icSARS-CoV-2-mNG were performed with Caco-2 cells. 104 cells/well were seeded in 96-well plates. The next day, cells were infected with icSARS-CoV-2-mNG at an MOI of 1.1 in media containing 5% FBS and serial dilutions of the indicated VHHs. Cells were subsequently cultivated for 48 h in the presence of the VHHs, fixed with 2% PFA, and stained with 1 µg/mL Hoechst 33342 at 37°C for 10 min. The staining solution was removed and exchanged to PBS. To quantify infection rates, images were recorded with a Cytation3 instrument (Biotek). Total (Hoechst positive) and infected (mNeonGreen positive) cells were quantified using the Gen5 Software (Biotek). Inhibitory concentration 50 (IC50) values were calculated as the half-maximal inhibitory dose using 4-parameter nonlinear regression (GraphPad Prism).

### Identification and isolation of escape mutants

To test the emergence or presence of escape mutants of replication-competent VSV SARS-CoV-2 SΔ18 eGFP in the presence of VHHs, virus was replicated in the presence of serial dilutions of VHHs on Vero E6 cells (26). 1.4-105 cells/well were seeded into 12-well plates. The next day, 0.5 mL virus dilution (equivalent to an MOI of 0.5) in DMEM (3% FBS, PS) was incubated with 0.5 mL VHH dilution in DMEM (3% FBS, PS) at room temperature for 30 min. The mixture was subsequently transferred to 12-wells with Vero E6 cells and cultivated at 37° C for four days. The fraction of eGFP-positive cells as well as cytopathic effect (CPE) were evaluated and selected wells harvested for further cultivation: Cells were lysed in RLT PLUS buffer containing 1% β-mercaptoethanol, followed by purification of RNA with the RNeasy Mini Kit (Qiagen). Supernatants were cleared and stored at 4° C. 1:5 dilutions in DMEM (3% FBS, PS) were used to infect cells for a second round of selection under otherwise identical conditions. eGFP-positive cells and CPE were evaluated 5days post infection and selected supernatants and cell lysates harvested.

To quantify escape variants of virus replicated in the presence of neutralizing VHHs, viral RNA was reversely transcribed into cDNA using a SARS-CoV-2 spike specific primer (FS1957 - 5'-ACTGCTGGAATGGCAGGAAC-3'). The RBD coding sequence flanked by additional 70 bp was amplified by PCR using Phusion DNA polymerase (New England Biolabs) and primer FS1956 (5'-TCTGAGCGAGACAAAGTGCACC-3') and FS1957, and subjected to Tn5-mediated tagmentation and incorporation of barcoded adapters. Amplicons were sequenced on an Illumina MiSeq machine (v2 nano, 2x150 bp paired-end). After sequencing, FASTQ files were examined using FastQC (Version 0.11.9) and multiqc (Version 1.9). For the alignment, the SARS-CoV-2 RBD reference was indexed using bowtie2-build (Version 2.4.1) and samtools (Version 1.10). The reads where aligned using bowtie2 (Version 2.4.1). Conversion into BAM file, sorting and indexing was performed using samtools. BAM files were examined with QualiMap (Version 2.2.2-dev) and multiqc. Variant calling was performed using default GATK HaplotypeCaller (Version 4.1.8.1), and variants were inspected in bam.files using IGV.

To isolate defined escape mutants, resistant virus was amplified from individual virus plaques grown on Vero E6 cells. 1.4-105 Vero E6 cells per well were seeded into 12-wells. The next day, 10-fold serial dilutions of supernatants from virus replicated in the presence of VHH E, U, V, W, E and U, E and V, E and W, or La-M4 (round 1) were prepared and incubated in the presence or absence of 1 µM of VHHs at RT for 30 min. Vero E6 cells were infected for 30 min at 37° C, followed by overlay of cells with 0.75% agarose, MEM, 2% FBS, PS, 25 mM Hepes with or without the respective VHHs. Two days post infection, fluorescent plaques grown in the presence of individual VHHs were identified and marked. No VHH-resistant fluorescent plaques emerged from supernatants of the combinations VHH E and U, E and V, or E and W. A plug of the agarose on top of marked plaques was removed with a Pasteur pipette and incubated in 500 uL DMEM for 4h at 4° C. 48-wells were infected with 250 µL of this virus dilution and cultivated in DMEM (2% FBS, PS, 25 mM Hepes) at 37° C until all cells were eGFP positive. Supernatants were stored at 4° C and cells lysed in RLT PLUS buffer containing 1% β-mercaptoethanol, followed by purification of RNA with the RNeasy Mini Kit (Qiagen). Viral RNA was reversely transcribed into cDNA using a SARS-CoV-2 spike specific primer. The RBD coding sequence flanked by additional 70 bp was amplified by PCR and sequenced by Sanger sequencing. 6/6 plaques resistant to VHH E exhibited the point mutation S494P, 5/5 plaques resistant to VHH U and 5/5 plaques resistant to VHH W encoded the point mutant S371P, and 8/8 plaques resistant to VHH V contained the point mutation K378Q. 4/4 plaques from virus cultivated in the presence of control VHH La-M4 did not contain any mutations. Selected clones were amplified in 15 cm dishes of Vero E6 cells and cleared supernatants stored at -80° C.

### Animal infection experiments

Animal infection experiments with SARS-CoV-2/human/USA/WA-CDC-WA1/2020 were carried out in accordance with the UK Animals (Scientific Procedures) Act. 3-4 week old BALB/c mice of both sexes were administered 3-10⁸ pfu of AdV-ACE2 (obtained from Stanley Perlman, University of Iowa Viral Vector Core) as per published protocol (29). Five days after AdV transduction, each animal was inoculated with 2·10⁵ focus forming units (ffu) of SARS-CoV-2 via intranasal route. 1 day post-infection, mice were treated via i.p route with 150 µg of the anti-SARS-CoV-2 RBD VHHs as indicated in figure legends or isotype control (La-M4). Weights were monitored on a daily basis, and animals were sacrificed 5 days post infection. Anti-SARS-CoV-2 VHH or control-treated mice were euthanized and perfused with 20 mL of PBS after serum collection via cardiac puncture and before tissue harvest.

### RNA extraction and quantitative RT PCR

Tissue sections harvested from sacrificed animals were weighed and homogenized with zirconia beads in 1ml of DMEM supplemented with 2% heat-inactivated FBS. Tissue homogenates were centrifuged at 10,000 rpm for 5 min and RNA was extracted using the RNeasy kit (Qiagen) according to the manufacturer's instructions. Primers were designed to target the conserved region of viral N-gene using SARS-CoV-2 (MN908947) sequence as a guide (L Primer: 5'-ATGCTGCAATCGTGCTACAA-3'; R primer: 5'-GACTGCCGCCTCTGCTC-3').

Concatenated segments of the viral N gene were cloned into the PCR-II topo vector (Invitrogen) to generate an RNA standard curve. Viral RNA levels were quantified with the primer/probe set for N-gene, compared to the standard curve and normalized to mg of tissue.

### Cytokine mRNA measurements

RNA was isolated from lung homogenates as described above. cDNA was synthesized from DNase-treated RNA using the HighCapacity cDNA Reverse Transcription kit (Thermo Fisher Scientific) with the addition of RNase inhibitor following the manufacturer's protocol. Cytokine and chemokine expression was determined using TaqMan Fast Universal PCR master mix (Thermo Scientific) with commercial primers/probe sets specific for 116 (Mm.PT.58.10005566), Il1b (Mm.PT.58.41616450), Ccl₅ (Mm.PT.58.43548565), Ifnbi (Mm.PT.58.30132453.g), and results were normalized to Gapdh (Mm.PT.39a.1) levels. Fold change was determined using the 2-ΔΔCt method comparing treated mice to naive controls.

### Histopathological analyses

Animals were euthanized, and tissues were harvested before lung inflation and fixation. The lung was inflated with 10% neutral buffered formalin using a 3 mL syringe and catheter inserted into the trachea. Inflated lungs were kept in a 40 mL suspension of neutral buffered formalin for 7 days before further processing. Tissues were embedded in paraffin, and sections were stained with hematoxylin and eosin. Tissue sections were visualized using a Leica DM6B microscope equipped with a Leica DFC7000T camera....

### REFERENCES

The references are:
1. J. Abraham, Passive antibody therapy in COVID-19. Nat. Rev. Immunol. 20 (2020), pp. 401-403.
2. A. Iwasaki, Y. Yang, The potential danger of suboptimal antibody responses in COVID-19. Nat. Rev. Immunol. 20 (2020), pp. 339-341.
3. J. R. Ingram, F. I. Schmidt, H. L. Ploegh, Exploiting VHHs' Singular Traits. Annu. Rev. Immunol. 36, 695-715 (2018).
4. E. A. F. Simões, L. Bont, P. Manzoni, B. Fauroux, B. Paes, J. Figueras-Aloy, P. A. Checchia, X. Carbonell-Estrany, Past, Present and Future Approaches to the Prevention and Treatment of Respiratory Syncytial Virus Infection in Children. Infect. Dis. Ther. 7 (2018), pp. 87-120.
5. M. Hoffmann, H. Kleine-Weber, S. Schroeder, N. Krüger, T. Herrler, S. Erichsen, T. S. Schiergens, G. Herrler, N.-H. Wu, A. Nitsche, M. A. Müller, C. Drosten, S. Pöhlmann, SARS-CoV-2 Cell Entry Depends on ACE2 and TMPRSS2 and Is Blocked by a Clinically Proven Protease Inhibitor Article SARS-CoV-2 Cell Entry Depends on ACE2 and TMPRSS2 and Is Blocked by a Clinically Proven Protease Inhibitor. Cell. 181, 1-10 (2020).
6. T. F. Rogers, F. Zhao, D. Huang, N. Beutler, A. Burns, W. He, O. Limbo, C. Smith, G. Song, J. Woehl, L. Yang, R. K. Abbott, S. Callaghan, E. Garcia, J. Hurtado, M. Parren, L. Peng, S. Ramirez, J. Ricketts, M. J. Ricciardi, S. A. Rawlings, N. C. Wu, M. Yuan, D. M. Smith, D. Nemazee, J. R. Teijaro, J. E. Voss, I. A. Wilson, R. Andrabi, B. Briney, E. Landais, D. Sok, J. G. Jardine, D. R. Burton, Isolation of potent SARS-CoV-2 neutralizing antibodies and protection from disease in a small animal model. Science (80-. ). 369, eabc7520 (2020).
7. D. Wrapp, N. Wang, K. S. Corbett, J. A. Goldsmith, C. L. Hsieh, O. Abiona, B. S. Graham, J. S. McLellan, Cryo-EM structure of the 2019-nCoV spike in the prefusion conformation. Science (80-.). 367, 1260-1263 (2020).
8. A. C. Walls, Y. J. Park, M. A. Tortorici, A. Wall, A. T. McGuire, D. Veesler, Structure, Function, and Antigenicity of the SARS-CoV-2 Spike Glycoprotein. Cell. 181, 281-292.e6 (2020).
9. J. D. Walter, C. A. J. Hutter, I. Zimmermann, J. Earp, P. Egloff, M. Sorgenfrei, L. M. Hürlimann, I. Gonda, G. Meier, S. Remm, S. Thavarasah, P. Plattet, M. A. Seeger, bioRxiv, in press, doi:10.1101/2020.04.16.045419.
10. L. Hanke, L. Vidakovics Perez, D. J. Sheward, H. Das, T. Schulte, A. Moliner-Morro, M. Corcoran, A. Achour, G. B. Karlsson Hedestam, B. M. Hällberg, B. Murrell, G. M. McInerney, An alpaca VHH neutralizes SARS-CoV-2 by blocking receptor interaction. Nat. Commun. 11, 4420 (2020).
11. D. Wrapp, D. De Vlieger, K. S. Corbett, G. M. Torres, N. Wang, W. Van Breedam, K. Roose, L. van Schie, M. Hoffmann, S. Pöhlmann, B. S. Graham, N. Callewaert, B. Schepens, X. Saelens, J. S. McLellan, Structural Basis for Potent Neutralization of Betacoronaviruses by Single-Domain Camelid Antibodies. Cell. 181, 1004-1015.615 (2020).
12. M. Yuan, H. Liu, N. C. Wu, C.-C. D. Lee, X. Zhu, F. Zhao, D. Huang, W. Yu, Y. Hua, H. Tien, T. F. Rogers, E. Landais, D. Sok, J. G. Jardine, D. R. Burton, I. A. Wilson, Structural basis of a shared antibody response to SARS-CoV-2. Science (2020), doi:10.1126/science.abd2321.
13. J. Huo, A. Le Bas, R. R. Ruza, H. M. E. Duyvesteyn, H. Mikolajek, T. Malinauskas, T. K. Tan, P. Rijal, M. Dumoux, P. N. Ward, J. Ren, D. Zhou, P. J. Harrison, M. Weckener, D. K. Clare, V. K. Vogirala, J. Radecke, L. Moynié, Y. Zhao, J. Gilbert-Jaramillo, M. L. Knight, J. A. Tree, K. R. Buttigieg, N. Coombes, M. J. Elmore, M. W. Carroll, L. Carrique, P. N. M. Shah, W. James, A. R. Townsend, D. I. Stuart, R. J. Owens, J. H. Naismith, Neutralizing VHHs bind SARS-CoV-2 spike RBD and block interaction with ACE2. Nat. Struct. Mol. Biol., 1-9 (2020).
14. D. Li, T. Li, H. Cai, H. Yao, B. Zhou, Y. Zhao, W. Qin, C. A. J. Hutter, Y. Lai, J. Bao, J. Lan, G. Wong, M. Seeger, D. Lavillette, bioRxiv, in press, doi:10.1101/2020.06.09.143438.
15. Q. Wang, Y. Zhang, L. Wu, S. Niu, C. Song, Z. Zhang, G. Lu, C. Qiao, Y. Hu, K. Y. Yuen, Q. Wang, H. Zhou, J. Yan, J. Qi, Structural and Functional Basis of SARS-CoV-2 Entry by Using Human ACE2. Cell. 181, 894-904.09 (2020).
16. B. Turoňová, M. Sikora, C. Schürmann, W. J. H. Hagen, S. Welsch, F. E. C. Blanc, S. von Bülow, M. Gecht, K. Bagola, C. Hörner, G. van Zandbergen, J. Landry, N. T. D. de Azevedo, S. Mosalaganti, A. Schwarz, R. Covino, M. D. Mühlebach, G. Hummer, J. Krijnse Locker, M. Beck, In situ structural analysis of SARS-CoV-2 spike reveals flexibility mediated by three hinges. Science (80-. )., eabd5223 (2020).
17. W. Song, M. Gui, X. Wang, Y. Xiang, Cryo-EM structure of the SARS coronavirus spike glycoprotein in complex with its host cell receptor ACE2. PLOS Pathog. 14, 61007236 (2018).
18. J. Pallesen, N. Wang, K. S. Corbett, D. Wrapp, R. N. Kirchdoerfer, H. L. Turner, C. A. Cottrell, M. M. Becker, L. Wang, W. Shi, W. P. Kong, E. L. Andres, A. N. Kettenbach, M. R. Denison, J. D. Chappell, B. S. Graham, A. B. Ward, J. S. McLellan, Immunogenicity and structures of a rationally designed prefusion MERS-CoV spike antigen. Proc. Natl. Acad. Sci. U. S. A. 114, E7348-E7357 (2017).
19. S. Xia, M. Liu, C. Wang, W. Xu, Q. Lan, S. Feng, F. Qi, L. Bao, L. Du, S. Liu, C. Qin, F. Sun, Z. Shi, Y. Zhu, S. Jiang, L. Lu, Inhibition of SARS-CoV-2 (previously 2019-nCoV) infection by a highly potent pan-coronavirus fusion inhibitor targeting its spike protein that harbors a high capacity to mediate membrane fusion. Cell Res. 30, 343-355 (2020).
20. S. Liu, A. Hossinger, A. Hornberger, O. Buravlova, S. Müller, S. F. Lichtenthaler, M. Neumann, P. Denner, I. M. Vorberg, "Highly efficient intercellular spreading of protein misfolding mediated by viral ligand-receptor interactions" (Cold Spring Harbor Laboratory, 2020), , doi: 10.1101/2020.06.26.173070.
21. G. Simmons, S. Bertram, I. Glowacka, I. Steffen, C. Chaipan, J. Agudelo, K. Lu, A. J. Rennekamp, H. Hofmann, P. Bates, S. Pöhlmann, Different host cell proteases activate the SARS-coronavirus spike-protein for cell-cell and virus-cell fusion. Virology. 413, 265-274 (2011).
22. J. ter Meulen, E. N. van den Brink, L. L. M. Poon, W. E. Marissen, C. S. W. Leung, F. Cox, C. Y. Cheung, A. Q. Bakker, J. A. Bogaards, E. van Deventer, W. Preiser, H. W. Doerr, V. T. Chow, J. de Kruif, J. S. M. Peiris, J. Goudsmit, Human Monoclonal Antibody Combination against SARS Coronavirus: Synergy and Coverage of Escape Mutants. PLoS Med. 3, e237 (2006).
23. F. Li, M. Berardi, W. Li, M. Farzan, P. R. Dormitzer, S. C. Harrison, Conformational States of the Severe Acute Respiratory Syndrome Coronavirus Spike Protein Ectodomain. J. Virol. 80, 6794-6800 (2006).
24. J. B. Case, P. W. Rothlauf, R. E. Chen, Z. Liu, H. Zhao, A. S. Kim, L. M. Bloyet, Q. Zeng, S. Tahan, L. Droit, M. X. G. Ilagan, M. A. Tartell, G. Amarasinghe, J. P. Henderson, S. Miersch, M. Ustav, S. Sidhu, H. W. Virgin, D. Wang, S. Ding, D. Corti, E. S. Theel, D. H. Fremont, M. S. Diamond, S. P. J. Whelan, Neutralizing Antibody and Soluble ACE2 Inhibition of a Replication-Competent VSV-SARS-CoV-2 and a Clinical Isolate of SARS-CoV-2. Cell Host Microbe (2020), doi: 10.1016/j.chom.2020.06.021.
25. F. Schmidt, Y. Weisblum, F. Muecksch, H. H. Hoffmann, E. Michailidis, J. C. C. Lorenzi, P. Mendoza, M. Rutkowska, E. Bednarski, C. Gaebler, M. Agudelo, A. Cho, Z. Wang, A. Gazumyan, M. Cipolla, M. Caskey, D. F. Robbiani, M. C. Nussenzweig, C. M. Rice, T. Hatziioannou, P. D. Bieniasz, Measuring SARS-CoV-2 neutralizing antibody activity using pseudotyped and chimeric viruses. J. Exp. Med. 217 (2020), doi:10.1084/jem.20201181.
26. A. Baum, B. O. Fulton, E. Wloga, R. Copin, K. E. Pascal, V. Russo, S. Giordano, K. Lanza, N. Negron, M. Ni, Y. Wei, G. S. Atwal, A. J. Murphy, N. Stahl, G. D. Yancopoulos, C. A. Kyratsous, Antibody cocktail to SARS-CoV-2 spike protein prevents rapid mutational escape seen with individual antibodies. Science (80-. )., eabd0831 (2020).
27. P. C. Fridy, Y. Li, S. Keegan, M. K. Thompson, I. Nudelman, J. F. Scheid, M. Oeffinger, M. C. Nussenzweig, D. Fenyo, B. T. Chait, M. P. Rout, A robust pipeline for rapid production of versatile VHH repertoires. Nat Methods. 11, 1253-1260 (2014).
28. Y. Weisblum, F. Schmidt, F. Zhang, J. DaSilva, D. Poston, J. C. C. Lorenzi, F. Muecksch, M. Rutkowska, H.-H. Hoffmann, E. Michailidis, C. Gaebler, M. Agudelo, A. Cho, Z. Wang, A. Gazumyan, M. Cipolla, L. Luchsinger, C. D. Hillyer, M. Caskey, D. F. Robbiani, C. M. Rice, M. C. Nussenzweig, T. Hatziioannou, P. D. Bieniasz, Escape from neutralizing antibodies by SARS-CoV-2 spike protein variants. bioRxiv, doi:10.1101/2020.07.21.214759.
29. A. O. Hassan, J. B. Case, E. S. Winkler, L. B. Thackray, N. M. Kafai, A. L. Bailey, B. T. McCune, J. M. Fox, R. E. Chen, W. B. Alsoussi, J. S. Turner, A. J. Schmitz, T. Lei, S. Shrihari, S. P. Keeler, D. H. Fremont, S. Greco, P. B. McCray, S. Perlman, M. J. Holtzman, A. H. Ellebedy, M. S. Diamond, A SARS-CoV-2 Infection Model in Mice Demonstrates Protection by Neutralizing Antibodies. Cell. 182, 744-753.e4 (2020).
30. E. Beirnaert, A. Desmyter, S. Spinelli, M. Lauwereys, L. Aarden, T. Dreier, R. Loris, K. Silence, C. Pollet, C. Cambillau, H. de Haard, Bivalent Llama Single-Domain Antibody Fragments against Tumor Necrosis Factor Have Picomolar Potencies due to Intramolecular Interactions. Front. Immunol. 8, 867 (2017).
31. G. Hussack, T. N. Baral, J. Baardsnes, H. van Faassen, S. Raphael, K. A. Henry, J. Zhang, C. R. MacKenzie, A Novel Affinity Tag, ABTAG, and Its Application to the Affinity Screening of Single-Domain Antibodies Selected by Phage Display. Front. Immunol. 8, 1406 (2017).
32. L. Detalle, T. Stohr, C. Palomo, P. A. Piedra, B. E. Gilbert, V. Mas, A. Millar, U. F. Power, C. Stortelers, K. Allosery, J. A. Melero, E. Depla, Generation and characterization of ALX-0171, a potent novel therapeutic VHH for the treatment of respiratory syncytial virus infection. Antimicrob. Agents Chemother. 60, 6-13 (2016).
33. A. C. Walls, X. Xiong, Y. J. Park, M. A. Tortorici, J. Snijder, J. Quispe, E. Cameroni, R. Gopal, M. Dai, A. Lanzavecchia, M. Zambon, F. A. Rey, D. Corti, D. Veesler, Unexpected Receptor Functional Mimicry Elucidates Activation of Coronavirus Fusion. Cell. 176, 1026-1039.e15 (2019).
34. J. Huo, Y. Zhao, J. Ren, D. Zhou, H. M. Duyvesteyn, H. M. Ginn, L. Carrique, T. Malinauskas, R. R. Ruza, P. N. Shah, T. K. Tan, P. Rijal, N. Coombes, K. R. Bewley, J. A. Tree, J. Radecke, N. G. Paterson, P. Supasa, J. Mongkolsapaya, G. R. Screaton, M. Carroll, A. Townsend, E. E. Fry, R. J. Owens, D. I. Stuart, Neutralisation of SARS-CoV-2 by destruction of the prefusion Spike. Cell Host Microbe (2020), doi:10.1016/j.chom.2020.06.010.
35. Y. Wan, J. Shang, S. Sun, W. Tai, J. Chen, Q. Geng, L. He, Y. Chen, J. Wu, Z. Shi, Y. Zhou, L. Du, F. Li, Molecular Mechanism for Antibody-Dependent Enhancement of Coronavirus Entry. J. Virol. 94 (2020), doi:10.1128/JVI.02015-19.
36. S. Klepfer, A. P. Reed, M. Martinez, B. Bhogal, E. Jones, T. J. Miller, in Advances in Experimental Medicine and Biology (Springer New York LLC, 1995; https://link.springer.com/chapter/10.1007/978-1-4615-1899-0_38), vol. 380, pp. 235-241.
37. C. W. Olsen, W. V Corapi, C. K. Ngichabe, J. D. Baines, F. W. Scott, Monoclonal antibodies to the spike protein of feline infectious peritonitis virus mediate antibody-dependent enhancement of infection of feline macrophages. J. Virol. 66 (1992).
38. J. Lan, J. Ge, J. Yu, S. Shan, H. Zhou, S. Fan, Q. Zhang, X. Shi, Q. Wang, L. Zhang, X. Wang, Structure of the SARS-CoV-2 spike receptor-binding domain bound to the ACE2 receptor. Nature. 581, 215-220 (2020).
39. H. Streeck, B. Schulte, B. Kuemmerer, E. Richter, T. Hoeller, C. Fuhrmann, E. Bartok, R. Dolscheid, M. Berger, L. Wessendorf, M. Eschbach-Bludau, A. Kellings, A. Schwaiger, M. Coenen, P. Hoffmann, M. Noethen, A.-M. Eis-Huebinger, M. Exner, R. Schmithausen, M. Schmid, B. Kuemmerer, medRxiv, in press, doi:10.1101/2020.05.04.20090076.
40. X. Xie, A. Muruato, K. G. Lokugamage, K. Narayanan, X. Zhang, J. Zou, J. Liu, C. Schindewolf, N. E. Bopp, P. V. Aguilar, K. S. Plante, S. C. Weaver, S. Makino, J. W. LeDuc, V. D. Menachery, P. Y. Shi, An Infectious cDNA Clone of SARS-CoV-2. Cell Host Microbe. 27, 841-848.e3 (2020).
41. S. P. J. Whelan, L. A. Ball, J. N. Barr, G. T. W. Wertz, Efficient recovery of infectious vesicular stomatitis virus entirely from cDNA clones. Biochemistry. 92, 8388-8392 (1995).
42. C.-L. Hsieh, J. A. Goldsmith, J. M. Schaub, A. M. DiVenere, H.-C. Kuo, K. Javanmardi, K. C. Le, D. Wrapp, A. G. Lee, Y. Liu, C.-W. Chou, P. O. Byrne, C. K. Hjorth, N. V. Johnson, J. Ludes-Meyers, A. W. Nguyen, J. Park, N. Wang, D. Amengor, J. J. Lavinder, G. C. Ippolito, J. A. Maynard, I. J. Finkelstein, J. S. McLellan, Structure-based design of prefusion-stabilized SARS-CoV-2 spikes. Science (80-. )., eabdo826 (2020).
43. F. I. Schmidt, A. Lu, J. W. Chen, J. Ruan, C. Tang, H. Wu, H. L. Ploegh, A single domain antibody fragment that recognizes the adaptor ASC defines the role of ASC domains in inflammasome assembly. J. Exp. Med. 213, 771-790 (2016).
44. Z. Otwinowski, W. Minor, Processing of X-ray diffraction data collected in oscillation mode. Methods Enzymol. 276, 307-326 (1997).
45. A. J. McCoy, R. W. Grosse-Kunstleve, P. D. Adams, M. D. Winn, L. C. Storoni, R. J. Read, Phaser crystallographic software. J. Appl. Crystallogr. 40, 658-674 (2007).
46. H. Liu, N. C. Wu, M. Yuan, S. Bangaru, J. L. Torres, T. G. Caniels, J. van Schooten, X. Zhu, C.-C. D. Lee, P. J. M. Brouwer, M. J. van Gils, R. W. Sanders, A. B. Ward, I. A. Wilson, bioRxiv, in press, doi:10.1101/2020.08.02.233536.
47. P. Emsley, B. Lohkamp, W. G. Scott, K. Cowtan, Features and development of Coot. Acta Crystallogr. Sect. D Biol. Crystallogr. 66, 486-501 (2010).
48. P. D. Adams, P. V Afonine, G. Bunkoczi, V. B. Chen, I. W. Davis, N. Echols, J. J. Headd, L. W. Hung, G. J. Kapral, R. W. Grosse-Kunstleve, A. J. McCoy, N. W. Moriarty, R. Oeffner, R. J. Read, D. C. Richardson, J. S. Richardson, T. C. Terwilliger, P. H. Zwart, PHENIX: a comprehensive Python-based system for macromolecular structure solution. Acta Crystallogr D Biol Crystallogr. 66, 213-221 (2010).
49. E. Krissinel, K. Henrick, Inference of Macromolecular Assemblies from Crystalline State. J. Mol. Biol. 372, 774-797 (2007).
50. D. Tegunov, P. Cramer, Real-time cryo-electron microscopy data preprocessing with Warp. Nat. Methods. 16, 1146-1152 (2019).
51. A. Punjani, J. L. Rubinstein, D. J. Fleet, M. A. Brubaker, CryoSPARC: Algorithms for rapid unsupervised cryo-EM structure determination. Nat. Methods. 14, 290-296 (2017).
52. J. Zivanov, T. Nakane, B. O. Forsberg, D. Kimanius, W. J. H. Hagen, E. Lindahl, S. H. W. Scheres, New tools for automated high-resolution cryo-EM structure determination in RELION-3. Elife. 7 (2018), doi:10.7554/eLife.42166.
53. L. Hanke, M. L. P. Vidakovics, D. Sheward, H. Das, T. Schulte, A. M. Morro, M. Corcoran, A. Achour, G. K. Hedestam, B. M. Hällberg, B. Murrell, G. M. McInerney, bioRxiv, in press, doi:10.1101/2020.06.02.130161.
54. T. D. Goddard, C. C. Huang, E. C. Meng, E. F. Pettersen, G. S. Couch, J. H. Morris, T. E. Ferrin, UCSF ChimeraX: Meeting modern challenges in visualization and analysis. Protein Sci. 27,14-25 (2018).

## Claims

1. **A compound or composition for use in the treatment of disease caused by an infection with a corona virus,** wherein the compound or composition comprises at least a first antigen binding domain binding to a first epitope of a spike protein of the corona virus, **characterized in that** a binding of the at least first antigen binding domain to the first epitope of the spike protein stabilizes and/or induces an up-conformation of a receptor binding domain (RBD) of the spike protein.

2. The compound or composition for use of claim 1, further **characterized in that** a binding of the at least first antigen binding domain to a trimeric spike protein stabilizes and/or induces a post fusion confirmation of the trimeric spike protein a 2-up, more preferably a 3-up, conformation of the trimeric spike protein.

3. The compound or composition for use of claim 1 or 2, wherein the compound or composition comprises a second antigen binding domain binding to a second epitope of the spike protein, wherein the first and second epitope are (i) identical, (ii) overlapping, and/or (iii) distinct.

4. The compound or composition for use of claim 3, wherein a binding of the second antigen binding domain to the second epitope of the spike protein stabilizes and/or induces a post fusion confirmation of the trimeric spike protein and/or an up-conformation of a receptor binding domain (RBD) of the spike protein, and preferably wherein a binding of the second antigen binding domain to a trimeric spike protein stabilizes and/or induces a 2-up, more preferably a 3-up, conformation of the trimeric spike protein.

5. **A protein binding compound (PBC),** which is an isolated PBC comprising an amino acid sequence at least 80% identical to a sequence according to any one of SEQ ID NO: 1 to 92.

6. The PBC of claim 5, comprising at least one Complementary Determining Region (CDR) 3 having an amino acid sequence with at least 80% sequence identity to an amino acid sequence selected from SEQ ID Nos 3, 7, 11, 15, 19, 23, 27, 31, 35, 39, 43, 47, 51, 55, 59, 63, 67, 71, 75, 79, 83, 87, and 91, or having an amino acid sequence having no more than three, preferably no more than two or one, amino acid substitution(s), deletion(s), insertion(s) or addition(s) compared to these sequences.

7. The PBC of claim 5 or 6, wherein the PBC is a V_{H}H, or an antigen binding fragment thereof, composed of an antibody heavy chain sequence comprising any one of the sequences selected from SEQ ID Nos 4, 8, 12, 16, 20, 24, 28, 32, 36, 40, 44, 48, 52, 56, 60, 64, 68, 72, 76, 80, 84, 88, and 92, or in each case independently, optionally with not more than ten, nine, eight, five, three or two, preferably one, amino acid substitution(s), insertion(s) or deletion(s) compared to these.

8. The PBC of any one of claims 5 to 7, further comprising a second antigen binding domain binding to a second epitope of the spike protein.

9. **An isolated PBC,** which competes with a PBC as recited in any one of claims 5 to 8 for binding to the epitope of the spike protein.

10. **An isolated nucleic acid** encoding for a PBC which is a protein or polypeptide according to any one of the preceding claims.

11. **A recombinant host cell,** comprising a PBC or an isolated nucleic acid according to any one of the preceding claims.

12. **A pharmaceutical composition** comprising a PBC recited in any of claims 5 to 9, an isolated nucleic acid recited in claim 10, a recombinant host cell recited in claim 11, together with a pharmaceutically acceptable carrier and/or excipient.

13. **A compound or composition for use in medicine,** wherein the compound or composition comprises a PBC recited in any of claims 5 to 9, an isolated nucleic acid recited in claim 8, a recombinant host cell recited in claim 9, or a pharmaceutical composition of claim 12.

14. **An in vitro method for identifying and/or characterising a compound suitable for the treatment of a disease,** disorder or condition that is associated with, or caused by, a virus expressing a viral spike (S) protein, the method comprising the steps of:
**(a)** bringing into contact a candidate compound and a spike protein (or a variant or fragment thereof); and
**(b)** detecting and/or quantifying an induction and/or stabilization of an up-conformation of the spike protein (or the variant or fragment thereof) upon binding of the candidate compound to the spike protein (or the variant or fragment thereof),
wherein the induction and/or stabilization of an up-conformation of the spike protein (or the variant or fragment thereof) upon binding of the candidate compound to the spike protein (or the variant or fragment thereof)) compared to a control indicates that the compound is suitable for the treatment of a disease, disorder or condition that is associated with, or caused by, a virus expressing the spike protein.

15. **A method for identifying and/or characterising a compound suitable for the treatment of a disease, disorder or condition that is associated with, or caused by, a virus expressing a viral spike (S) protein,** the method comprising the steps of:
**(a)** Providing at least two cells each expressing the viral spike protein or a truncation thereof on the cell surface, preferably wherein the at least two cells do not express a spike protein receptor (such as angiotensin converting enzyme 2 (ACE2);
**(b)** bringing into contact the at least two cells, a candidate compound, and a protein binding compound (PBC) of any one of claims 5 to 9; and
**(c)** detecting and/or quantifying an induction of a cellular fusion between the at least two cells induced by the PBC of the invention,
wherein a reduced induction of a cellular fusion between the at least two cells compared to a control without the candidate compound indicates that the candidate compound is suitable for the treatment of a disease, disorder or condition that is associated with, or caused by, a virus expressing the spike protein.
